(19) Europäisches Patentamt
European Patent Office
Office européen des brevets

(11) Publication number: **0 288 196 B1**

(12) **EUROPEAN PATENT SPECIFICATION**

(45) Date of publication of patent specification: **28.07.93**

(51) Int. Cl.5: **C07D 471/14**, A61K 31/47, //(C07D471/14,221:00,221:00, 221:00)

(21) Application number: **88303271.6**

(22) Date of filing: **12.04.88**

(54) **Sulfonyldecahydro-8H-isoquino[2,1-g][1,6]naphthyridines, optical isomers thereof and related compounds.**

(30) Priority: **13.04.87 US 37320**

(43) Date of publication of application:
**26.10.88 Bulletin 88/43**

(45) Publication of the grant of the patent:
**28.07.93 Bulletin 93/30**

(84) Designated Contracting States:
**AT BE CH DE ES FR GB GR IT LI LU NL SE**

(56) References cited:
**US-A- 4 454 139**
**US-A- 4 550 114**

**CHEMICAL ABSTRACTS, vol. 93, no. 19, November 10, 1980, Columbus, Ohio, US; L. SZABO: "Investigation on the chemistry of berbans. Part IX. 13-Azaberban derivatives via reductive cyclisation of delta-oxonitriles" page 692, column2, abstract-no. 186 627h**

(73) Proprietor: **SYNTEX (U.S.A.) INC.**
**3401 Hillview Avenue**
**Palo Alto California 94303(US)**

(72) Inventor: **Clark, Robin D.**
**114 Lowell Avenue**
**Palo Alto California 94301(US)**

(74) Representative: **Armitage, Ian Michael et al**
**MEWBURN ELLIS & CO. 2 Cursitor Street**
**London EC4A 1BO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

### BACKGROUND OF THE INVENTION

### Field of the Invention

The invention relates to various sulfonyldecahydro-8H-isoquino[2,1-g][1,6]naphthyridines, optical isomers thereof and to pharmaceutical compositions containing these compounds. Specifically the compounds of the invention exhibit selective $\alpha_2$-blockade in mammals, and which, therefore, are useful as medicaments for the treatment of physiological conditions affected by such selective blockade. Such activities include, for example, lowering of blood pressure, amelioration of depression, inhibition of platelet aggregation, palliation of diabetes, alleviation of male impotence and weight-loss stimulation. In addition, the compounds of formula (I) have been found to be useful for the treatment of irritable-bowel syndrome, cyclic mood disturbances in women, anxiolytic conditions and in the lowering of intraoccular pressure.

### Previous Disclosures

The novel compounds of this invention are optical isomers of various sulfonyldecahydro-8H-isoquino-[2,1-g][1,6]naphthyridines, useful as selective $\alpha_2$-blockers. Compounds somewhat related to the novel compounds of this invention are described in U.S. Patent Nos. 3,953,598, 4,353,911, 4,454,139 and 4,550,114, and in Nouveau J. Chim. 4(3), 199-202 (1980).

### SUMMARY OF THE INVENTION

One aspect of the invention concerns novel compounds represented by the formula:

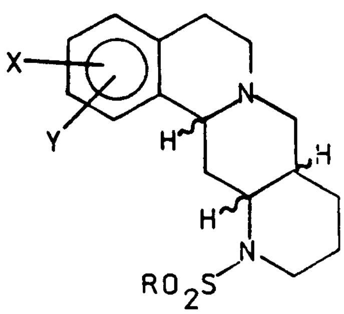

(I)

in which:

X and Y are independently hydrogen, hydroxy, lower alkyl of one to six carbon atoms, lower alkoxy of one to six carbon atoms or halo, or X and Y taken together is methylenedioxy or ethylene-1,2-dioxy, and

R is lower alkyl of one to six carbon atoms; phenyl optionally substituted by one or two substituents chosen from halo or amino groups or lower alkyl or lower alkoxy groups of one to four carbon atoms; $-(CH_2)_mOR^1$; or $-NR^1R^2$ wherein m is an integer of 1 to 6 and $R^1$ and $R^2$ are independently hydrogen or lower alkyl, or the group $-NR^1R^2$ forms a heterocycle of the formula:

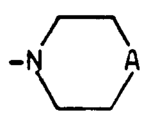

wherein A is $-CH_2-$, $-NR^1-$ or oxygen, wherein $R^1$ has the above meaning and the wavy lines indicate that the hydrogen atom attached thereto is in either the $\alpha$- or $\beta$-position; or a pharmaceutically acceptable

salt thereof.

Another aspect of the invention concerns novel compounds represented by the formulas:

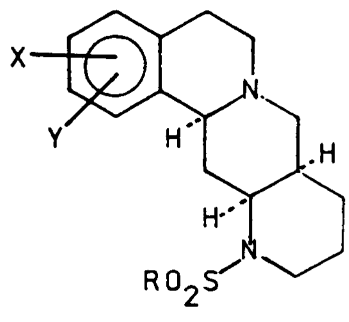

(1)

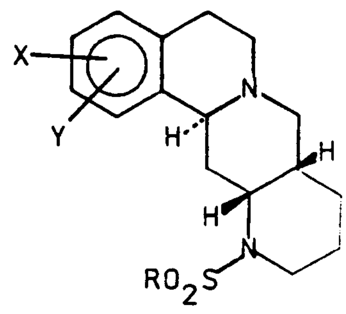

(2)

in which X, Y and R are as defined hereinabove. The compounds of formula (1) are preferred.

Other aspects of the invention relate to the methods of preparation of compounds of formula (I) thereof, to pharmaceutical compositions containing such compounds in admixture with one or more pharmaceutically acceptable, non-toxic carriers, to methods pertaining to their use and to intermediates.

## DETAILED DESCRIPTION OF THE INVENTION

### Definitions

As used herein:

"Alkyl" means a branched or unbranched saturated hydrocarbon chain containing 1 to 8 carbon atoms, such as methyl, ethyl, propyl, tert-butyl, n-hexyl, n-octyl and the like;

"Lower alkyl" means a branched or unbranched saturated hydrocarbon chain containing 1 to 6 carbon atoms, such as methyl, ethyl, propyl, isopropyl, tert-butyl, butyl, n-hexyl and the like.

"Lower alkoxy" means the group -OR wherein R is lower alkyl as herein defined.

"Halo" means fluoro, chloro, bromo and iodo.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like. It is self-evident that the counterions associated with said pharmaceutically acceptable acid addition salts are the anions derived from said inorganic and organic acids. Furthermore, it is self-evident that said inorganic and organic anions may be mono- or polyvalent depending on the acid from which they are derived.

"Optional" or "optionally" means that the subsequently described event or circumstance may or may not occur, and that the description includes instances where said event or circumstances occurs and instances in which it does not. For example, "optionally substituted phenyl" means that the phenyl may or may not be substituted and that the description includes both unsubstituted phenyl and substituted phenyl.

The term "treatment" as used herein covers any treatment of a disease in a mammal, particularly a human, and includes:

(i) preventing the disease from occurring in a subject which may be predisposed to the disease but has not yet been diagnosed as having it;

(ii) inhibiting the disease, i.e., arresting its development; or

(iii) relieving the disease, i.e., causing regression of the disease.

The term "(±)" is used to designate a racemic mixture of individual (+) and (-) isomers. The (±) racemate as well as the individual (+) and (-) enantiomers and non-racemic mixtures thereof are included within the scope of this invention.

"Isomers" are different compounds that have the same molecular formula.

"Stereoisomers" are isomers that differ only in the way the atoms are arranged in space.

"Enantiomers" are a pair of stereoisomers that are non-superimposable mirror images of each other. A 1:1 mixture of a pair of enantiomers is a "racemic" mixture.

"Racemic" or "non-racemic" refer to mixtures of the enantiomers of a chiral molecule wherein the ratio of the amount of one enantiomer to the amount of its optical isomer is 1:1 or is not 1:1, respectively.

"Diastereoisomers" are stereoisomers which are not mirror-images of each other.

"Epimers" are diastereoisomers which differ only in the configuration of one asymmetric center.

"Epimerizing" refers to a process whereby one epimer is converted to another epimer.

The terms "$\alpha$" and "$\beta$" indicate the specific stereochemical configuration of a substituent at an asymmetric carbon atom in a chemical structure as drawn. Thus "$\alpha$", denoted by a broken line, indicates that the group at the position in question is below the general plane of the molecule as drawn, and "$\beta$", denoted by a bold line, indicates that the group at the position in question is above the general plane of the molecule as drawn.

The absolute stereochemistry at carbons 8a, 12a and 13a is specified according to the Cahn-Ingold-Prelog R-S system. When the compound is a pure enantiomer, the stereochemistry at each chiral carbon is specified by either R or S. When a compound is a racemic mixture the stereochemistry at each chiral carbon is specified by either RS or SR by reference to a single enantiomer of the racemate. In this manner relative stereochemistry is conveyed unambiguously. A description of the R-S convention may be found, for example, in "Introduction to Organic Chemistry" by A. Streitwieser, Jr. and C. Heathcock, (Macmillan Pub. Co., New York, 1976), pages 110-114.

The compounds of the invention will be named using the numbering system shown below.

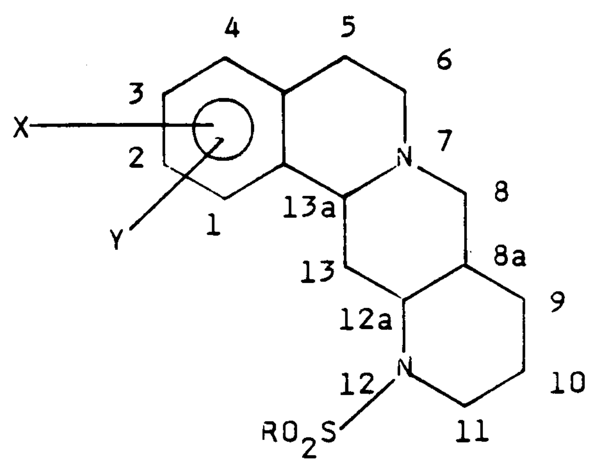

Compounds of formula (I) are named and numbered as derivatives of isoquinonaphthyridine using a modified form of IUPAC nomenclature. For example, an optically active compound of formula (1) below wherein X is 3-methoxy, Y is hydrogen and R is methyl is named (8aR,12aS,13aS)-3-methoxy-12-methanesulfonyl-5,6,8a,9,10,11,12,12a,13,13a-decahydro-8H-isoquino[2,1-g][1,6]-naphthyridine.

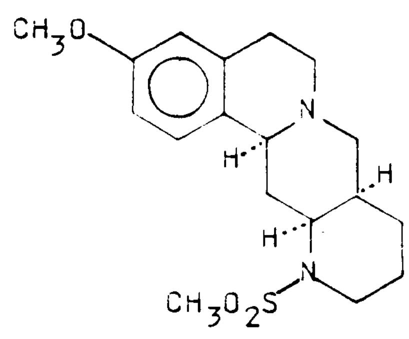

In a similar manner, a compound of formula (1) wherein X and Y are hydrogen and R is 2-methylpropyl is named (8aR,12aS,13aS)-12-(2-methylpropanesulfonyl)-5,6,8a,9,10,11,12,12a,13,13a-decahydro-8H-isoquino[2,1-g][1,6]-naphthyridine and a compound of formula (1) wherein X and Y taken together is 2,3-methylenedioxy and R is dimethylamino, is named (8aR,12aS,13aS)-2,3-methylenedioxy-12-(N,N-dimethylaminosulfonyl)-5,6,8a,9, 10,11,12,12a,13,13a-decahydro-8H-isoquino[2,1-g][1,6]naphthyridine.

In a similar manner, racemic compounds of formula (1) and (2) are named as follows:

A compound of formula (1) wherein X is 3-methoxy, Y is hydrogen and R is methyl is named:

(±)-3-methoxy-12-methanesulfonyl-5,6,8aα,9,10,11,12,12aα,13,13aα-decahydro-8H-isoquino[2,1-g][1,6]-naphthyridine.

A compound of formula (1) wherein X and Y are hydrogen and R is 2-methylpropyl is named:

(±)-12-(2-methylpropanesulfonyl)-5,6,8aα,9,10,11,12,12aα,13,13aα-decahydro-8H-isoquino[2,1-g][1,6]-naphthyridine.

A compound of formula (2) wherein X and Y taken together is 2,3-methylenedioxy and R is dimethylamino, is named:

(±)-2,3-methylenedioxy-12-(N,N-dimethylaminosulfonyl)-5,6,8aβ,9,10,11,12,12aβ,13,13aα-decahydro-8H-isoquino[2,1-g][1,6]naphthyridine.

Preferred Embodiments:

Among the family of compounds of the present invention, a first preferred group includes the compounds of formula (1) and the pharmaceutically acceptable salts thereof. More preferred are the compounds of formula (1) in which X and Y are independently hydrogen or lower alkoxy, or X and Y taken together is methylenedioxy, and R is lower alkyl, $-(CH_2)_mOR^1$ or $-NR^1R^2$ and the pharmaceutically acceptable salts thereof. Preferred compounds of this type include those in which R is $-(CH_2)_2OR^1$ where $R^1$ is methyl and X and Y are independently hydrogen or lower alkoxy having one to four carbon atoms, or X and Y taken together is methylenedioxy. One preferred class within this first group includes compounds in which X and Y taken together is methylenedioxy and R is lower alkyl, especially where R is methyl and the pharmaceutically acceptable salts thereof. A second preferred class within this group includes compounds in which X and Y are independently hydrogen or methoxy and R is methyl, dimethylamino or 2-methoxyethyl, especially where X is methoxy and Y is hydrogen and the pharmaceutically acceptable salts thereof.

A second preferred group includes compounds of formula (2). Within this group a preferred subgroup includes the compounds in which X and Y are independently hydrogen or lower alkoxy, or X and Y taken together is methylenedioxy, and R is lower alkyl, $-(CH_2)_mOR^1$ or $-NR^1R^2$.

At present, the preferred compounds are:

(8aR,12aS,13aS)-3-methoxy-12-methanesulfonyl-5,6,8a,9,10,11,12,12a,13,13a-decahydro-8H-isoquino-[2,1-g][1,6]naphthyridine;

(8aR,12aS,13aS)-2,3-dimethoxy-12-methanesulfonyl-5,6,8a,9,10,11,12,12a,13,13a-decahydro-8H-isoquino [2,1-g][1,6]naphthyridine;

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aS)-12-methanesulfonyl-5,6,8a,9,10,11,12,12a,13,13a-decahydro-8H-isoquino[2,1-g][1,6]-naphthyridine;

(8aR,12aS,13aS)-12-(2-methoxyethanesulfonyl)-5,6,8a,9,10,11,12,12a,13,13a-decahydro-8H-isoquino[2,1-g][1,6]naphthyridine;

(8aR,12aS,13aS)-3-methoxy-12-(2-methoxyethanesulfonyl)-5,6,8a,9,10,11,12,12a,13,13a-decahydro-8H-isoquino[2,1-g][1,6]naphthyridine; and

(8aR,12aS,13aS)-3-methoxy-12-N,N-dimethylamino-sulfonyl-5,6,8a,9,10,11,12,12a,13,13a-decahydro-8H-isoquino[2,1-g][1,6]naphthyridine and the pharmaceutically acceptable salts thereof.

(±)-3-methoxy-12-methanesulfonyl-5,6,8aα,9,10,11,12,12aα,13,13aα-decahydro-8H-isoquino[2,1-g][1,6]-naphthyridine or its salts, especially the hydrochloride;

(±)-2,3-dimethoxy-12-methanesulfonyl-5,6,8aα,9,10,11,12,12aα,13,13aα-decahydro-8H-isoquino[2,1-g]-[1,6]naphthyridine hydrochloride;

(±)-12-methanesulfonyl-5,6,8aα,9,10,11,12,12aα,13,13aα-decahydro-8H-isoquino[2,1-g][1,6]naphthyridine hydrochloride;

(±)-12-(2-methoxyethanesulfonyl)-5,6,8aα,9,10,11,12,12aα,13,13aα-decahydro-8H-isoquino[2,1-g][1,6]-naphthyridine hydrochloride;

(±)-3-methoxy-12-(2-methoxyethanesulfonyl)-5,6,8aα,9,10,11,-12,12aα,13,13aα-decahydro-8H-isoquino-

[2,1-g][1,6]naphthyridine hydrochloride; and

(±)-12-(N,N-dimethylaminosulfonyl)-5,6,8aα,9,10,11,12,2aα,13,13aα-decahydro-8H-isoquino[2,1-g][1,6]-naphthyridine hydrochloride.

Intermediates

As indicated above at page 3 one aspect of the present invention relates to intermediates for the formation of compounds of formula (I).

Preferred intermediates include:

(i) compounds of the formula

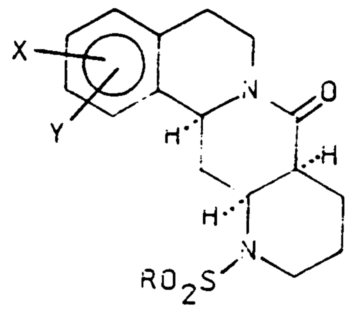

wherein X, Y and R are as broadly defined above for formula (I);

(ii) compounds of the formula

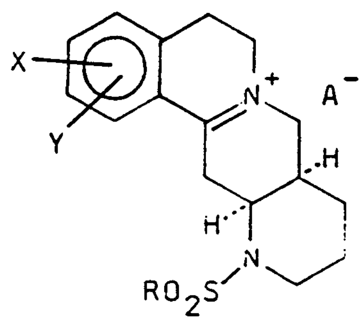

in which X, Y and R are as defined above and A represents an organic or inorganic anion; and

(iii) compounds of the formula

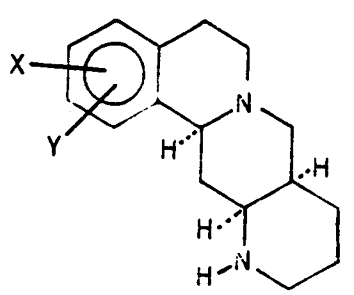

in which X and Y are as broadly defined above. In this last case preferably at least one of X and Y is not hydrogen; X is then preferably lower alkoxy of one to six carbons and Y is hydrogen; alternatively X and

Y taken together are methylenedioxy or ethylene- 1,2-dioxy.
(iv) single enantiomers of the formula

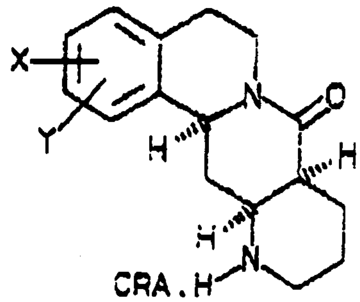

in which X and Y are as defined above, and CRA is an optically active acid (chiral resolving acid).

Method of Preparation

The compounds of formula (I), (1) and (2) are prepared from the intermediates of formulae (VII, VIII), (VII) and (VIII), respectively, the preparation of which is illustrated below in Reaction Scheme I.

It should be understood that the structures illustrated in Reaction Scheme I and in all subsequent Reaction Schemes are intended to represent racemic mixtures unless otherwise stated, although for the sake of clarity, one one enantiomer is shown.

## REACTION SCHEME I

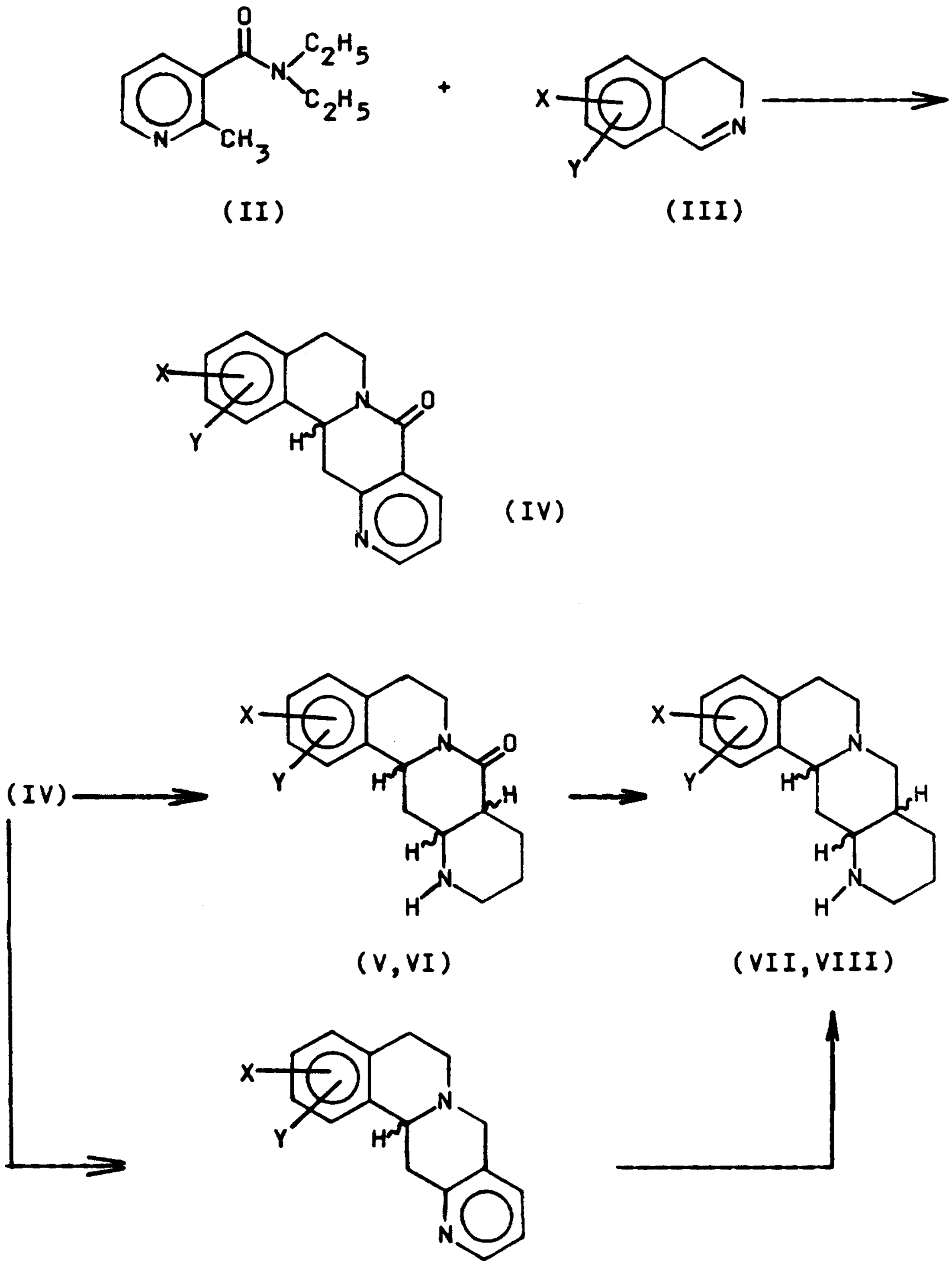

(II)

(III)

(IV)

(IV)

(V,VI)

(VII,VIII)

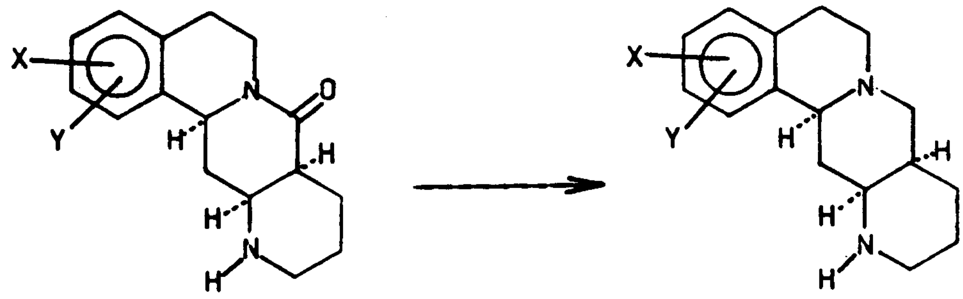

(V) (VII)

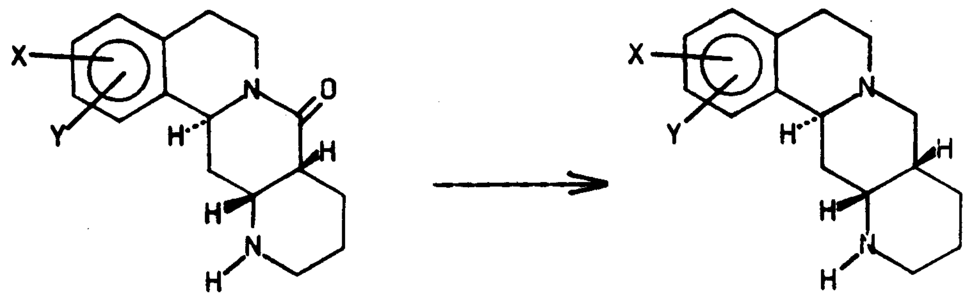

(VI) (VIII)

The intermediate of formula (II), 2-methylnicotinic acid diethylamide, is prepared according to the method disclosed in Ber., 72B, 563 (1939). The intermediates of formula (III), optionally substituted dihydroisoquinolines, are prepared according to the method of Bischler-Napieralski, disclosed in Organic Reactions, Vol. VI, p 74 (1951), by the cyclization of formamidines of commercially available optionally substituted phenylethylamines. To prepare the compounds of formula (IV), the compounds of formula (II) and (III) are reacted together in the presence of a strong base, for example potassium t-butoxide, sodamide, sodium triphenylmethane, lithium diethylamide or preferably lithium diisopropylamide. The reaction is preferably carried out in an ethereal solvent, for example diethyl ether, dimethoxyethane, dioxane or tetrahydrofuran, at a temperature of about 0°C to -50°C, preferably at about -10°C to -40°C, for about 30 minutes to 4 hours. For example, diisopropylamine is dissolved in an ethereal solvent, preferably tetrahydrofuran, and cooled to a temperature of about -20° to -80°C, preferably about -65°C. To the cooled solution about 1 molar equivalent of an alkyl lithium, preferably 1.6M n-butyllithium, is added. To this cold solution is added a mixture of about 1 molar equivalent of the compound of formula (II) and about 1 molar equivalent of the compound of formula (III) in an ethereal solvent, preferably tetrahydrofuran. The reaction mixture is allowed to warm to about -10° to -40°C, preferably about -20°C, over a period of about 1 hour, and the reaction then quenched with an acid, preferably hydrochloric acid. The product of formula (IV), a (±)-5,6,13,13a-tetrahydroisoquino[2,1-g][1,6]naphthyridine-8-one hydrochloride, is isolated and purified by conventional means, preferably recrystallization of an acid salt.

The compound of formula (IV) or an acid salt thereof is then hydrogenated with a suitable heterogeneous catalyst, for example palladium on carbon, platinum oxide or preferably rhodium on alumina, to give a mixture of the diastereoisomers of formula (V,VI). For example, for every gram of the compound of formula (IV) in a solution of acetic acid is added from 0.1 to 0.6 g, preferably aoout 0.25 g, of 5% rhodium on alumina catalyst and the mixture hydrogenated at a pressure of about 25-80 psi (1.72-5.52 bar), preferably about 50 psi (3.45 bar). The reaction is conducted at a temperature of about 0° to 50°C, preferably about 25°C, for about 24 -72 hours, preferably about 42 hours. When the reaction is substantially complete, the mixture of diastereomers of formula (V,VI) is isolated by conventional means. Preferably, the mixture of diastereomers of formula (V,VI) is separated by chromatography on silica gel, eluting with a suitable solvent mixture, for example 5-20% methanol in methylene chloride. The first component eluted is the compound of formula (VI), followed by the compound of formula (V).

The mixture of diasteromers of formula (V,VI) is then reduced to the mixture of diastereomers of formula (VII,VIII) according to the procedures described hereinbelow. Preferably, the individual compounds of

formula (V) and (VI) are reduced to the compounds of formula (VII) and (VIII) with a suitable reducing agent, for example borane, triethyloxonium tetrafluoroborate followed by sodium borohydride, sodium borohydride in the presence of a carboxylic acid, or preferably lithium aluminum hydride. For example, a solution of a compound of formula (V) in an ethereal solvent, preferably tetrahydrofuran, is slowly added to a solution of about 1 to 4 molar equivalents, preferably about 1.5 to 2 molar equivalents, of lithium aluminum hydride in the same ethereal solvent at about 25 °C. The mixture is then refluxed for about 1-10 hours, preferably about 3 hours. When the reaction is substantially complete, the compound of formula (VII) is separated and purified by conventional means. In a similar fashion, the compound of formula (VI) is reduced to the compound of formula (VIII), and likewise separated and purified.

Alternatively, the mixture of diastereomers of formula (VII,VIII) or the individual compounds of the formula (VII) or (VIII) may be prepared by reducing the lactam moiety of the compound of the formula (IV) with a suitable reducing agent as described above, followed by catalytic hydrogenation under conditions as described above, and, if desired, separation of the compounds of the formula (VII) and (VIII).

Compounds of Formula (I), (1) and (2)

Compounds of formula (I), (1) and (2) are prepared from a mixture of diastereomers of formula (VII,VIII) or the individual compounds of formula (VII) and (VIII), respectively, as depicted in Reaction Sequence II below.

## REACTION SCHEME II

(VII,VIII)

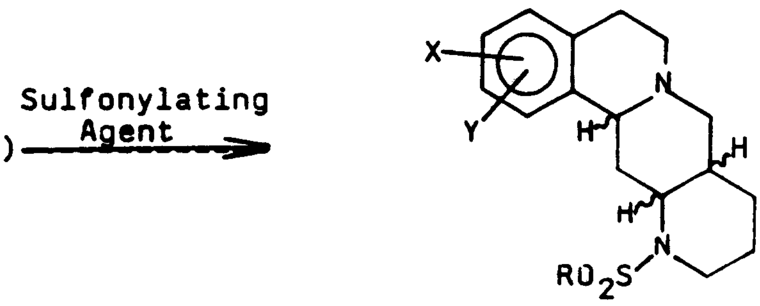


(I)

(VII)

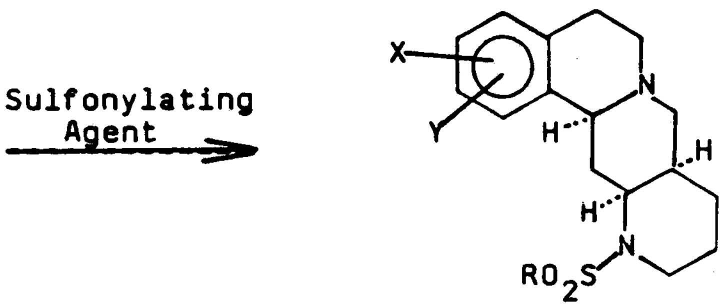


(1)

(VIII)

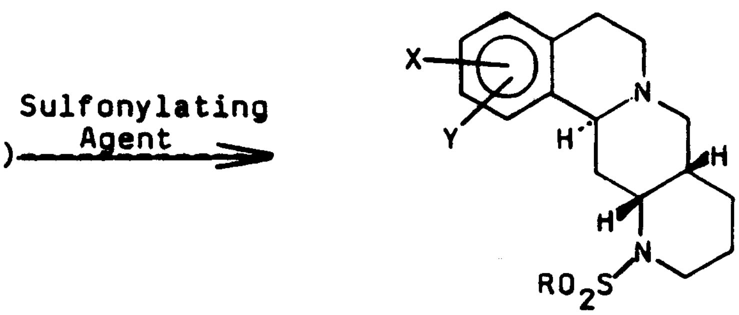


(2)

The compounds of formula (I), (1) and (2) are prepared by reacting a mixture of diastereomers of formula (VII,VIII) or the individual compounds of formula (VII) and (VIII), respectively, with a sulfonic acid of the formula $HOSO_2R$ or an activated form thereof, wherein R is as defined above. Activated forms of the formula $HOSO_2R$ may be represented by the formula $LSO_2R$ wherein R is as defined above and L is a leaving group. A preferred activated form is a substituted sulfonyl halide of the formula $ZSO_2R$, where Z is chlorine or bromine and R is as defined above. The sulfonyl halides of formula $ZSO_2R$ are either commercially available from, inter alia, Aldrich Chemical Co., or may be prepared according to the method of Zeigler and Sprague, disclosed in J. Org. Chem., Vol 16, p 621 (1951).

For example, to prepare the compound of formula (1) the compound of formula (VII) is dissolved in an inert organic solvent, such as benzene, toluene, ethyl acetate, tetrahydrofuran, diethyl ether, chloroform or preferably dichloromethane, containing from 1-10 molar equivalents, preferably about 2 molar equivalents of a base. The base may be selected from an inorganic base such as sodium carbonate, potassium bicarbonate and the like, or an organic base, such as pyridine, N-methylpiperidine and the like. Preferably, the reaction is carried out with a tertiary organic base such as triethylamine. The mixture is cooled to about -10° to 10°C, preferably about 0°C, and about 1-4 molar equivalents, preferably about 1.25 molar equivalents, of the appropriately substituted sulfonic acid of the formula $HOSO_2R$ wherein R is as defined hereinabove or an activated form thereof, preferably a sulfonyl halide of formula $ZSO_2R$, is added and the

mixture stirred for about 30 minutes to 4 hours, preferably about 1 hour at a temperature of about 10° to 40°C, preferably about 25°C. An inert solvent, preferably diethyl ether, is then added, and the compound of formula (1) separated and purified by conventional means, for example recrystallization of an acid salt.

Similarly, the opposite enantiomer of formula (1), formula (3), is obtained if the enantiomer of the intermediate of formula (VII) is reacted as shown in Reaction Scheme II, i.e.:

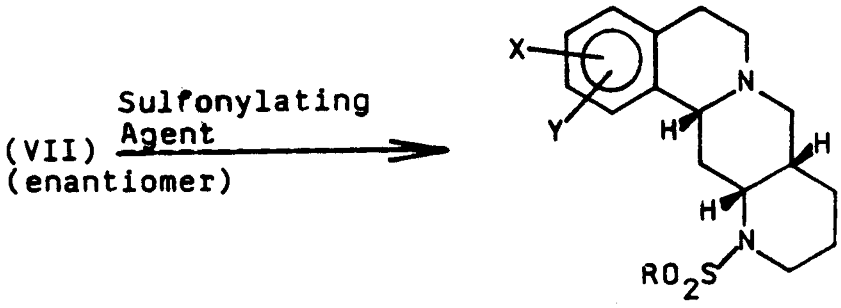


(3)

An alternative procedure for the preparation of compounds of formula (1) and (2) is from the mixture of diastereomers of formula (VII,VIII) obtained as shown above in Reaction Scheme I . The mixture of diastereomers of the formula (VII,VIII) is treated with an appropriately substituted sulfonic acid of the formula $HOSO_2R$ or an activated form thereof, preferably a sulfonyl halide of formula $ZSO_2R$ in the same manner as shown above, to give a mixture of the compounds of formula (1) and (2) which is separated by conventional means, preferably chromatography, into the individual diastereoisomers of formula (1) and (2).

Another alternative procedure for the preparation of the compounds of formula (1) and (2) is from the mixture of diastereomers of the formula (V,VI) or the individual compounds of the formula (V) and (VI). The mixture of diastereomers of the formula (V,VI) or the individual compounds of the formula (V) and (VI), are sulfonylated according to the conditions described hereinabove followed by reduction of the lactam moiety according to the procedures described hereinabove. If a mixture of the diastereomers of the formula (V,VI) is used, then the separation of the isomers may be performed either after the sulfonylation step or after the reduction step, according to methods well known in the art.

An alternative preparation of the compound of formula (1) is shown in Reaction Scheme III below.

## REACTION SCHEME III

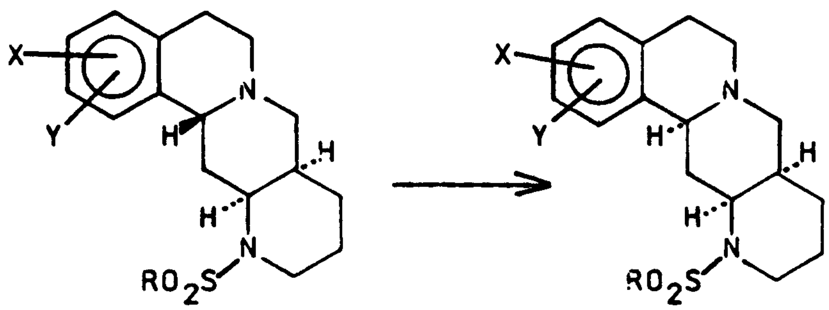

(2) (1)

In this procedure, the compound of formula (2) is epimerized to the compound of formula (1).

The compound of formula (2) is epimerized to the compound of formula (1) via an oxidation/reduction sequence. Typically, the compound of formula (2) is oxidized under conditions appropriate for producing the intermediate of the formula (4) wherein X, Y and R are as defined hereinabove and A is representative of an inorganic or organic anion, followed by reduction with a suitable reducing agent.

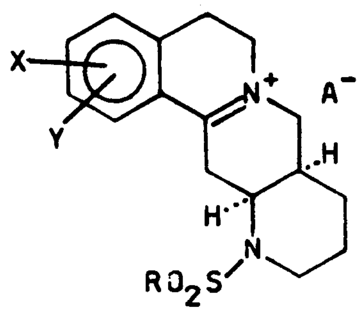

(4)

Typically, the compound of formula (2) may be oxidized with about 1-10 molar equivalents, preferably about 4 molar equivalents of an oxidizing agent. Representative oxidizing agents are iodine pentafluoride, Hg(II) salts, Pb(IV) salts, Ni(I) salts, Ag(II) salts, N-bromo- and N-chlorosuccinimide, $Cl_2$ and the like. A preferred oxidizing agent is a mercuric salt, preferably mercuric acetate, in acetic acid containing from about 5%-50%, preferably about 10%,, of water and the mixture is heated at a temperature of about 70 °C to the reflux temperature, preferably about 105 °C, for about 30 minutes to 4 hours, preferably about 1 hour. After filtering, hydrogen sulfide is passed through, followed by refiltering and removal of the solvent from the filtrate.

To effect the reduction, the residue is then dissolved in a protic solvent, preferably an organic solvent, more preferably an alcoholic solvent, most preferably ethanol, the solution cooled to a temperature of about 0° to -40 °C, preferably about -20 °C, and treated with about 1 to 10 molar equivalents, preferably about 4 molar equivalents, of a reducing agent. Representative reducing agents are hydride reducing agents such as lithium aluminum hydride, sodium borohydride, sodium cyanoborohydride, diborane, diisobutylaluminum hydride, zinc borohydride and the like. A preferred reducing agent is a borohydride salt, more preferably sodium borohydride. When the reaction is substantially complete the compound of formula (1) is isolated by conventional means, for example chromatography.

Alternatively, the epimerization of a compound of formula (2) to a compound of the formula (1) may be carried out by dissolving the compound of formula (2) in an inert solvent, preferably chloroform, at a temperature of about -20° to 10 °C, preferably about 0 °C, and treated with about 0.9 to 2 molar equivalents, preferably about 1.4 molar equivalents of an oxidizing agent, preferably $H_2O_2$ or a peracid oxidizing agent, more preferably peracetic acid, perbenzoic acid or, most preferably meta-chloroperbenzoic acid, for about 10 minutes to 4 hours, preferably about 30 minutes, followed by about 20 minutes at room temperature. The reaction mixture is then recooled to about -20° to 10 °C, preferably about 0 °C, and treated with about 1 to 20 molar equivalents, preferably about 5.5 molar equivalents, of an acylating agent, preferably trifluoroacetic anhydride. The reaction is carried out at a temperature of about 10° to 30 °C, preferably about 20 °C, for about 5 minutes to about 2 hours, preferably about 30 minutes. The solvent is then removed under reduced pressure, and a protic solvent added, preferably ethanol. An excess of a reducing agent, preferably sodium borohydride is then added slowly at a temperature of about -10° to 30 °C, preferably about 0 °C, until the solution becomes basic. When the reaction is substantially complete the compound of formula (1) is isolated by conventional means, for example chromatography or preferably recrystallization.

A mixture of compounds of formula (1) and (2) may replace the compound of formula (2) as a starting material in the procedures described above, giving rise to the same product of formula (1). Such a mixture of compounds of formula (1) and (2) is obtained as shown in Reaction Scheme II.

The compounds of formula (I), (1) and (2) in which R is $-(CH_2)_mOH$ wherein m is as defined hereinabove are preferably prepared by dealkylating the compounds of formula (I), (1) and (2) in which R is $-(CH_2)_mOR^1$ wherein $R^1$ is lower alkyl and m is as defined hereinabove. Typically, the compound of formula (I), (1) or (2) in which R is $-(CH_2)_mOR^1$ wherein $R^1$ and m are as defined hereinabove is dissolved in an inert solvent as defined above, preferably methylene chloride, and reacted with about 1 to 4 molar equivalents, preferably about 1.7 molar equivalents of a dealkylating agent. Representative dealkylating agents are boron trihalides, trialkylsilyl iodides, aluminum trihalides and the like. A preferred dealkylating agent is boron tribromide and the reaction is performed at a temperature of about -40 °C to -80 °C, preferably about -60 °C. The mixture is then allowed to warm to about 0 °C to 40 °C, preferably about 25 °C. When the reaction is substantially complete the compound of formula (I), (1) or (2) in which R is $-(CH_2)_mOH$ wherein m is as defined hereinabove is isolated by conventional means, for example chromatography.

Alternatively, the compounds of formula (I), (1) and (2) in which R is $-(CH_2)_mOR^1$ wherein $R^1$ is lower alkyl and m is as defined hereinabove are preferably prepared by alkylating the compounds of formula (I), (1) and (2) in which R is $-(CH_2)_mOH$ wherein m is as defined hereinabove. Typically, the compound of formula (I), (1) or (2) in which R is $-(CH_2)_mOH$ wherein m is as defined hereinabove is dissolved in an inert solvent as defined above, and reacted with about 1 to 4 molar equivalents, preferably about 1.0 molar equivalent of an alkylating agent. Representive alkylating agents are lower alkyl alkylating agents such as lower alkyl halides and sulfonates and the like. The compound of formula (I), (1) or (2) in which R is $-(CH_2)_mOR^1$ wherein $R^1$ and m are as defined hereinabove is isolated by conventional means, for example chromatography.

The compounds of formula (I), (1) and (2) in which R is phenyl substituted with one or two amino groups are preferably prepared from the corresponding compounds in which R is phenyl substituted with one or two nitro groups; these are prepared by reacting the appropriate compounds of formula (VII,VIII), (VII) and (VIII) with a nitrobenzenesulfonyl chloride or bromide to give the corresponding compounds of the general formula (I), (1) or (2) in which R is phenyl substituted with one or two nitro groups, using the general method shown in Reaction Scheme I above. The latter compound is then dissolved in a protic solvent, preferably ethanol, and hydrogenated at about 50 psi (3.45 bar) in the presence of a palladium on carbon catalyst. When the reaction is substantially complete the compound of formula (I), (1) or (2) in which R is phenyl substituted with one or two amino groups is isolated by conventional means, for example crystallization of an acid salt.

Alternatively, the compounds of formula (I), (1) and (2) wherein X and Y are independently lower alkoxy or wherein X and Y taken together is methylenedioxy or ethylene-1,2-dioxy may be prepared from the corresponding hydroxy compounds of formula (I), (1) and (2) by treatment with an appropriate alkylating agent, optionally in the presence of a base. Typical alkylating agents for preparing compounds wherein X and Y are independently lower alkoxy include alkyl halides, dialkyl sulfates, trialkyloxonium salts, diazoalkanes and reagents that generate diazoalkanes. Typical alkylating agents for preparing compounds wherein X and Y taken together are methylenedioxy or ethylene-1,2-dioxy are dihalomethane derivatives and 1,2-disubstituted ethane derivatives wherein the substituents are leaving groups, respectively. Typical bases include organic and inorganic bases such as sodium hydroxide, potassium carbonate, potassium fluoride, potassium t-butoxide and the like.

Alternatively, a compound of the formula (I), (1) or (2) may be prepared from the compounds of the formula (VII,VIII), (VII) and (VIII) by a two step procedure. A compound of the formula (VII,VIII), (VII) or (VIII) is reacted with an acid of the formula RWSOH, wherein R is as defined hereinabove and W is an electron pair or oxygen, or an activated form thereof represented by the formula RWSL, wherein R and W are as defined hereinabove and L is a leaving group. The resulting product is treated with an oxidizing agent, preferably a peracid oxidizing agent, more preferably meta-chloroperbenzoic acid, to afford a compound of the formula (I), (1) or (2), or a mixture thereof.

Alternatively, a compound of the formula (I), (1) or (2) may be prepared by reducing the N-oxide of a compound of the formula (I), (1) or (2), or a mixture thereof with a reducing agent, preferably triphenylphosphine. The N-oxide at position 7 of the decahydroisoquinonaphthyridine nucleus may be formed with an appropriate oxidizing agent, preferably hydrogen peroxide. Formation of the N-oxide may be required during the synthesis of the compounds of the formula (I), (1) or (2) to protect position 7 from competing undesirably with reactions being performed on other areas of the molecule.

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the Examples hereinbelow. However, other equivalent separation or isolation procedures could, of course, also be used.

The salt products are also isolated by conventional means. For example, the reaction mixtures may be evaporated to dryness, and the salts can be further purified by conventional methods such as those listed above.

Salts of Compounds of Formula (I), (1) or (2)

The compounds of formula (I), (1) or (2) may be converted to a corresponding acid addition salt by virtue of the presence of the tertiary nitrogen atom.

The conversion is accomplished by treatment with at least a stoichiometric amount of an appropriate acid, such as hydrochloric acid, hydrobromic acid, sulfuric acid, nitric acid, phosphoric acid and the like,

and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained at 0°-50°C. The resulting salt precipitates spontaneously or may be brought out of solution with a less polar solvent.

The acid addition salts of the compounds of formula (I), (1) or (2) may be decomposed to the corresponding free bases by treatment with at least a stoichiometric amount of a suitable base such as sodium or potassium hydroxide, potassium carbonate, sodium bicarbonate, ammonia, and the like.

Salts of the compound of formula (I), (1) or (2) may be interchanged by taking advantage of differential solubilities of the salts, volatilities or activities of the acids, or by treating with the appropriately loaded ion exchange resin. For example, the interchange is effected by the reaction of a salt of the compound of formula (I) with a slight stoichiometric excess of an acid of a lower pKa than the acid component of the starting salt. This conversion is carried out at a temperature between about 0°C and the boiling point of the solvent being used as the medium for the procedure.

In summary, the compounds of the present invention or pharmaceutically acceptable salts thereof are made by the procedures outlined below:

(a) sulfonylating a compound of the formula

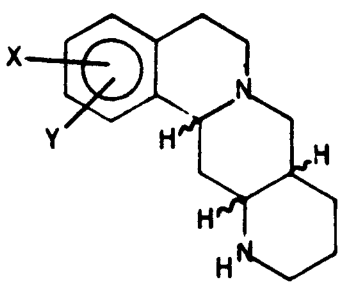

in which:

X and Y are independently hydrogen, hydroxy, lower alkyl of one to six carbon atoms, lower alkoxy of one to six carbon atoms or halo, or X and Y taken together is methylenedioxy or ethylene-1,2-dioxy and the wavy lines indicate that the hydrogen atom attached thereto is in either the α- or β-position;

with a sulfonic acid of the formula $RO_2SOH$ or an activated form thereof, wherein

R is lower alkyl of one to six carbon atoms; phenyl optionally substituted by one or two substituents chosen from halo or amino groups or lower alkyl or lower alkoxy groups of one to four carbon atoms; $-(CH_2)_mOR^1$; or $-NR^1R^2$ wherein m is an integer of 1 to 6 and $R^1$ and $R^2$ are independently hydrogen or lower alkyl, or the group $-NR^1R^2$ forms a heterocycle of the formula:

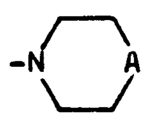

wherein A is $-CH_2-$, $-NR^1-$ or oxygen, wherein $R^1$ has the above meaning; or

(b) reducing a compound of the formula

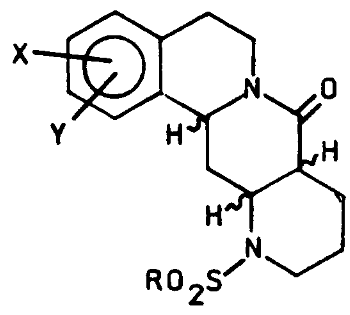

in which:

X, Y, R and the wavy lines are as defined above; or

(c) alkylating a compound of the formula (I) in which:

at least one of X and Y is hydroxy and R and the wavy lines are as defined above, to give a compound of the formula (I)

wherein:

at least one of X and Y is lower alkoxy of one to six carbon atoms or X and Y taken together is methylenedioxy or ethylene-1,2-dioxy; or

(d) oxidizing a compound of the formula

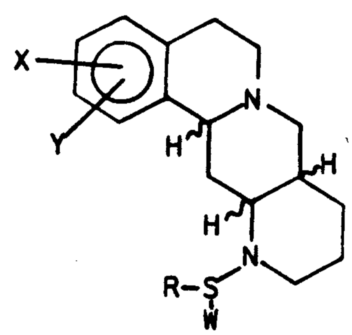

in which X, Y, R and the wavy lines are as defined above and W is an electron pair or an oxygen atom; or

(e) reducing a compound of the formula

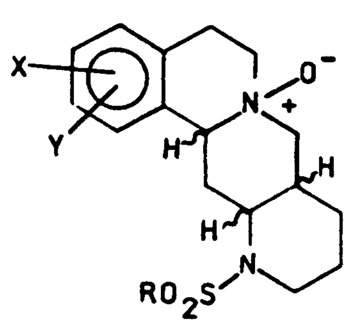

in which X, Y, R and the wavy lines are as defined above; or

(f) epimerizing a compound of the formula

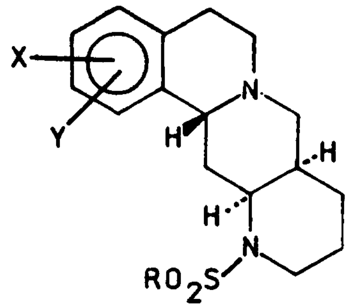

in which X, Y and R are as defined above to a compound of the formula

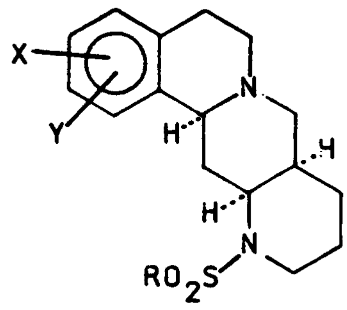

in which X, Y and R are as defined above; or
(g) reducing a compound of the formula

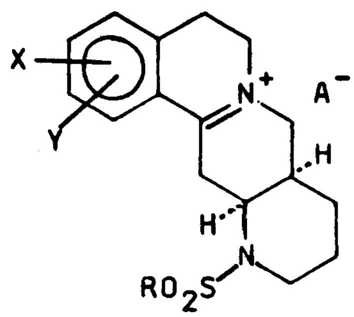

in which X, Y and R are as defined above and A is representative of an organic or inorganic anion, to give a compound of the formula

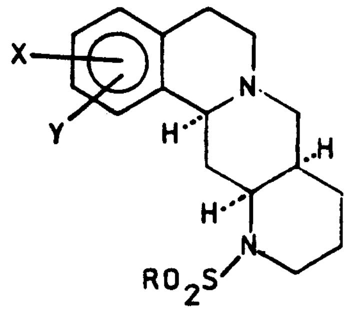

in which X, Y and R are as defined above; or

(h) reducing a compound of the formula (I) in which:

X, Y and the wavy lines are as defined above and R is phenyl substituted by one or two nitro groups, to give a compound of the formula (I) in which:

X, Y and the wavy lines are as defined above and R is phenyl substituted by one or two amine groups; or

(i) dealkylating a compound of the formula (I) in which:

X, Y and the wavy lines are as defined above and R is $-(CH_2)_mOR^1$ wherein m is an integer of 1 to 6 and $R^1$ is lower alkyl,

to give a compound of the formula (I) in which:

X, Y and the wavy lines are as defined above and R is $-(CH_2)_mOH$ wherein m is as defined above; or

(j) alkylating a compound of the formula (I) in which:

X, Y and the wavy lines are as defined above and R is $-(CH_2)_mOH$ wherein m is an integer of 1 to 6,

to give a compound of the formula (I) in which:

X, Y and the wavy lines are as defined above and R is $-(CH_2)_mOR^1$ wherein m is as defined above and $R^1$ is lower alkyl; or

(k) converting a compound of the formula (I) to a salt thereof; or

(l) converting a salt of a compound of the formula (I) to the free compound of the formula (I); or

(m) converting a salt of a compound of the formula (I), preferably a soluble salt, to another salt of the compound of the formula (I), preferably less soluble than said soluble salt; or

(n) resolving a racemic or non-racemic mixture of the compounds of the formulae (1) and (3)

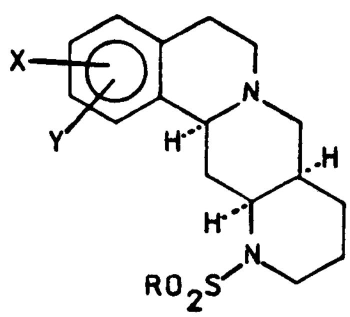

(1)

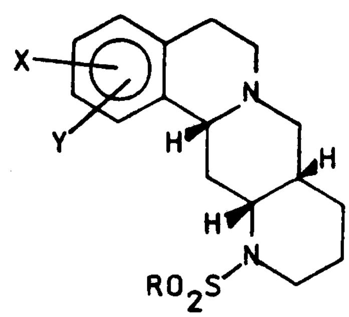

(3)

in which:

X, Y and R are as defined above,

to give an optically enriched compound of the formula (1). Optionally said process steps (a) through (m) being carried out to achieve an optically enriched product.

Methods of Resolution

The compound of formula (I) has three or more asymmetric centers. Accordingly, it may be prepared in either optically active (+) or (-) form or as a (±) racemic mixture. The scope of the invention described and claimed encompasses the individual optical isomers and non-racemic mixtures of the compounds of formula (1) and (2) as well as the racemic forms thereof.

If desired, the compounds herein may be resolved into their optical antipodes by conventional resolution means; for example by separation (e.g. fractional crystallization) of the diastereomeric salts formed by the reaction of these compounds with optically active acids, at temperatures between 0°C and the reflux temperature of the solvent employed for fraction crystallization. Exemplary of such optically active acids are the optically active forms of camphor-10-sulfonic acid, 2-bromo-camphor-10-sulfonic acid, camphoric acid, menthoxyacetic acid, tartaric acid, malic acid, diacetyltartaric acid, pyrrolidine-5-carboxylic acid and the like. The separated pure diastereomeric salts may then be cleaved by standard means, such as treatment with a base, to afford the respective optical isomers of the compounds of formula (1) and (2), for example.

If desired, the racemic mixture of formula (V) may be separated into its two enantiomers. This may be accomplished by conventional resolution means; for example by separation (e.g. fractional crystallization) of the diastereomeric salts formed by the reaction of these compounds with optically active acids at temperatures between 0°C and the reflux temperature of the solvent employed for fractional crystallization. Exemplary of such optically active acids are the optically active forms of camphor-10-sulfonic acid, 2-bromo-camphor-10-sulfonic acid, camphoric acid, menthoxyacetic acid, tartaric acid, malic acid, mandelic acid, diacetyltartaric acid, pyrrolidine-5-carboxylic acid, binaphthyl hydrogen phosphate and the like. The preferred optically active acid is d-camphor-10-sulfonic acid, and an exemplary solvent for recrystallization is a lower alkanol, for example methanol or ethanol, optionally with acetone as a cosolvent. The preferred solvent for recrystallization is ethyl acetate. The separated pure diastereomeric salts may then be cleaved by standard means, such as treatment with a base, to afford the respective enantiomers of the compound of formula (V). Conversion of the appropriate enantiomer of formula (V) to the compounds of formula (1) is then carried out as shown in Schemes I and II.

Preferably, the compound of formula (V) is reacted with a chiral isocyanate to form a mixture of two diastereoisomeric ureas of formula (IX) and (X) according to Scheme IV below wherein $R^*$ represents a chiral moiety; in this case a chiral urea formed by reaction of the secondary amine of the compound of formula (V) with the chiral isocyanate.

Representative chiral urea derivatives have the partial structure

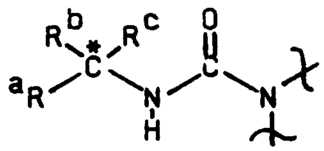

wherein $R^a$, $R^b$ and $R^c$ are different from each other and can be hydrogen, lower alkyl, $C_6$ - $C_{15}$ aryl such as phenyl, $\alpha$- or $\beta$-naphthyl, etc. The lower alkyl of the aryl group may be substituted by one or more substituents not interfering with the preparation of the chiral urea derivative and the subsequent reduction. In general, the chiral urea derivatives will be prepared from conventional resolving agents with a primary amino group.

REACTION SCHEME IV

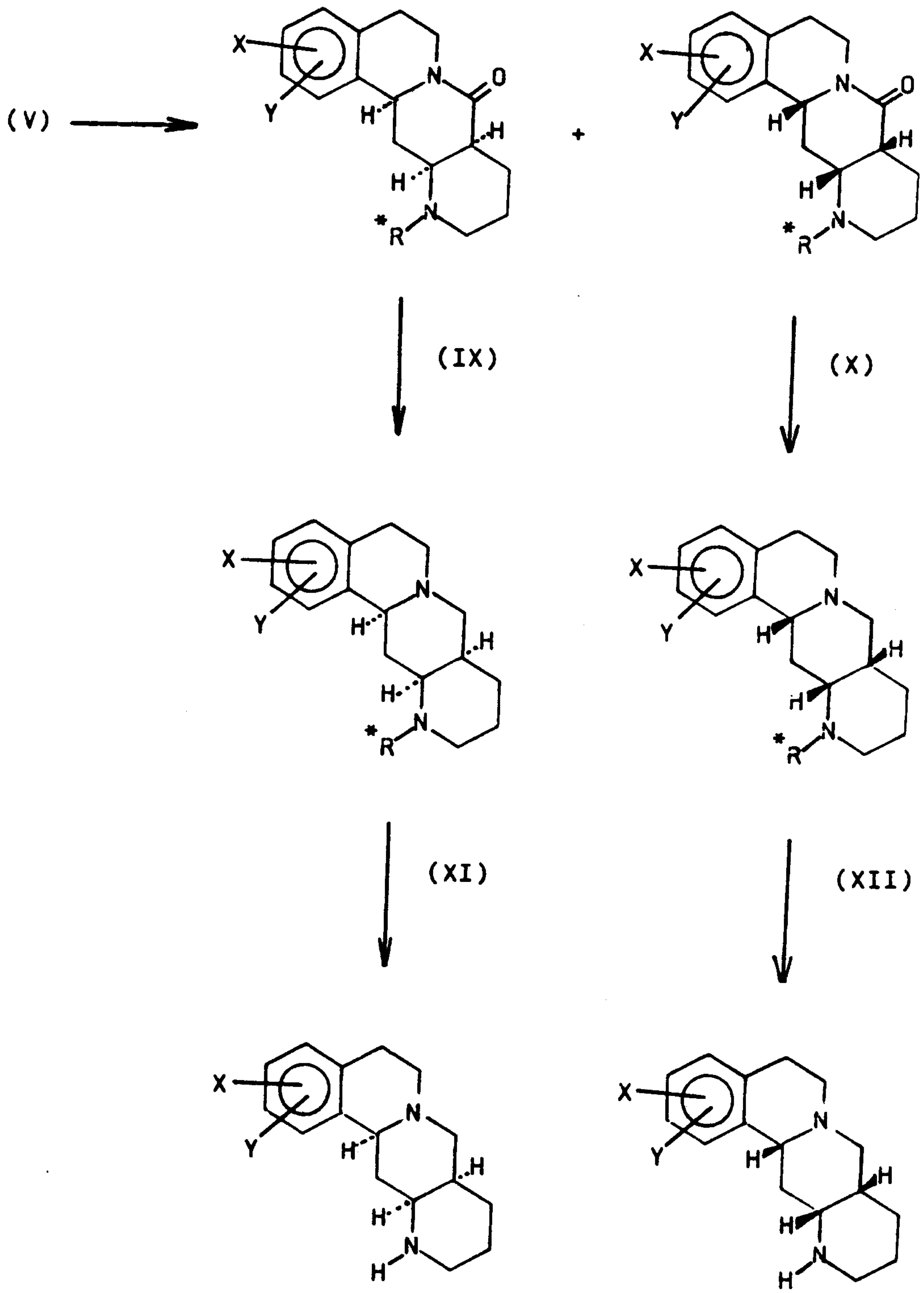


(XIII) (XIV)

For example, the compound of formula (V) is dissolved in an inert solvent, for example benzene, toluene, ethyl acetate, tetrahydrofuran, diethyl ether, chloroform or preferably dichloromethane, containing about 1 molar equivalent of a chiral isocyanate, preferably (R)-(+)-α-methylbenzylisocyanate. The reaction is conducted at a temperature of about 0° to 50°C, preferably about 25°C, for about 5 minutes to 4 hours, preferably about 30 minutes. When the reaction is substantially complete, the mixture of compounds of

formula (IX) and (X) is isolated by conventional means. The two diastereoisomers are then preferably separated by chromatography on silica gel, especially medium pressure chromatography. The first component eluted is the compound of formula (IX), followed by the compound of formula (X).

The compounds of formula (IX) and (X) are then individually reduced to the compounds of formula (XI) and (XII) with a suitable reducing agent, for example borane, triethyloxonium tetrafluoroborate followed by sodium borohydride, sodium borohydride in the presence of a carboxylic acid, or preferably lithium aluminum hydride. For example, a solution of a compound of formula (IX) in an ethereal solvent, preferably tetrahydrofuran, is slowly added to a solution of about 1 to 4 molar equivalents, preferably about 1.5 to 2 molar equivalents, of lithium aluminum hydride in the same ethereal solvent at about 25°C. The mixture is then refluxed for about 1-10 hours, preferably about 3 hours. When the reaction is substantially complete, the compound of formula (XI) is separated and purified by conventional means. In a similar fashion, the compound of formula (X) is reduced to the compound of formula (XII), and likewise separated and purified.

The compounds of formula (XI) and (XII) are then individually hydrolyzed to the enantiomers of formula (XIII) and (XIV). Typically, the compound of formula (XI) or (XII) is dissolved in a protic solvent, for example ethanol, propanol or preferably n-butanol, and added to a solution containing about 4 to 30 molar equivalents, preferably about 10 molar equivalents, of sodium dissolved in the same solvent. The mixture is refluxed for about 1-10 hours, preferably about 4 hours. When the reaction is substantially complete, the compound of formula (XIII) is separated and purified by conventional means, preferably chromatography. In a similar fashion, the compound of formula (XII) is hydrolyzed to the compound of formula (XIV), and likewise separated and purified.

It should be noted that separation into optical isomers which is described above for the separation of the two enantiomers represented by the formula (V) may alternatively be carried out during the subsequent step, by the same procedures as shown above. That is, the compound of formula (V) could first be reduced with, for example, lithium aluminum hydride as shown above to a racemic mixture of the compounds represented by the formulas (XIII) and (XIV), and this racemic mixture similarly separated into its two enantiomers by the methods described supra.

It should be noted that a racemic or non-racemic mixture of the compounds of formula (1) and (3), which would be obtained if no previous separation of optical isomers had been carried out could be separated into its two enantiomers by conventional resolution means, for example by separation (e.g. fractional crystallization) of the diastereomeric salts formed by the reaction of these compounds with optically active acids, or any of the methods described above, especially separation by chromatography on a chiral column. For example, separation of the enantiomers of formula (1) and (3) may be carried out on an $\alpha_1$-acid glycoprotein column eluting with a phosphate buffer composition.

If desired, racemic or non-racemic intermediates of the formulae (V), (V′), (VI), (VI′), (VII) or (VIII), or mixtures thereof, or final product of the formula (1) or (2), or mixtures thereof, prepared herein may be resolved into their optical antipodes by conventional means known in the art, for example, by the separation (e.g., fractional crystallization, chromatography) of the diastereomeric derivatives formed by reaction of, e.g., racemic or non-racemic mixtures of the above-mentioned compounds with an optically active resolving agent. Exemplary optically active resolving agents include optically active carboxylic and phosphoric acids such as the optically active forms of camphor-10-sulfonic acid, 2-bromo-camphor-10-sulfonic acid, camphoric acid, menthoxyacetic acid, tartaric acid, malic acid, mandelic acid, diacetyltartaric acid, pyrrolidine-5-carboxylic acid, binaphthyl hydrogen phosphate and the like. Other methods of resolution include reacting the racemic amine with a chiral acid, for example 2R,3R-(+)-tartaric acid, using methods well known in the art, forming a mixture of two diastereoisomeric amides, which may be separated conventionally, for example by chromatography. Alternatively, reaction with a chiral chloroformate, for example R-(-)-menthyl chloroformate, gives two diastereoisomeric carbamates, or reaction with a chiral isocyanate, for example (R)-(+)-$\alpha$-methylbenzylisocyanate, to form a mixture of two diastereoisomeric ureas; said mixtures may also be separated conventionally.

Racemic mixtures may also may be separated by chromatography on a chiral column. For example, on an $\alpha_1$-acid glycoprotein column eluting with a phosphate buffer composition.

Other methods of enantiomer separation include reacting the racemic amine with a chiral acid, for example 2R,3R-(+)-tartaric acid, using methods well known in the art, forming a mixture of two diastereoisomeric amides, which may be separated conventionally, for example by chromatography. Alternatively, reaction with a chiral chloroformate, for example R-(-)-menthyl chloroformate, gives two diastereoisomeric carbamates, which may also be separated conventionally.

The compound of formula (2) in optically active form is obtained by substituting the compound of formula (VI) for the compound of the formula (V) in Reaction Scheme IV above and carrying out the identical procedures as outlined below.

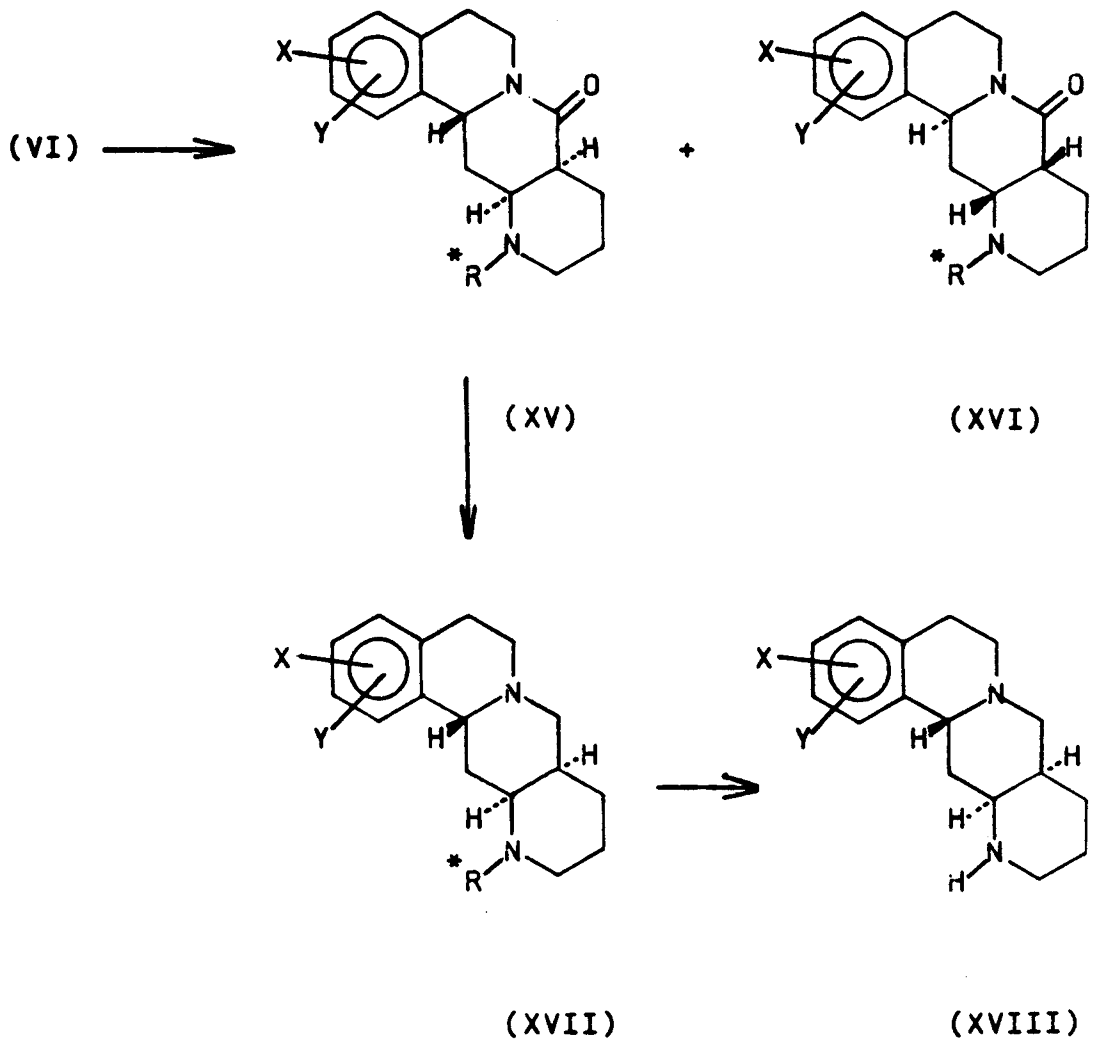

(VI)

(XV)

(XVI)

(XVII)

(XVIII)

Reacting the product of formula (XVIII) thus obtained with a sulfonyl halide as shown in Reaction Scheme II gives a compound of the formula (2).

Utility and Administration:

The compounds of formula (I), preferably the compounds of the formula (1), and the pharmaceutically acceptable acid addition salts thereof have been found to possess valuable pharmacological properties in the central nervous system and, in particular, have been shown to selectively block $\alpha$ -receptors in standard laboratory tests. Accordingly these compounds and pharmaceutically acceptable compositions containing them are useful in the regulation of physiological phenomena related to $\alpha_2$-receptors, including lowering of blood pressure (useful for treating e.g. hypertension), amelioration of depression, inhibition of platelet aggregation, palliation of diabetes, alleviation of male impotence, weight-loss stimulation (useful in treating e.g. obesity). In addition, the compounds of formula (I), preferably the compounds of the formula (1), have been found to be useful for the treatment of irritable-bowel syndrome, cyclic mood disturbances in women, anxiolytic conditions and in the lowering of intraoccular pressure (useful in treating e.g. glaucoma).

In applying the compounds of this invention to treatment of conditions which are regulated by the CNS, administration of the active compounds and salts described herein can be via any of the accepted modes of administration for agents which relieve depression or affect the central nervous system including oral, systemic (e.g., transdermal, intranasal or by suppository) or parenteral (e.g., intramuscular, subcutaneous and intravenous) and otherwise systemic route of administration or topical administration. Any pharmaceutically acceptable mode of administration can be used, including solid, semi-solid or liquid dosage forms, such as, for example, tablets, suppositories, pills, capsules, powders, liquids, suspensions, or the like, preferably in unit dosage forms suitable for single administration of precise dosages, or in sustained or

controlled release dosage forms for the prolonged administration of the compound at a predetermined rate. The compositions will typically include a conventional pharmaceutical carrier or excipient and an active compound of formula (I), preferably an active compound of formula (1), or the pharmaceutically acceptable salts thereof and, in addition, may include other medicinal agents, pharmaceutical agents, carriers, adjuvants, etc.

The amount of active compound administered will of course, be dependent on the subject being treated, the severity of the affliction, the manner of administration and the judgement of the prescribing physician. However, an effective dosage is in the range of 0.0001-10 mg/kg/day, preferably 0.01-1 mg/kg/day, more preferably 0.1-0.5 mg/kg/day. For an average 70 kg human, this would amount to 0.007-700 mg per day, preferably 0.7-70 mg per day, or preferably 7-35 mg per day.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see Remington's Pharmaceutical Sciences, Mack Publishing Company, Easton, Pennsylvania, 16th Edition, 1980. The composition or formulation to be administered will, in any event, contain a quantity of the active compound(s) in an amount effective to alleviate the symptoms of the subject being treated.

Dosage forms of compositions containing active ingredient (compounds of formula (I), preferably compounds of formula (1), or its salts) in the range of 0.25 to 95% with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium, carbonate, and the like. Such compositions take the form of solutions, suspensions, tablets, pills, capsules, powders, sustained release formulations and the like. Such compositions may contain 1%-95% active ingredient, preferably 5-50%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, etc.

A more recently devised approach for parenteral administration employs the implantation of a slow-release or sustained-release system, such that a constant level of dosage is maintained. See, e.g., U.S. Patent No. 3,710,795.

The percentage of active compound contained in such parenteral compositions is highly dependent on the specific nature thereof, as well as the activity of the compound and the needs of the subject. However, percentages of active ingredient of 0.1% to 10% in solution are employable, and will be higher if the composition is a solid which will be subsequently diluted to the above percentages. Preferably the composition will comprise 0.2-2% of the active agent in solution.

For systemic administration via suppository, traditional binders and carriers include, e.g. polyalkalene glycols or triglycerides. Such suppositories may be formed from mixtures containing active ingredient in the range of 0.5%-10%; preferably 1-2%.

In applying the compounds of the invention to treatment of diseases or disorders of the eye which are associated with an abnormally high intraoccular pressure, administration may be achieved by any pharmaceutically acceptable mode of administration which provides adequate local concentrations to provide the desired response. These include direct administration to the eye via drops and controlled release inserts or implants, as well as systemic administration as previously described.

Drops and solutions applied directly to the eye are typically sterilized aqueous solutions containing 0.001% to 10%, most preferably 0.005% to 1% of the active ingredient, along with suitable buffer, stabilizer, and preservative. The total concentration of solutes should be such that, if possible, the resulting solution is isotonic with the lacrimal fluid (though this is not absolutely necessary) and has an equivalent pH in the range of pH 6-8. Typical sterilants are phenyl mercuric acetate, thimerosal, chlorobutanol, and benzalconium chloride. Typical buffer systems and salts are based on, for example, citrate, borate or phosphate; suitable stabilizers include glycerin and polysorbate 80. The aqueous solutions are formulated simply by dissolving the solutes in a suitable quantity of water, adjusting the pH to about 6.8-8.0, making a final volume adjustment with additional water, and sterilizing the preparation using methods known to those skilled in the art.

The dosage level of the resulting composition will, of course, depend on the concentration of the drops, the condition of the subject and the individual magnitude of responses to treatment. However, a typical occular composition could be administered at the rate of about 2-10 drops per day per eye of a 0.1% solution of active ingredient.

The compositions of the present invention may also be formulated for administration in any convenient way by analogy with other topical compositions adapted for use in mammals. These compositions may be presented for use in any conventional manner with the aid of any of a wide variety of pharmaceutical carriers or vehicles. For such topical administration, a pharmaceutically acceptable non-toxic formulation can take the form of semisolid, liquid, or solid, such as, for example, gels, creams, lotions, solutions, suspensions, ointments, powders, or the like. As an example, the active components may be formulated into a gel using ethanol, propylene glycol, propylene carbonate, polyethylene glycols, diisopropyl adipate, glycerol, water, etc., with appropriate gelling agents, such as Carbomers, Klucels, etc. If desired, the formulation may also contain minor amounts of non-toxic auxiliary substances such as preservatives, antioxidants, pH buffering agents, surface active agents, and the like. Actual methods of preparing such dosage forms are known, or will be apparent, to those skilled in the art; for example, see Remington's Pharmaceutical Sciences, Mack Publishing Company, Easton, Pennsylvania, 16th Edition, 1980.

Embodiments of the invention are described below by way of example only.

## PREPARATION 1

### Preparation of (±)-5,6,13,13a-Tetrahydroisoguino[2,1-g][1,6]naphthyridin-8-one hydrochloride and Related Compounds of Formula (IV).

A. Diisopropylamine (28 ml) and 150 ml of tetrahydrofuran were cooled to -65 °C and 125 mL of 1.6 M n-butyllithium was added. To the resulting solution was added a solution of 16.2 g of 3,4-dihydroisoquinoline and 38.4 g of 2-methylnicotinic acid diethylamide in tetrahydrofuran. The mixture was allowed to warm to -20 °C and 600 ml of 3N hydrochloric acid was then added followed by 200 ml of water. The mixture was basified with $NH_4OH$ and extracted twice with ether. The ether extracts were combined, dried over anhydrous magnesium sulfate and evaporated to a residue, which was dissolved in methanol and acidified with anhydrous HCl in ether. Acetone (50 ml) was added and the mixture was allowed to stand overnight. The crystalline product was collected by filtration, yielding 34 g of (±)-5,6,13,13a-tetrahydroisoquino[2,1-g[[1,6]naphthyridine-8-one hydrochloride, m.p. 220-222 °C.

An additional 7.5 g of the title compound as the free base was obtained by evaporation of the mother liquor followed by partitioning between ether and aqueous $NH_4OH$ and silica gel chromatography of the residue obtained from evaporation of the ether, eluting with ethyl acetate, giving the free base, m.p. 72-73 °C.

B. Similarly, replacing 3,4-dihydroisoquinoline with the appropriate compound of formula (III) and following the procedure in paragraph A above, the following compounds of formula (IV) were prepared:

(±)-3-methoxy-5,6,13,13a-tetrahydroisoquino[2,1-g][1,6]naphthyridine-8-one hydrochloride, m.p. 244-246 °C;

(±)-3-dimethoxy-5,6,13,13a-tetrahydroisoquino[2,1-g][1,6]naphthyridine-8-one, m.p. 115-116 °C;

(±)-2,3-dimethoxy-5,6,13,13a-tetrahydroisoquino[2,1-g][1,6]naphthyridine-8-one hydrochloride, m.p. 238-240 °C;

(±)-1,4-dimethoxy-5,6,13,13a-tetrahydroisoquino[2,1-g][1,6]naphthyridine-8-one hydrochloride;

(±)-2,3-methylenedioxy-5,6,13,13a-tetrahydroisoquino[2,1-g][1,6]naphthyridine-8-one, m.p. 177-179 °C; and

(±)-2,3-(ethylene-1,2-dioxy)-5,6,13,13a-tetrahydroisoquino[2,1-g][1,6]naphthyridine-8-one.

C. Similarly, replacing 3,4-dihydroisoquinoline with other compounds of formula (III) and following the procedure in paragraph A above, the following exemplary compounds of formula (IV) are prepared:

(±)-1-methyl-5,6,13,13a-tetrahydroisoquino[2,1-g][1,6]naphthyridine-8-one hydrochloride;
(±)-2-methyl-5,6,13,13a-tetrahydroisoquino[2,1-g][1,6]naphthyridine-8-one hydrochloride;
(±)-3-methyl-5,6,13,13a-tetrahydroisoquino[2,1-g][1,6]naphthyridine-8-one hydrochloride;
(±)-2,3-dimethyl-5,6,13,13a-tetrahydroisoquino[2,1-g][1,6]naphthyridine-8-one hydrochloride;
(±)-3-ethyl-5,6,13,13a-tetrahydroisoquino[2,1-g][1,6]naphthyridine-8-one hydrochloride;
(±)-3-isobutyl-5,6,13,13a-tetrahydroisoquino[2,1-g][1,6]naphthyridine-8-one hydrochloride;
(±)-3-n-hexyl-5,6,13,13a-tetrahydroisoquino[2,1-g][1,6]naphthyridine-8-one hydrochloride;
(±)-1-methoxy-5,6,13,13a-tetrahydroisoquino[2,1-g][1,6]naphthyridine-8-one hydrochloride;
(±)-2-methoxy-5,6,13,13a-tetrahydroisoquino[2,1-g][1,6]naphthyridine-8-one hydrochloride;
(±)-4-methoxy-5,6,13,13a-tetrahydroisoquino[2,1-g][1,6]naphthyridine-8-one hydrochloride;
(±)-3-methoxy-2-methyl-5,6,13,13a-tetrahydroisoquino[2,1-g][1,6]naphthyridine-8-one hydrochloride;
(±)-3-ethoxy-5,6,13,13a-tetrahydroisoquino[2,1-g][1,6]naphthyridine-8-one hydrochloride;
(±)-3-isopropoxy-5,6,13,13a-tetrahydroisoquino[2,1-g][1,6]naphthyridine-8-one hydrochloride;
(±)-3-isobutoxy-5,6,13,13a-tetrahydroisoquino[2,1-g][1,6]naphthyridine-8-one hydrochloride;
(±)-3-n-hexyloxy-5,6,13,13a-tetrahydroisoquino[2,1-g][1,6]naphthyridine-8-one hydrochloride;
(±)-3-hydroxy-5,6,13,13a-tetrahydroisoquino[2,1-g][1,6]naphthyridine-8-one hydrochloride;
(±)-2,3-dihydroxy-5,6,13,13a-tetrahydroisoquino[2,1-g][1,6]naphthyridine-8-one hydrochloride;
(±)-1,2-dimethoxy-5,6,13,13a-tetrahydroisoquino[2,1-g][1,6]naphthyridine-8-one hydrochloride;
(±)-1,4-dimethoxy-5,6,13,13a-tetrahydroisoquino[2,1-g][1,6]naphthyridine-8-one hydrochloride;
(±)-3,4-dimethoxy-5,6,13,13a-tetrahydroisoquino[2,1-g][1,6]naphthyridine-8-one hydrochloride;
(±)-2,3-diethoxy-5,6,13,13a-tetrahydroisoquino[2,1-g][1,6]naphthyridine-8-one hydrochloride;
(±)-2,3-di-n-butoxy-5,6,13,13a-tetrahydroisoquino[2,1-g][1,6]naphthyridine-8-one hydrochloride;
(±)-1,2-methylenedioxy-5,6,13,13a-tetrahydroisoquino[2,1-g][1,6]naphthyridine-8-one hydrochloride;
(±)-3,4-methylenedioxy-5,6,13,13a-tetrahydroisoquino[2,1-g][1,6]naphthyridine-8-one hydrochloride;
(±)-1-chloro-5,6,13,13a-tetrahydroisoquino[2,1-g][1,6]naphthyridine-8-one hydrochloride;
(±)-2-chloro-5,6,13,13a-tetrahydroisoquino[2,1-g][1,6]naphthyridine-8-one hydrochloride;
(±)-3-chloro-5,6,13,13a-tetrahydroisoquino[2,1-g][1,6]naphthyridine-8-one hydrochloride;
(±)-4-chloro-5,6,13,13a-tetrahydroisoquino[2,1-g][1,6]naphthyridine-8-one hydrochloride;
(±)-3-bromo-5,6,13,13a-tetrahydroisoquino[2,1-g][1,6]naphthyridine-8-one hydrochloride;
(±)-3-fluoro-5,6,13,13a-tetrahydroisoquino[2,1-g][1,6]naphthyridine-8-one hydrochloride; and
(±)-2-fluoro-5,6,13,13a-tetrahydroisoquino[2,1-g][1,6]naphthyridine-8-one hydrochloride.

## PREPARATION 2A

Preparation of (±)-5,6,8aα,9,10,11,12,12aα,13,13aα-decahydroisoquino[2,1-g][1,6]naphthyridin-8-one (V) and (±)- 5,6,8aβ,9,10,11,12,12aβ,13,13aα-Decahydroisoquino[2,1-g][1,6]naphthyridin-8-one (VI) and Related Compounds of Formula (V) and (VI).

A. A mixture of 30 g of (±)-5,6,13,13a-tetrahydroisoquino[2,1-g][1,6]naphthyridine-8-one hydrochloride, prepared as shown in Preparation 1 above, and 7.5 g of 5% $Rh\text{-}Al_2O_3$ in 300 ml of acetic acid was hydrogenated at 50 psi (3.45 bar) for 42 hours. The catalyst was removed by filtration and the filtrate was concentrated under reduced pressure. The residue was partitioned between methylene chloride and aqueous $NH_4OH$ and the methylene chloride layer was separated and the solvent removed under reduced pressure. The residue was purified by silica gel chromatography, eluting with from 5-20% methanol in methylene chloride. The first component eluted was (±)-5,6,8aβ,9,10,11,12,12aβ,13,13aα-decahydroisoquino[2,1-g][1,6]naphthyridin-8-one (9.7 g), (VI), m.p. 105-106 °C. The second component eluted was (±)-5,6,8aα,9,10,11,12,12aα,13,13aα-decahydroisoquino[2,1-g][1,6]naphthyridin-8-one (11.0 g), (V), m.p. 91-92 °C.

B. Similarly, replacing (±)-5,6,13,13a-tetrahydroisoquino[2,1-g][1,6]naphthyridine-8-one hydrochloride with the appropriate compound of formula (IV) and following the procedure in paragraph A above, the following compounds of formula (V) and (VI) were prepared:

(±)-3-methoxy-5,6,8aβ,9,10,11,12,12aβ,13,13aα-decahydroisoquino[2,1-g][1,6]naphthyridin-8-one; and
(±)-3-methoxy-5,6,8aα,9,10,11,12,12aα,13,13aα-decahydroisoquino[2,1-g][1,6]naphthyridin-8-one, m.p. 118-119 °C;
(±)-2,3-dimethoxy-5,6,8aβ,9,10,11,12,12aβ,13,13aα-decahydroisoquino[2,1-g][1,6]naphthyridin-8-one; and

(±)-2,3-dimethoxy-5,6,8aα,9,10,11,12,12aα,13,13aα-decahydroisoquino[2,1-g][1,6]naphthyridin-8-one;
(±)-1,4-dimethoxy-5,6,8aβ,9,10,11,12,12aβ,13,13aα-decahydroisoquino[2,1-g][1,6]naphthyridin-8-one; and
(±)-1,4-dimethoxy-5,6,8aα,9,10,11,12,12aα,13,13aα-decahydroisoquino[2,1-g][1,6]naphthyridin-8-one;
(±)-2,3-methylenedioxy-5,6,8aβ,9,10,11,12,12aβ,13,13aα-decahydroisoquino[2,1-g][1,6]naphthyridin-8-one; and
(±)-2,3-methylenedioxy-5,6,8aα,9,10,11,12,12aα,13,13aα-decahydroisoquino[2,1-g][1,6]naphthyridin-8-one;
(±)-2,3-(ethylene-1,2-dioxy)-5,6,8aβ,9,10,11,12,12aβ,13,13aα-decahydroisoquino[2,1-g][1,6]-naphthyridin-8-one; and
(±)-2,3-(ethylene-1,2-dioxy)-5,6,8aα,9,10,11,12,12aα,13,13aα-decahydroisoquino[2,1-g][1,6]-naphthyridin-8-one;

C. Similarly, replacing (±)-5,6,13,13a-tetrahydroisoquino[2,1-g][1,6]naphthyridine-8-one hydrochloride with other compounds of formula (IV) and following the procedure in paragraph A above, the following exemplary compounds of formula (V) and (VI) are prepared:

(±)-1-methyl-5,6,8aβ,9,10,11,12,12aβ,13,13aα-decahydroisoquino[2,1-g][1,6]naphthyridin-8-one; and
(±)-1-methyl-5,6,8aα,9,10,11,12,12aα,13,13aα-decahydroisoquino[2,1-g][1,6]naphthyridin-8-one;
(±)-2-methyl-5,6,8aβ,9,10,11,12,12aβ,13,13aα-decahydroisoquino[2,1-g][1,6]naphthyridin-8-one; and
(±)-2-methyl-5,6,8aα,9,10,11,12,12aα,13,13aα-decahydroisoquino[2,1-g][1,6]naphthyridin-8-one;
(±)-3-methyl-5,6,8aβ,9,10,11,12,12aβ,13,13aα-decahydroisoquino[2,1-g][1,6]naphthyridin-8-one; and
(±)-3-methyl-5,6,8aα,9,10,11,12,12aα,13,13aα-decahydroisoquino[2,1-g][1,6]naphthyridin-8-one;
(±)-2,3-dimethyl-5,6,8aβ,9,10,11,12,12aβ,13,13aα-decahydroisoquino[2,1-g][1,6]naphthyridin-8-one; and
(±)-2,3-dimethyl-5,6,8aα,9,10,11,12,12aα,13,13aα-decahydroisoquino[2,1-g][1,6]naphthyridin-8-one;
(±)-3-ethyl-5,6,8aβ,9,10,11,12,12aβ,13,13aα-decahydroisoquino[2,1-g][1,6]naphthyridin-8-one; and
(±)-3-ethyl-5,6,8aα,9,10,11,12,12aα,13,13aα-decahydroisoquino[2,1-g][1,6]naphthyridin-8-one;
(±)-3-isobutyl-5,6,8aβ,9,10,11,12,12aβ,13,13aα-decahydroisoquino[2,1-g][1,6]naphthyridin-8-one; and
(±)-3-isobutyl-5,6,8aα,9,10,11,12,12aα,13,13aα-decahydroisoquino[2,1-g][1,6]naphthyridin-8-one;
(±)-3-n-hexyl-5,6,8aβ,9,10,11,12,12aβ,13,13aα-decahydroisoquino[2,1-g][1,6]naphthyridin-8-one; and
(±)-3-n-hexyl-5,6,8aα,9,10,11,12,12aα,13,13aα-decahydroisoquino[2,1-g][1,6]naphthyridin-8-one;
(±)-1-methoxy-5,6,8aβ,9,10,11,12,12aβ,13,13aα-decahydroisoquino[2,1-g][1,6]naphthyridin-8-one; and
(±)-1-methoxy-5,6,8aα,9,10,11,12,12aα,13,13aα-decahydroisoquino[2,1-g][1,6]naphthyridin-8-one;
(±)-2-methoxy-5,6,8aβ,9,10,11,12,12aβ,13,13aα-decahydroisoquino[2,1-g][1,6]naphthyridin-8-one; and
(±)-2-methoxy-5,6,8aα,9,10,11,12,12aα,13,13aα-decahydroisoquino[2,1-g][1,6]naphthyridin-8-one;
(±)-4-methoxy-5,6,8aβ,9,10,11,12,12aβ,13,13aα-decahydroisoquino[2,1-g][1,6]naphthyridin-8-one; and
(±)-4-methoxy-5,6,8aα,9,10,11,12,12aα,13,13aα-decahydroisoquino[2,1-g][1,6]naphthyridin-8-one;
(±)-3-methoxy-2-methyl-5,6,8aβ,9,10,11,12,12aβ,13,13aα-decahydroisoquino[2,1-g][1,6]naphthyridin-8-one; and
(±)-3-methoxy-2-methyl-5,6,8aα,9,10,11,12,12aα,13,13aα-decahydroisoquino[2,1-g][1,6]naphthyridin-8-one;
(±)-3-ethoxy-5,6,8aβ,9,10,11,12,12aβ,13,13aα-decahydroisoquino[2,1-g][1,6]naphthyridin-8-one; and
(±)-3-ethoxy-5,6,8aα,9,10,11,12,12aα,13,13aα-decahydroisoquino[2,1-g][1,6]naphthyridin-8-one;
(±)-3-isopropoxy-5,6,8aβ,9,10,11,12,12aβ,13,13aα-decahydroisoquino[2,1-g][1,6]naphthyridin-8-one; and
(±)-3-isopropoxy-5,6,8aα,9,10,11,12,12aα,13,13aα-decahydroisoquino[2,1-g][1,6]naphthyridin-8-one;
(±)-3-isobutoxy-5,6,8aβ,9,10,11,12,12aβ,13,13aα-decahydroisoquino[2,1-g][1,6]naphthyridin-8-one; and
(±)-3-isobutoxy-5,6,8aα,9,10,11,12,12aα,13,13aα-decahydroisoquino[2,1-g][1,6]naphthyridin-8-one;
(±)-3-n-hexyloxy-5,6,8aβ,9,10,11,12,12aβ,13,13aα-decahydroisoquino[2,1-g][1,6]naphthyridin-8-one; and
(±)-3-n-hexyloxy-5,6,8aα,9,10,11,12,12aα,13,13aα-decahydroisoquino[2,1-g][1,6]naphthyridin-8-one;
(±)-3-hydroxy-5,6,8aβ,9,10,11,12,12aβ,13,13aα-decahydroisoquino[2,1-g][1,6]naphthyridin-8-one; and
(±)-3-hydroxy-5,6,8aα,9,10,11,12,12aα,13,13aα-decahydroisoquino[2,1-g][1,6]naphthyridin-8-one;
(±)-2,3-dihydroxy-5,6,8aβ,9,10,11,12,12aβ,13,13aα-decahydroisoquino[2,1-g][1,6]naphthyridin-8-one; and
(±)-2,3-dihydroxy-5,6,8aα,9,10,11,12,12aα,13,13aα-decahydroisoquino[2,1-g][1,6]naphthyridin-8-one;
(±)-1,2-dimethoxy-5,6,8aβ,9,10,11,12,12aβ,13,13aα-decahydroisoquino[2,1-g][1,6]naphthyridin-8-one; and

(±)-1,2-dimethoxy-5,6,8aα,9,10,11,12,12aα,13,13aα-decahydroisoquino[2,1-g][1,6]naphthyridin-8-one;
(±)-1,4-dimethoxy-5,6,8aβ,9,10,11,12,12aβ,13,13aα-decahydroisoquino[2,1-g][1,6]naphthyridin-8-one; and
(±)-1,4-dimethoxy-5,6,8aα,9,10,11,12,12aα,13,13aα-decahydroisoquino[2,1-g][1,6]naphthyridin-8-one;
(±)-3,4-dimethoxy-5,6,8aβ,9,10,11,12,12aβ,13,13aα-decahydroisoquino[2,1-g][1,6]naphthyridin-8-one; and
(±)-3,4-dimethoxy-5,6,8aα,9,10,11,12,12aα,13,13aα-decahydroisoquino[2,1-g][1,6]naphthyridin-8-one;
(±)-2,3-diethoxy-5,6,8aβ,9,10,11,12,12aβ,13,13aα-decahydroisoquino[2,1-g][1,6]naphthyridin-8-one; and
(±)-2,3-diethoxy-5,6,8aα,9,10,11,12,12aα,13,13aα-decahydroisoquino[2,1-g][1,6]naphthyridin-8-one;
(±)-2,3-di-n-butoxy-5,6,8aβ,9,10,11,12,12aβ,13,13aα-decahydroisoquino[2,1-g][1,6]naphthyridin-8-one; and
(±)-2,3-di-n-butoxy-5,6,8aα,9,10,11,12,12aα,13,13aα-decahydroisoquino[2,1-g][1,6]naphthyridin-8-one;
(±)-1,2-methylenedioxy-5,6,8aβ,9,10,11,12,12aβ,13,13aα-decahydroisoquino[2,1-g][1,6]naphthyridin-8-one; and
(±)-1,2-methylenedioxy-5,6,8aα,9,10,11,12,12aα,13,13aα-decahydroisoquino[2,1-g][1,6]naphthyridin-8-one;
(±)-2-chloro-5,6,8aβ,9,10,11,12,12aβ,13,13aα-decahydroisoquino[2,1-g][1,6]naphthyridin-8-one; and
(±)-2-chloro-5,6,8aα,9,10,11,12,12aα,13,13aα-decahydroisoquino[2,1-g][1,6]naphthyridin-8-one;
(±)-3-chloro-5,6,8aβ,9,10,11,12,12aβ,13,13aα-decahydroisoquino[2,1-g][1,6]naphthyridin-8-one; and
(±)-3-chloro-5,6,8aα,9,10,11,12,12aα,13,13aα-decahydroisoquino[2,1-g][1,6]naphthyridin-8-one;
(±)-4-chloro-5,6,8aβ,9,10,11,12,12aβ,13,13aα-decahydroisoquino[2,1-g][1,6]naphthyridin-8-one; and
(±)-4-chloro-5,6,8aα,9,10,11,12,12aα,13,13aα-decahydroisoquino[2,1-g][1,6]naphthyridin-8-one;
(±)-3-bromo-5,6,8aβ,9,10,11,12,12aβ,13,13aα-decahydroisoquino[2,1-g][1,6]naphthyridin-8-one; and
(±)-3-bromo-5,6,8aα,9,10,11,12,12aα,13,13aα-decahydroisoquino[2,1-g][1,6]naphthyridin-8-one;
(±)-3-fluoro-5,6,8aβ,9,10,11,12,12aβ,13,13aα-decahydroisoquino[2,1-g][1,6]naphthyridin-8-one; and
(±)-3-fluoro-5,6,8aα,9,10,11,12,12aα,13,13aα-decahydroisoquino[2,1-g][1,6]naphthyridin-8-one;
(±)-2-fluoro-5,6,8aβ,9,10,11,12,12aβ,13,13aα-decahydroisoquino[2,1-g][1,6]naphthyridin-8-one; and
(±)-2-fluoro-5,6,8aα,9,10,11,12,12aα,13,13aα-decahydroisoquino[2,1-g][1,6]naphthyridin-8-one;

## PREPARATION 2B

Preparation of (8aS,12aS,13aS)-3-methoxy-5,6,8a,9,10,11,12,12a,13,13a-decahydroisoquino[2,1-g][1,6]-naphthyridin-8-one (V) and Related Compounds of Formula (V).

A. To a slurry of 1.16 g of (±)-3-methoxy-5,6,8aα,9,10,11,12,12aα,13,13aα-decahydroisoquino[2,1-g][1,6]-naphthyridin-8-one in 36.3 mL of isopropyl alcohol was added 0.94 g (1.00 equivalents) of d-camphorsulfonic acid. The slurry was stirred and heated at reflux for 2 h, then cooled to room temperature over 0.5 h. After an additional 2 h at room temperature, the crystalline product was removed by filtration, washed with acetone and air dried to afford 1.34 g of product (63.8% yield). Two successive recrystallizations of this material from isopropyl alcohol provided 0.86 g (42.8% yield) of the d-camphorsulfonic acid addition salt of the 8aS,12aS,13aS-enantiomer of (V) (enantiomeric excess, 97%); m.p. >280 °C, $[\alpha]_D^{25}$ = -44.44 ° (c = 1.0, $CHCl_3$). The salt was converted to the free base under standard conditions to provide (8aS,12aS,13aS)-3-methoxy-5,6,8a,9,10,11,12,12a,13,13a-decahydroisoquino[2,1-g][1,6]naphthyridin-8-one.

B. Similarly, replacing (±)-3-methoxy-5,6,8aα,9,10,11,12,12aα,13,13aα-decahydroiso-quino[2,1-g][1,6]-naphthyridin-8-one with the appropriate compound of formula (V) and following the procedure in paragraph A above, the following compounds were prepared:
(8aS,12aS,13aS)-5,6,8a,9,10,11,12,12a,13,13-decahydro-isoquino[2,1-g][1,6]naphthyridin-8-one;
(8aS,12aS,13aS)-2,3-dimethoxy-5,6,8a,9,10,11,12,12a,13,13a-decahydroisoquino[2,1-g][1,6]-naphthyridin-8-one; and
(8aS,12aS,13aS)-1,4-dimethoxy-5,6,8a,9,10,11,12,12a,13,13a-decahydroisoquino[2,1-g][1,6]-naphthyridin-8-one; and
(8aS,12aS,13aS)-2,3-methylenedioxy-5,6,8a,9,10,11,12,12a,13,13a-decahydroisoquino[2,1-g][1,6]-naphthyridin-8-one; and
(8aS,12aS,13aS)-2,3-(ethylene-1,2-dioxy)-5,6,8a,9,10,11,12,12a,13,13a-decahydroisoquino[2,1-g][1,6]-naphthyridin-8-one.

PREPARATION 3A

Preparation of (±)-5,6,8aα,9,10,11,12,12aα,13,13aα-Decahydro-8H-isoquino[2,1g][1,6]naphthyridine and Related Compounds of formula (VII) and (VIII)

A. A solution of 9.6 g of (±)-5,6,8aα,9,10,11,12,12aα,13,13aα-decahydroisoquino[2,1-g][1,6]naphthyridin-8-one (V), prepared as shown in Preparation 2, in 50 ml of tetrahydrofuran was added slowly to a solution of 2.5 g of lithium aluminum hydride in 75 ml of tetrahydrofuran. The resulting mixture was stirred at reflux for 3 hours, cooled, and treated sequentially with 2.5 ml of water, 2.5 ml of 15% sodium hydroxide, and 7.5 ml of water. The mixture was filtered and the filtrate was evaporated to afford 8.8 g of (±)-5,6,8aα,9,10,11,12,12aα,13,13aα-decahydro-8H-isoquino[2,1-g][1,6]naphthyridine (VII) as a thick oil. The oil was dissolved in ethanol and acidified with anhydrous HCl in ether, from which a dihydrochloride salt was crystallized, m.p. 290-295 °C.

B. Similarly, replacing (±)-5,6,8aα,9,10,11,12,12aα,13,13aα-decahydroisoquino[2,1-g][1,6]naphthyridin-8-one with the appropriate compound of formula (V) or (VI) and following the procedure in paragraph A above, the following compounds of formula (VII) and (VIII) were prepared:

(±)-5,6,8aα,9,10,11,12,12aα,13,13aα-decahydro-8H-isoquino[2,1-g][1,6]naphthyridine;
(±)-3-methoxy-5,6,8aβ,9,10,11,12,12aβ,13,13aα-decahydro-8H-isoquino[2,1-g][1,6]naphthyridine;
(±)-3-methoxy-5,6,8aα,9,10,11,12,12aα,13,13aα-decahydro-8H-isoquino[2,1-g][1,6]naphthyridine;
(±)-2,3-dimethoxy-5,6,8aβ,9,10,11,12,12aβ,13,13aα-decahydro-8H-isoquino[2,1-g][1,6]naphthyridine;
(±)-2,3-dimethoxy-5,6,8aα,9,10,11,12,12aα,13,13aα-decahydro-8H-isoquino[2,1-g][1,6]naphthyridine;
(±)-1,4-dimethoxy-5,6,8aβ,9,10,11,12,12aβ,13,13aα-decahydro-8H-isoquino[2,1-g][1,6]naphthyridine;
(±)-1,4-dimethoxy-5,6,8aα,9,10,11,12,12aα,13,13aα-decahydro-8H-isoquino[2,1-g][1,6]naphthyridine;
(±)-2,3-methylenedioxy-5,6,8aβ,9,10,11,12,12aβ,13,13aα-decahydro-8H-isoquino[2,1-g][1,6]-naphthyridine; and
(±)-2,3-methylenedioxy-5,6,8aα,9,10,11,12,12aα,13,13aα-decahydro-8H-isoquino[2,1-g][1,6]-naphthyridine;
(±)-2,3-(ethylene-1,2-dioxy)-5,6,8aβ,9,10,11,12,12aβ,13,13aα-decahydro-8H-isoquino[2,1-g][1,6]-naphthyridine; and
(±)-2,3-(ethylene-1,2-dioxy)-5,6,8aα,9,10,11,12,12aα,13,13aα-decahydro-8H-isoquino[2,1-g][1,6]-naphthyridine;

C. Similarly, replacing (±)-5,6,8aα,9,10,11,12,12aα,13,13aα-decahydroisoquino[2,1-g][1,6]naphthyridin-8-one with other compounds of formula (V) or (VI) and following the procedure in paragraph A above, the following exemplary compounds of formula (VII) and (VIII) are prepared:

(±)-1-methyl-5,6,8aβ,9,10,11,12,12aβ,13,13aα-decahydro-8H-isoquino[2,1-g][1,6]naphthyridine; and
(±)-1-methyl-5,6,8aα,9,10,11,12,12aα,13,13aα-decahydro-8H-isoquino[2,1-g][1,6]naphthyridine;
(±)-2-methyl-5,6,8aβ,9,10,11,12,12aβ,13,13aα-decahydro-8H-isoquino[2,1-g][1,6]naphthyridine; and
(±)-2-methyl-5,6,8aα,9,10,11,12,12aα,13,13aα-decahydro-8H-isoquino[2,1-g][1,6]naphthyridine;
(±)-3-methyl-5,6,8aβ,9,10,11,12,12aβ,13,13aα-decahydro-8H-isoquino[2,1-g][1,6]naphthyridine; and
(±)-3-methyl-5,6,8aα,9,10,11,12,12aα,13,13aα-decahydro-8H-isoquino[2,1-g][1,6]naphthyridine;
(±)-2,3-dimethyl-5,6,8aβ,9,10,11,12,12aβ,13,13aα-decahydro-8H-isoquino[2,1-g][1,6]naphthyridine; and
(±)-2,3-dimethyl-5,6,8aα,9,10,11,12,12aα,13,13aα-decahydro-8H-isoquino[2,1-g][1,6]naphthyridine;
(±)-3-ethyl-5,6,8aβ,9,10,11,12,12aβ,13,13aα-decahydro-8H-isoquino[2,1-g][1,6]naphthyridine; and
(±)-3-ethyl-5,6,8aα,9,10,11,12,12aα,13,13aα-decahydro-8H-isoquino[2,1-g][1,6]naphthyridine;
(±)-3-isobutyl-5,6,8aβ,9,10,11,12,12aβ,13,13aα-decahydro-8H-isoquino[2,1-g][1,6]naphthyridine; and
(±)-3-isobutyl-5,6,8aα,9,10,11,12,12aα,13,13aα-decahydro-8H-isoquino[2,1-g][1,6]naphthyridine;
(±)-3-n-hexyl-5,6,8aβ,9,10,11,12,12aβ,13,13aα-decahydro-8H-isoquino[2,1-g][1,6]naphthyridine; and
(±)-3-n-hexyl-5,6,8aα,9,10,11,12,12aα,13,13aα-decahydro-8H-isoquino[2,1-g][1,6]naphthyridine;
(±)-1-methoxy-5,6,8aβ,9,10,11,12,12aβ,13,13aα-decahydro-8H-isoquino[2,1-g][1,6]naphthyridine; and
(±)-1-methoxy-5,6,8aα,9,10,11,12,12aα,13,13aα-decahydro-8H-isoquino[2,1-g][1,6]naphthyridine;
(±)-2-methoxy-5,6,8aβ,9,10,11,12,12aβ,13,13aα-decahydro-8H-isoquino[2,1-g][1,6]naphthyridine; and
(±)-2-methoxy-5,6,8aα,9,10,11,12,12aα,13,13aα-decahydro-8H-isoquino[2,1-g][1,6]naphthyridine;
(±)-4-methoxy-5,6,8aβ,9,10,11,12,12aβ,13,13aα-decahydro-8H-isoquino[2,1-g][1,6]naphthyridine; and
(±)-4-methoxy-5,6,8aα,9,10,11,12,12aα,13,13aα-decahydro-8H-isoquino[2,1-g][1,6]naphthyridine;
(±)-3-ethoxy-5,6,8aβ,9,10,11,12,12aβ,13,13aα-decahydro-8H-isoquino[2,1-g][1,6]naphthyridine; and
(±)-3-ethoxy-5,6,8aα,9,10,11,12,12aα,13,13aα-decahydro-8H-isoquino[2,1-g][1,6]naphthyridine;
(±)-3-isobutoxy-5,6,8aβ,9,10,11,12,12aβ,13,13aα-decahydro-8H-isoquino[2,1-g][1,6]naphthyridine; and

(±)-3-isobutoxy-5,6,8aα,9,10,11,12,12aα,13,13aα-decahydro-8H-isoquino[2,1-g][1,6]naphthyridine;
(±)-3-n-hexyloxy-5,6,8aβ,9,10,11,12,12aβ,13,13aα-decahydro-8H-isoquino[2,1-g][1,6]naphthyridine; and
(±)-3-n-hexyloxy-5,6,8aα,9,10,11,12,12aα,13,13aα-decahydro-8H-isoquino[2,1-g][1,6]naphthyridine;
(±)-3-hydroxy-5,6,8aβ,9,10,11,12,12aβ,13,13aα-decahydro-8H-isoquino[2,1-g][1,6]naphthyridine; and
(±)-3-hydroxy-5,6,8aα,9,10,11,12,12aα,13,13aα-decahydro-8H-isoquino[2,1-g][1,6]naphthyridine;
(±)-2,3-dihydroxy-5,6,8aβ,9,10,11,12,12aβ,13,13aα-decahydro-8H-isoquino[2,1-g][1,6]naphthyridine; and
(±)-2,3-dihydroxy-5,6,8aα,9,10,11,12,12aα,13,13aα-decahydro-8H-isoquino[2,1-g][1,6]naphthyridine;
(±)-1,2-dimethoxy-5,6,8aβ,9,10,11,12,12aβ,13,13aα-decahydro-8H-isoquino[2,1-g][1,6]naphthyridine; and
(±)-1,2-dimethoxy-5,6,8aα,9,10,11,12,12aα,13,13aα-decahydro-8H-isoquino[2,1-g][1,6]naphthyridine;
(±)-1,4-dimethoxy-5,6,8aβ,9,10,11,12,12aβ,13,13aα-decahydro-8H-isoquino[2,1-g][1,6]naphthyridine; and
(±)-1,4-dimethoxy-5,6,8aα,9,10,11,12,12aα,13,13aα-decahydro-8H-isoquino[2,1-g][1,6]naphthyridine;
(±)-3-4-dimethoxy-5,6,8aβ,9,10,11,12,12aβ,13,13aα-decahydro-8H-isoquino[2,1-g][1,6]naphthyridine; and
(±)-3,4-dimethoxy-5,6,8aα,9,10,11,12,12aα,13,13aα-decahydro-8H-isoquino[2,1-g][1,6]naphthyridine;
(±)-2,3-diethoxy-5,6,8aβ,9,10,11,12,12aβ,13,13aα-decahydro-8H-isoquino[2,1-g][1,6]naphthyridine; and
(±)-2,3-diethoxy-5,6,8aα,9,10,11,12,12aα,13,13aα-decahydro-8H-isoquino[2,1-g][1,6]naphthyridine;
(±)-2,3-di-n-butoxy-5,6,8aβ,9,10,11,12,12aβ,13,13aα-decahydro-8H-isoquino[2,1-g][1,6]naphthyridine; and
(±)-2,3-di-n-butoxy-5,6,8aα,9,10,11,12,12aα,13,13aα-decahydro-8H-isoquino[2,1-g][1,6]naphthyridine;
(±)-1,2-methylenedioxy-5,6,8aβ,9,10,11,12,12aβ,13,13aα-decahydro-8H-isoquino[2,1-g][1,6]-naphthyridine; and
(±)-1,2-methylenedioxy-5,6,8aα,9,10,11,12,12aα,13,13aα-decahydro-8H-isoquino[2,1-g][1,6]-naphthyridine;
(±)-2-chloro-5,6,8aβ,9,10,11,12,12aβ,13,13aα-decahydro-8H-isoquino[2,1-g][1,6]naphthyridine; and
(±)-2-chloro-5,6,8aα,9,10,11,12,12aα,13,13aα-decahydro-8H-isoquino[2,1-g][1,6]naphthyridine;
(±)-3-chloro-5,6,8aβ,9,10,11,12,12aβ,13,13aα-decahydro-8H-isoquino[2,1-g][1,6]naphthyridine; and
(±)-3-chloro-5,6,8aα,9,10,11,12,12aα,13,13aα-decahydro-8H-isoquino[2,1-g][1,6]naphthyridine;
(±)-4-chloro-5,6,8aβ,9,10,11,12,12aβ,13,13aα-decahydro-8H-isoquino[2,1-g][1,6]naphthyridine; and
(±)-4-chloro-5,6,8aα,9,10,11,12,12aα,13,13aα-decahydro-8H-isoquino[2,1-g][1,6]naphthyridine;
(±)-3-bromo-5,6,8aβ,9,10,11,12,12aβ,13,13aα-decahydro-8H-isoquino[2,1-g][1,6]naphthyridine; and
(±)-3-bromo-5,6,8aα,9,10,11,12,12aα,13,13aα-decahydro-8H-isoquino[2,1-g][1,6]naphthyridine;
(±)-3-fluoro-5,6,8aβ,9,10,11,12,12aβ,13,13aα-decahydro-8H-isoquino[2,1-g][1,6]naphthyridine; and
(±)-3-fluoro-5,6,8aα,9,10,11,12,12aα,13,13aα-decahydro-8H-isoquino[2,1-g][1,6]naphthyridine;
(±)-2-fluoro-5,6,8aβ,9,10,11,12,12aβ,13,13aα-decahydro-8H-isoquino[2,1-g][1,6]naphthyridine; and
(±)-2-fluoro-5,6,8aα,9,10,11,12,12aα,13,13aα-decahydro-8H-isoquino[2,1-g][1,6]naphthyridine;

## PREPARATION 3B

Preparation of (8aS,12aS,13aS)-3-methoxy-12-(1-R-phenethylaminocarbonyl)-5,6,8a,9,10,11,12,12a,13,13a-decahydroisoquino[2,1-g][1,6]naphthyridin-8-one (IX) and (8aR,12aR,13aR)-3-methoxy-12-(1-R-phenethylaminocarbonyl)-5,6,8a,9,10,11,12,12a,13,13a-decahydroisoquino[2,1-g][1,6]naphthyridin-8-one (X) and Related Compounds of Formula (IX), (X), (XV) and (XVI)

A. A solution of 1.95 g of (±)-3-methoxy-5,6,8aα,9,10,11,12,12aα,13,13aα-decahydroisoquino[2,1-g][1,6]-naphthyridin-8-one, a compound of formula (V), and 1.0 g of (R)-(+)-α-methylbenzyl isocyanate in 50 ml of methylene chloride was stirred at room temperature for 30 minutes. Solvent was then removed under reduced pressure, and the residue chromatographed on silica gel, using multiple medium pressure chromatography and eluting with 5% methanol in ethyl acetate. The first compound eluted was (8aS,12aS,13as)-3-methoxy-12-(1-R-phenethylaminocarbonyl)-5,6,8a,9,10,11,12,12a,13,13a-decahydroisoquino[2,1-g][1,6]naphthyridin-8-one,
mp 198-199°C, $[\alpha]_D^{25} = +36.5$ ($CHCl_3$)
followed by (8aR,12aR,13aR)-3-methoxy-12-(1-R-phenethylaminocarbonyl)-5,6,8a,9,10,11,12,12a,13,13a-decahydroisoquino[2,1-g][1,6]naphthyridin-8-one,

mp 220-221 °C, $[\alpha]_D^{25}$ = -11.4 ($CHCl_3$).

B. Similarly, replacing (±)-3-methoxy-5,6,8aα,9,10,11,12,12aα,13,13aα-decahydroisoquino[2,1-g][1,6]-naphthyridin-8-one with the appropriate compound of formula (V) and following the procedure in paragraph A above, the following compounds of formula (IX) and (X) were prepared:

(8aS,12aS,13aS)-12-(1-R-phenethylaminocarbonyl)-5,6,8a,9,10,11,12,12a,13,13a-decahydroisoquino-[2,1-g][1,6]naphthyridin-8-one; and

(8aR,12aR,13aR)-12-(1-R-phenethylaminocarbonyl)-5,6,8a,9,10,11,12,12a,13,13a-decahydroisoquino-[2,1-g][1,6]naphthyridin-8-one;

(8aS,12aS,13aS)-2,3-methylenedioxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-methylenedioxy-12-(1-R-phenethylaminocarbonyl)-5,6,8a,9,10,11,12,12a,-13,13a-decahydroisoquino[2,1-g][1,6]naphthyridin-8-one;

C. Similarly, replacing (±)-3-methoxy-5,6,8aα,9,10,11,12,12aα,13,13aα-decahydroisoquino[2,1-g][1,6]-naphthyridin-8-one with the appropriate compound of formula (V) and following the procedure in paragraph A above, the following compounds of formula (IX) and (X) are prepared:

(8aS,12aS,13aS)-2,3-dimethoxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-dimethoxy-12-(1-R-phenethylaminocarbonyl)-5,6,8a,9,10,11,12,12a,13,13a-decahydroisoquino[2,1-g][1,6]naphthyridin-8-one;

(8aS,12aS,13aS)-1,4-dimethoxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1,4-dimethoxy-12-(1-R-phenethylaminocarbonyl)-5,6,8a,9,10,11,12,12a,13,13a-decahydroisoquino[2,1-g][1,6]naphthyridin-8-one;

(8aS,12aS,13aS)-2,3-(ethylene-1,2-dioxy)-12-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-(ethylene-1,2-dioxy)-12-(1-R-phenethylaminocarbonyl)-5,6,8a,9,10,11,12,12a,13,13a-decahydroisoquino[2,1-g][1,6]naphthyridin-8-one;

(8aS,12aS,13aS)-1-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1-methyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-2-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2-methyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-methyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-2,3-dimethyl-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-dimethyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-ethyl-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-ethyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-isobutyl-(1-R-phenethylaminocarbonyl)-5,6,8a,9,10,11,12,12a,13,-13a-decahydroisoquino[2,1-g][1,6]naphthyridin-8-one; and

(8aR,12aR,13aR)-3-isobutyl-(1-R-phenethylaminocarbonyl)-5,6,8a,9,10,11,12,12a,13,-13a-decahydroisoquino[2,1-g][1,6]naphthyridin-8-one;

(8aS,12aS,13aS)-3-n-hexyl-(1-R-phenethylaminocarbonyl)-5,6,8a,9,10,11,12,12a,13,13a-decahydroisoquino[2,1-g][1,6]naphthyridin-8-one;and

(8aR,12aR,13aR)-3-n-hexyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-1-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-2-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-4-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-4-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-methoxy-2-methyl-(1-R-phenethylaminocarbonyl)-5,6,8a,9,10,11,12,12a,-13,13a-decahydroisoquino[2,1-g][1,6]naphthyridin-8-one; and

(8aR,12aR,13aR)-3-methoxy-2-methyl-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-ethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-ethoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-isopropoxy-(1-R-phenethylaminocarbonyl)-5,6,8a,9,10,11,12,12a,13,-13a-decahydroisoquino[2,1-g][1,6]naphthyridin-8-one; and

(8aR,12aR,13aR)-3-isopropoxy-(1-R-phenethylaminocarbonyl)-5,6,8a,9,10,11,12,12a,13,-13a-decahydroisoquino[2,1-g][1,6]naphthyridin-8-one;

(8aS,12aS,13aS)-3-isobutoxy-(1-R-phenethylaminocarbonyl)-5,6,8a,9,10,11,12,12a,13,-13a-decahydroisoquino[2,1-g][1,6]naphthyridin-8-one; and

(8aR,12aR,13aR)-3-isobutoxy-(1-R-phenethylaminocarbonyl)-5,6,8a,9,10,11,12,12a,13,-13a-decahydroisoquino[2,1-g][1,6]naphthyridin-8-one;

(8aS,12aS,13aS)-3-n-hexyloxy-(1-R-phenethylaminocarbonyl)-5,6,8a,9,10,11,12,12a,13,-13a-decahydroisoquino[2,1-g][1,6]naphthyridin-8-one; and

(8aR,12aR,13aR)-3-n-hexyloxy-(1-R-phenethylaminocarbonyl)-5,6,8a,9,10,11,12,12a,13,-13a-decahydroisoquino[2,1-g][1,6]naphthyridin-8-one;

(8aS,12aS,13aS)-3-hydroxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-hydroxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-2,3-dihydroxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-dihydroxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-1,2-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1,2-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-1,4-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1,4-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-3,4-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3,4-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-2,3-diethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2,3-diethoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-2,3-di-n-butoxy-(1-R-phenethylaminocaroonyl)-5,6,8a,9,10,11,12,12a,13,13a-decahydroisoquino[2,1-g][1,6]naphthyridin-8-one; and

(8aR,12aR,13aR)-2,3-di-n-butoxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-1,2-methylenedioxy-(1-R-phenethylaminocarbonyl)-5,6,8a,9,10,11,12,12a,13,13a-decahydroisoquino[2,1-g][1,6]naphthyridin-8-one; and

(8aR,12aR,13aR)-1,2-methylenedioxy-(1-R-phenethylaminocarbonyl)-5,6,8a,9,10,11,12,12a,13,13a-decahydroisoquino[2,1-g][1,6]naphthyridin-8-one;

(8aS,12aS,13aS)-2-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2-chloro-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-chloro-(1-R-phenethylaminocarbonyl)-5,6,8a,9,10,11,12,12a,13,13a-decahydroisoquino[2,1-g][1,6]naphthyridin-8-one;

(8aS,12aS,13aS)-4-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aR)-4-chloro-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-bromo-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-bromo-(1-R-phenethylaminocarbonyl)-5,6,8a,9,10,11,12,12a,13,13a-decahydroisoquino[2,1-g][1,6]naphthyridin-8-one;

(8aS,12aS,13aS)-3-fluoro-(1-R-phenethylaminocarbonyl)-5,6,8a,9,10,11,12,12a,13,13a-decahydroisoquino[2,1-g][1,6]naphthyridin-8-one; and

(8aR,12aR,13aR)-3-fluoro-(1-R-phenethylaminocarbonyl)-5,6,8a,9,10,11,12,12a,13,13a-decahydroisoquino[2,1-g][1,6]naphthyridin-8-one;

(8aS,12aS,13aS)-2-fluoro-(1-R-phenethylaminocarbonyl)-5,6,8a,9,10,11,12,12a,13,13a-decahydroisoquino[2,1-g][1,6]naphthyridin-8-one; and

(8aR,12aR,13aR)-2-fluoro-(1-R-phenethylaminocarbonyl)-5,6,8a,9,10,11,12,12a,13,13a-decahydroisoquino[2,1-g][1,6]naphthyridin-8-one.

D. Similarly, replacing (±)-3-methoxy-5,6,8aα,9,10,11,12,12aα,13,13aα-decahydroisoquino[2,1-g][1,6]-naphthyridin-8-one with the appropriate compound of formula (VI) and following the procedure in paragraph A above, the following compounds of formula (XV) and (XVI) are prepared:

(8aS,12aS,13aR)-3-methoxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-methoxy-12-(1-R-phenethylaminocarbonyl)-5,6,8a,9,10,11,12,12a,13,13a-decahydroisoquino[2,1-g][1,6]naphthyridin-8-one;

(8aS,12aS,13aR)-12-(1-R-phenethylaminocarbonyl)-5,6,8a,9,10,11,12,12a,13,13a-decahydroisoquino-[2,1-g][1,6]naphthyridin-8-one; and

(8aR,12aR,13aS)-12-(1-R-phenethylaminocarbonyl)-5,6,8a,9,10,11,12,12a,13,13a-decahydroisoquino-[2,1-g][1,6]naphthyridin-8-one;

(8aS,12aS,13aR)-2,3-methylenedioxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-methylenedioxy-12-(1-R-phenethylaminocarbonyl)-5,6,8a,9,10,11,12,12a,-13,13a-decahydroisoquino[2,1-g][1,6]naphthyridin-8-one;

(8aS,12aS,13aR)-2,3-dimethoxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-dimethoxy-12-(1-R-phenethylaminocarbonyl)-5,6,8a,9,10,11,12,12a,13,13a-decahydroisoquino[2,1-g][1,6]naphthyridin-8-one;

(8aS,12aS,13aR)-1,4-dimethoxy-12-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1,4-dimethoxy-12-(1-R-phenethylaminocarbonyl)-5,6,8a,9,10,11,12,12a,13,13a-decahydroisoquino[2,1-g][1,6]naphthyridin-8-one;

(8aS,12aS,13aR)-2,3-(ethylene-1,2-dioxy)-12-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-(ethylene-1,2-dioxy)-12-(1-R-phenethylaminocarbonyl)-5,6,8a,9,10,11,12,12a,13,13a-decahydroisoquino[2,1-g][1,6]naphthyridin-8-one;

(8aS,12aS,13aR)-1-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1-methyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-2-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2-methyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-methyl-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-methyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-2,3-dimethyl-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-dimethyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-ethyl-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-ethyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-isobutyl-(1-R-phenethylaminocarbonyl)-5,6,8a,9,10,11,12,12a,13,-13a-decahydroisoquino[2,1-g][1,6]naphthyridin-8-one; and

(8aR,12aR,13aS)-3-isobutyl-(1-R-phenethylaminocarbonyl)-5,6,8a,9,10,11,12,12a,13,-13a-decahydroisoquino[2,1-g][1,6]naphthyridin-8-one;

(8aS,12aS,13aR)-3-n-hexyl-(1-R-phenethylaminocarbonyl)-5,6,8a,9,10,11,12,12a,13,13a-decahydroisoquino[2,1-g][1,6]naphthyridin-8-one;and

(8aR,12aR,13aS)-3-n-hexyl-(1-R-phenethylaminocarbonyl)-5,6,8a,9,10,11,12,12a,13,13a-decahydroisoquino[2,1-g][1,6]naphthyridin-8-one;

(8aS,12aS,13aR)-1-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-2-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-4-methoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-4-methoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-methoxy-2-methyl-(1-R-phenethylaminocarbonyl)-5,6,8a,9,10,11,12,12a,-13,13a-decahydroisoquino[2,1-g][1,6]naphthyridin-8-one; and

(8aR,12aR,13aS)-3-methoxy-2-methyl-(1-R-phenethylaminocarbonyl)-5,6,8a,9,10,11,12,12a,-13,13a-decahydroisoquino[2,1-g][1,6]naphthyridin-8-one;

(8aS,12aS,13aR)-3-ethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-ethoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-isopropoxy-(1-R-phenethylaminocarbonyl)-5,6,8a,9,10,11,12,12a,13,-13a-decahydroisoquino[2,1-g][1,6]naphthyridin-8-one; and

(8aR,12aR,13aS)-3-isopropoxy-(1-R-phenethylaminocarbonyl)-5,6,8a,9,10,11,12,12a,13,-13a-decahydroisoquino[2,1-g][1,6]naphthyridin-8-one;

(8aS,12aS,13aR)-3-isobutoxy-(1-R-phenethylaminocarbonyl)-5,6,8a,9,10,11,12,12a,13,-13a-decahydroisoquino[2,1-g][1,6]naphthyridin-8-one; and

(8aR,12aR,13aS)-3-isobutoxy-(1-R-phenethylaminocarbonyl)-5,6,8a,9,10,11,12,12a,13,-13a-decahydroisoquino[2,1-g][1,6]naphthyridin-8-one;

(8aS,12aS,13aR)-3-n-hexyloxy-(1-R-phenethylaminocarbonyl)-5,6,8a,9,10,11,12,12a,13,-13a-decahydroisoquino[2,1-g][1,6]naphthyridin-8-one; and

(8aR,12aR,13aS)-3-n-hexyloxy-(1-R-phenethylaminocarbonyl)-5,6,8a,9,10,11,12,12a,13,-13a-decahydroisoquino[2,1-g][1,6]naphthyridin-8-one;

(8aS,12aS,13aR)-3-hydroxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-hydroxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-2,3-dihydroxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-dihydroxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-1,2-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1,2-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-1,4-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1,4-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-3,4-dimethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3,4-dimethoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-2,3-diethoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-diethoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-2,3-di-n-butoxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2,3-di-n-butoxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-1,2-methylenedioxy-(1-R-phenethylaminocarbonyl)-5,6,8a,9,10,11,12,12a,13,13a-decahydroisoquino[2,1-g][1,6]naphthyridin-8-one; and

(8aR,12aR,13aS)-1,2-methylenedioxy-(1-R-phenethylaminocarbonyl)-5,6,8a,9,10,11,12,12a,13,13a-decahydroisoquino[2,1-g][1,6]naphthyridin-8-one;

(8aS,12aS,13aR)-2-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2-chloro-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-chloro-(1-R-phenethylaminocarbonyl)-5,6,8a,9,10,11,12,12a,13,13a-decahydroisoquino[2,1-g][1,6]naphthyridin-8-one;

(8aS,12aS,13aR)-4-chloro-(1-R-phenethylaminocarbonyl)-5,6,8a,9,10,11,12,12a,13,13a-decahydroisoquino[2,1-g][1,6]naphthyridin-8-one; and

(8aR,12aR,13aS)-4-chloro-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-bromo-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-bromo-(1-R-phenethylaminocarbonyl)-5,6,8a,9,10,11,12,12a,13,13a-decahydroisoquino[2,1-g][1,6]naphthyridin-8-one;

(8aS,12aS,13aR)-3-fluoro-(1-R-phenethylaminocarbonyl)-5,6,8a,9,10,11,12,12a,13,13a-decahydroisoquino[2,1-g][1,6]naphthyridin-8-one; and

(8aR,12aR,13aS)-3-fluoro-(1-R-phenethylaminocarbonyl)-5,6,8a,9,10,11,12,12a,13,13a-decahydroisoquino[2,1-g][1,6]naphthyridin-8-one;

(8aS,12aS,13aR)-2-fluoro-(1-R-phenethylaminocarbonyl)-5,6,8a,9,10,11,12,12a,13,13a-decahydroisoquino[2,1-g][1,6]naphthyridin-8-one; and

(8aR,12aR,13aS)-2-fluoro-(1-R-phenethylaminocarbonyl)-5,6,8a,9,10,11,12,12a,13,13a-decahydroisoquino[2,1-g][1,6]naphthyridin-8-one.

PREPARATION 4

Preparation of (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine and (8aS,12aR,13aR)-3-methoxy-12-(1-R-phenethylaminocarbonyl)-5,6,8a,9,10,11,12,12a,13,13a-decahydroiso-8H-quino[2,1-g][1,6]naphthyridine and Related Compounds of Formula (XI), (XII) and (XVII)

A. A solution of 11.5 g of (8aS,12aS,13aS)-3-methoxy-12-(1-R-phenethylaminocarbonyl)-5,6,8a,9,10,11,12,12a,13a,13a-decahydroisoquino[2,1-g][1,6]naphthyridin-8-one, in 50 ml of tetrahydrofuran was added slowly to a solution of 2.0 g of lithium aluminum hydride in 75 ml of tetrahydrofuran. The resulting mixture was stirred at reflux for 2 hours, cooled, and treated sequentially with 2.5 ml of water, 2.5 ml of 15% sodium hydroxide, and 7.5 ml of water. The mixture was filtered and the filtrate was evaporated to afford 8.8 g of (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine, a compound of formula (XI), as a foam. The foam was used as such in the next reaction with no further purification.

B. Similarly, replacing (8aS,12aS,13aS)-3-methoxy-12-(1-R-phenethylaminocarbonyl)-5,6,8a,9,10,11,12,12a,13,13a-decahydroisoquino[2,1-g][1,6]naphthyridin-8-one with other compounds of formula (IX) or (X) and following the procedure in paragraph A above, the following compounds of formula (XI) and (XII) were prepared:

(8aS,12aR,13aR)-3-methoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aS)-12-(1-R-phenethylaminocarbonyl)-5,6,8a,9,10,11,12,12a,13,13a-decahydro-8H-isoquino[2,1-g][1,6]naphthyridine;

(8aS,12aR,13aR)-12-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-methylenedioxy-12-(1-R-phenethylaminocarbonyl)-5,6,8a,9,10,11,12,12a,13,13a-decahydro-8H-isoquino[2,1-g][1,6]naphthyridine; and

(8aS,12aR,13aR)-2,3-methylenedioxy-12-(1-R-phenethylaminocarbonyl)-5,6,8a,9,10,11,12,12a,13,13a-decahydro-8H-isoquino[2,1-g][1,6]naphthyridine.

C. Similarly, replacing (8aS,12aS,13aS)-3-methoxy-12-(1-R-phenethylaminocarbonyl)-5,6,8a,9,10,11,12,12a,13,13a-decahydroisoquino[2,1-g][1,6]naphthyridin-8-one with other compounds of formula (IX) or (X) and following the procedure in paragraph A above, the following exemplary compounds of formula (XI) and (XII) are prepared:

(8aR,12aS,13aS)-2,3-dimethoxy-12-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-dimethoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aS)-1,4-dimethoxy-12-(1-R-phenethylaminocarbonyl)-5,6,8a,9,10,11,12,12a,13,13a-decahydro-8H-isoquino[2,1-g][1,6]naphthyridine;

(8aS,12aR,13aR)-1,4-dimethoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-(ethylene-1,2-dioxy)-12-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-(ethylene-1,2-dioxy)-12-(1-R-phenethylaminocarbonyl)-5,6,8a,9,10,11,12,12a,13,13a-decahydro-8H-isoquino[2,1-g][1,6]naphthyridine;

(8aR,12aS,13aS)-1-methyl-(1-R-phenethylaminocarbonyl)-5,6,8a,9,10,11,12,12a,13,13a-decahydro-8H-isoquino[2,1-g][1,6]naphthyridine;

(8aS,12aR,13aR)-1-methyl-(1-R-phenethylaminocarbonyl)-5,6,8a,9,10,11,12,12a,13,13a-decahydro-8H-isoquino[2,1-g][1,6]naphthyridine;

(8aR,12aS,13aS)-2-methyl-(1-R-phenethylaminocarbonyl)-5,6,8a,9,10,11,12,12a,13,13a-decahydro-8H-isoquino[2,1-g][1,6]naphthyridine;

(8aS,12aR,13aR)-2-methyl-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-methyl-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-methyl-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-dimethyl-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-dimethyl-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-ethyl-(1-R-phenethylaminocarbonyl)-5,6,8a,9,10,11,12,12a,13,13a-decahydro-8H-isoquino[2,1-g][1,6]-naphthyridine;

(8aS,12aR,13aR)-3-ethyl-(1-R-phenethylaminocarbonyl)-5,6,8a,9,10,11,12,12a,13,13a-decahydro-8H-isoquino[2,1-g][1,6]-naphthyridine;

(8aR,12aS,13aS)-3-isobutyl-(1-R-phenethylaminocarbonyl)-5,6,8a,9,10,11,12,12a,13,-13a-decahydro-8H-isoquino[2,1-g][1,6]naphthyridine;

(8aS,12aR,13aR)-3-isobutyl-(1-R-phenethylaminocarbonyl)-5,6,8a,9,10,11,12,12a,13,-13a-decahydro-8H-isoquino[2,1-g][1,6]naphthyridine;

(8aR,12aS,13aS)-3-n-hexyl-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-n-hexyl-(1-R-phenethylaminocarbonyl)-5,6,8a,9,10,11,12,12a,13,13a-decahydro-8H-isoquino[2,1-g][1,6]naphthyridine;

(8aR,12aS,13aS)-1-methoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-1-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-2-methoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-2-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-4-methoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-4-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-methoxy-2-methyl-(1-R-phenethylaminocarbonyl)-5,6,8a,9,10,11,12,12a,-13,13a-decahydro-8H-isoquino[2,1-g][1,6]naphthyridine;

(8aS,12aR,13aR)-3-methoxy-2-methyl-(1-R-phenethylaminocarbonyl)-5,6,8a,9,10,11,12,12a,-13,13a-decahydro-8H-isoquino[2,1-g][1,6]naphthyridine;

(8aR,12aS,13aS)-3-ethoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-ethoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-isopropoxy-(1-R-phenethylaminocarbonyl)-5,6,8a,9,10,11,12,12a,13,-13a-decahydro-8H-isoquino[2,1-g][1,6]naphthyridine;

(8aS,12aR,13aR)-3-isopropoxy-(1-R-phenethylaminocarbonyl)-5,6,8a,9,10,11,12,12a,13,-13a-decahydro-8H-isoquino[2,1-g][1,6]naphthyridine;

(8aR,12aS,13aS)-3-isobutoxy-(1-R-phenethylaminocarbonyl)-5,6,8a,9,10,11,12,12a,13,-13a-decahydro-8H-isoquino[2,1-g][1,6]naphthyridine;

(8aS,12aR,13aR)-3-isobutoxy-(1-R-phenethylaminocarbonyl)-5,6,8a,9,10,11,12,12a,13,-13a-decahydro-8H-isoquino[2,1-g][1,6]naphthyridine;

(8aR,12aS,13aS)-3-n-hexyloxy-(1-R-phenethylaminocarbonyl)-5,6,8a,9,10,11,12,12a,13,-13a-decahydro-8H-isoquino[2,1-g][1,6]naphthyridine;

(8aS,12aR,13aR)-3-n-hexyloxy-(1-R-phenethylaminocarbonyl)-5,6,8a,9,10,11,12,12a,13,-13a-decahydro-8H-isoquino[2,1-g][1,6]naphthyridine;

(8aR,12aS,13aS)-3-hydroxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-hydroxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-dihydroxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-dihydroxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-1,2-dimethoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-1,2-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-1,4-dimethoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-1,4-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-3,4-dimethoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-3,4-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-diethoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-diethoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-2,3-di-n-butoxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-2,3-di-n-butoxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-1,2-methylenedioxy-(1-R-phenethylaminocarbonyl)-5,6,8a,9,10,11,12,12a,13,13a-decahydro-8H-isoquino[2,1-g][1,6]naphthyridine;

(8aS,12aR,13aR)-1,2-methylenedioxy-(1-R-phenethylaminocarbonyl)-5,6,8a,9,10,11,12,12a,13,13a-decahydro-8H-isoquino[2,1-g][1,6]naphthyridine;

(8aR,12aS,13aS)-2-chloro-(1-R-phenethylaminocarbonyl)-5,6,8a,9,10,11,12,12a,13,13a-decahydro-8H-isoquino[2,1-g][1,6]naphthyridine;

(8aS,12aR,13aR)-2-chloro-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-chloro-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-chloro-(1-R-phenethylaminocarbonyl)-5,6,8a,9,10,11,12,12a,13,13a-decahydro-8H-isoquino[2,1-g][1,6]naphthyridine;

(8aR,12aS,13aS)-4-chloro-(1-R-phenethylaminocarbonyl)-5,6,8a,9,10,11,12,12a,13,13a-decahydro-8H-isoquino[2,1-g][1,6]naphthyridine;

(8aS,12aR,13aR)-4-chloro-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-bromo-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-bromo-(1-R-phenethylaminocarbonyl)-5,6,8a,9,10,11,12,12a,13,13a-decahydro-8H-isoquino[2,1-g][1,6]naphthyridine;

(8aR,12aS,13aS)-3-fluoro-(1-R-phenethylaminocarbonyl)-5,6,8a,9,10,11,12,12a,13,13a-decahydro-8H-isoquino[2,1-g][1,6]naphthyridine;

(8aS,12aR,13aR)-3-fluoro-(1-R-phenethylaminocarbonyl)-5,6,8a,9,10,11,12,12a,13,13a-decahydro-8H-isoquino[2,1-g][1,6]naphthyridine;

(8aR,12aS,13aS)-2-fluoro-(1-R-phenethylaminocarbonyl)-5,6,8a,9,10,11,12,12a,13,13a-decahydro-8H-isoquino[2,1-g][1,6]naphthyridine; and

(8aS,12aR,13aR)-2-fluoro-(1-R-phenethylaminocarbonyl)-5,6,8a,9,10,11,12,12a,13,13a-decahydro-8H-isoquino[2,1-g][1,6]naphthyridine.

D. Similarly, replacing (8aS,12aS,13aS)-3-methoxy-12-(1-R-phenethylaminocarbonyl)-5,6,8a,9,10,11,12,12a,13,13a-decahydroisoquino[2,1-g][1,6]naphthyridin-8-one with other compounds of formula (XV) and following the procedure in paragraph A above, the following exemplary compounds of formula (XVII) are prepared:

(8aR,12aS,13aR)-3-methoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-methylenedioxy-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-dimethoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-1,4-dimethoxy-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-(ethylene-1,2-dioxy-12-(1-R-phenethylaminocarbonyl)-5,6,8a,9,10,11,12,12a,13,13a-decahydro-8H-isoquino[2,1-g][1,6]naphthyridine;

(8aR,12aS,13aR)-1-methyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-2-methyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-methyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-dimethyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-ethyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-isobutyl-(1-R-phenethylaminocarbonyl)-5,6,8a,9,10,11,12,12a,13,-13a-decahydro-8H-isoquino[2,1-g][1,6]naphthyridine;

(8aR,12aS,13aR)-3-n-hexyl-(1-R-phenethylaminocarbonyl)-5,6,8a,9,10,11,12,12a,13,13a-decahydro-8H-isoquino[2,1-g][1,6]naphthyridine;

(8aR,12aS,13aR)-1-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-2-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-4-methoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-methoxy-2-methyl-(1-R-phenethylaminocarbonyl)-5,6,8a,9,10,11,12,12a,-13,13a-decahydro-8H-isoquino[2,1-g][1,6]naphthyridine;

(8aR,12aS,13aR)-3-ethoxy-(1-R-phenethylaminocarbonyl)-5,6,8a,9,10,11,12,12a,13,13a-decahydro-8H-isoquino[2,1-g][1,6]-naphthyridine;

(8aR,12aS,13aR)-3-isopropoxy-(1-R-phenethylaminocarbonyl)-5,6,8a,9,10,11,12,12a,13,-13a-decahydro-8H-isoquino[2,1-g][1,6]naphthyridine;

(8aR,12aS,13aR)-3-isobutoxy-(1-R-phenethylaminocarbonyl)-5,6,8a,9,10,11,12,12a,13,-13a-decahydro-8H-isoquino[2,1-g][1,6]naphthyridine;

(8aR,12aS,13aR)-3-n-hexyloxy-(1-R-phenethylaminocarbonyl)-5,6,8a,9,10,11,12,12a,13,-13a-decahydro-8H-isoquino[2,1-g][1,6]naphthyridine;

(8aR,12aS,13aR)-3-hydroxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-dihydroxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-1,2-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-1,4-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-3,4-dimethoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-diethoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-2,3-di-n-butoxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-1,2-methylenedioxy-(1-R-phenethylaminocarbonyl)-5,6,8a,9,10,11,12,12a,13,13a-decahydro-8H-isoquino[2,1-g][1,6]naphthyridine;

(8aR,12aS,13aR)-2-chloro-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-chloro-(1-R-phenethylaminocarbonyl)-5,6,8a,9,10,11,12,12a,13,13a-decahydro-8H-isoquino[2,1-g][1,6]naphthyridine;

(8aR,12aS,13aR)-4-chloro-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-bromo-(1-R-phenethylaminocarbonyl)-5,6,8a,9,10,11,12,12a,13,13a-decahydro-8H-isoquino[2,1-g][1,6]naphthyridine;

(8aR,12aS,13aR)-3-fluoro-(1-R-phenethylaminocarbonyl)-5,6,8a,9,10,11,12,12a,13,13a-decahydro-8H-isoquino[2,1-g][1,6]naphthyridine; and

(8aR,12aS,13aR)-2-fluoro-(1-R-phenethylaminocarbonyl)-5,6,8a,9,10,11,12,12a,13,13a-decahydro-8H-isoquino[2,1-g][1,6]naphthyridine.

PREPARATION 5

Preparation of (8aR,12aS,13aS)-3-methoxy-5,6,8a,9,10,11,12,12a,13,13a-decahydro-8H-isoquino[2,1g][1,6]-naphthyridine and (8aS,12aR,13aR)-3-methoxy-5,6,8a,9,10,11,12,12a,13,13a-decahydro-8H-isoquino[2,1g]-[1,6]naphthyridine and Related Compounds of Formula (XIII), (XIV) and (XVIII)

A. A solution of 10.5 g of (8aR,12aS,13aS)-3-methoxy-12-(1-R-phenethylaminocarbonyl)-5,6,8a,9,10,11,12,12a,13,13a-decahydro-8H-isoquino-[2,1-g][1,6]-naphthyridine in 125 ml of 2N sodium n-butoxide in n-butanol was refluxed for 4 hours. After cooling, water was added and the solution acidified with 2N hydrochloric acid. The solution was then extracted with ethyl acetate, the aqueous portion basified with aqueous ammonium hydroxide and extracted further with methylene chloride. Solvent was then removed from the extract under reduced pressure and the residue chromatographed on silica gel, eluting with 10-20% methanol in methylene chloride, to give (8aR,12aS,13aS)-3-methoxy-5,6,8a,9,10,11,12,12a,13,13a-decahydro-8H-isoquino[2,1-g][1,6]naphthyridine, a compound of formula (XIII),

mp 125-127 °C, $[\alpha]_D^{25}$ = -150.7 ($CHCl_3$)

B. Similarly, replacing (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine with other compounds of formula (XI) or (XII) and following the procedure in paragraph A above, the following compounds of formula (XIII) and (XIV) were prepared:

(8aS,12aR,13aR)-3-methoxy-5,6,8a,9,10,11,12,12a,13,13a-decahydro-8H-isoquino-[2,1-g][1,6]-naphthyridine,

mp 125-127 °C, $[\alpha]_D^{25}$ = +154.5 ($CHCl_3$).

(8aR,12aS,13aS)-5,6,8a,9,10,11,12,12a,13,13a-decahydro-8H-isoquino[2,1-g][1,6]naphthyridine;

(8aS,12aR,13aR)-5,6,8a,9,10,11,12,12a,13,13a-decahydro-8H-isoquino[2,1-g][1,6]naphthyridine;

(8aR,12aS,13aS)-2,3-methylenedioxy-5,6,8a,9,10,11,12,12a,13,13a-decahydro-8H-isoquino[2,1-g][1,6]-naphthyridine; and

(8aS,12aR,13aR)-2,3-methylenedioxy-5,6,8a,9,10,11,12,12a,13,13a-decahydro-8H-isoquino[2,1-g]-[1,6]naphthyridine.

C. Similarly, replacing (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine with other compounds of formula (XI) or (XII) and following the procedure in paragraph A above, the following exemplary compounds of formula (XIII) and (XIV) are prepared:

(8aR,12aS,13aS)-2,3-dimethoxy-5,6,8a,9,10,11,12,12a,13,13a-decahydro-8H-isoquino[2,1-g][1,6]-naphthyridine;

(8aS,12aR,13aR)-2,3-dimethoxy-5,6,8a,9,10,11,12,12a,13,13a-decahydro-8H-isoquino[2,1-g][1,6]-naphthyridine;

(8aR,12aS,13aS)-1,4-dimethoxy-5,6,8a,9,10,11,12,12a,13,13a-decahydro-8H-isoquino[2,1-g][1,6]-naphthyridine;

(8aS,12aR,13aR)-1,4-dimethoxy-5,6,8a,9,10,11,12,12a,13,13a-decahydro-8H-isoquino[2,1-g][1,6]-naphthyridine;

(8aR,12aS,13aS)-2,3-(ethylene-1,2-dioxy)-5,6,8a,9,10,11,12,12a,13,13a-decahydro-8H-isoquino[2,1-g]-[1,6]naphthyridine;

(8aS,12aR,13aR)-2,3-(ethylene-1,2-dioxy)-5,6,8a,9,10,11,12,12a,13,13a-decahydro-8H-isoquino[2,1-g]-[1,6]naphthyridine;

(8aR,12aS,13aS)-1-methyl-5,6,8a,9,10,11,12,12a,13,13a-decahydro-8H-isoquino[2,1-g][1,6]-naphthyridine;

(8aS,12aR,13aR)-1-methyl-5,6,8a,9,10,11,12,12a,13,13a-decahydro-8H-isoquino[2,1-g][1,6]-naphthyridine;

(8aR,12aS,13aS)-2-methyl-5,6,8a,9,10,11,12,12a,13,13a-decahydro-8H-isoquino[2,1-g][1,6]-naphthyridine;

(8aS,12aR,13aR)-2-methyl-5,6,8a,9,10,11,12,12a,13,13a-decahydro-8H-isoquino[2,1-g][1,6]-naphthyridine;

(8aR,12aS,13aS)-3-methyl-5,6,8a,9,10,11,12,12a,13,13a-decahydro-8H-isoquino[2,1-g][1,6]-

naphthyridine;

(8aS,12aR,13aR)-3-methyl-5,6,8a,9,10,11,12,12a,13,13a-decahydro-8H-isoquino[2,1-g][1,6]-naphthyridine;

(8aR,12aS,13aS)-2,3-dimethyl-5,6,8a,9,10,11,12,12a,13,13a-decahydro-8H-isoquino[2,1-g][1,6]-naphthyridine;

(8aS,12aR,13aR)-2,3-dimethyl-5,6,8a,9,10,11,12,12a,13,13a-decahydro-8H-isoquino[2,1-g][1,6]-naphthyridine;

(8aR,12aS,13aS)-3-ethyl-5,6,8a,9,10,11,12,12a,13,13a-decahydro-8H-isoquino-[2,1-g][1,6]-naphthyridine;

(8aS,12aR,13aR)-3-ethyl-5,6,8a,9,10,11,12,12a,13,13a-decahydro-8H-isoquino[2,1-g][1,6]-naphthyridine;

(8aR,12aS,13aS)-3-isobutyl-5,6,8a,9,10,11,12,12a,13,13a-decahydro-8H-isoquino[2,1-g][1,6]-naphthyridine;

(8aS,12aR,13aR)-3-isobutyl-5,6,8a,9,10,11,12,12a,13,13a-decahydro-8H-isoquino[2,1-g][1,6]-naphthyridine;

(8aR,12aS,13aS)-3-n-hexyl-5,6,8a,9,10,11,12,12a,13,13a-decahydro-8H-isoquino[2,1-g][1,6]-naphthyridine;

(8aS,12aR,13aR)-3-n-hexyl-5,6,8a,9,10,11,12,12a,13,13a-decahydro-8H-isoquino[2,1-g][1,6]-naphthyridine;

(8aR,12aS,13aS)-1-methoxy-5,6,8a,9,10,11,12,12a,13,13a-decahydro-8H-isoquino[2,1-g][1,6]-naphthyridine;

(8aS,12aR,13aR)-1-methoxy-5,6,8a,9,10,11,12,12a,13,13a-decahydro-8H-isoquino[2,1-g][1,6]-naphthyridine;

(8aR,12aS,13aS)-2-methoxy-5,6,8a,9,10,11,12,12a,13,13a-decahydro-8H-isoquino[2,1-g][1,6]-naphthyridine;

(8aS,12aR,13aR)-2-methoxy-5,6,8a,9,10,11,12,12a,13,13a-decahydro-8H-isoquino[2,1-g][1,6]-naphthyridine;

(8aR,12aS,13aS)-4-methoxy-5,6,8a,9,10,11,12,12a,13,13a-decahydro-8H-isoquino[2,1-g][1,6]-naphthyridine;

(8aS,12aR,13aR)-4-methoxy-5,6,8a,9,10,11,12,12a,13,13a-decahydro-8H-isoquino[2,1-g][1,6]-naphthyridine;

(8aR,12aS,13aS)-3-methoxy-2-methyl-5,6,8a,9,10,11,12,12a,13,13a-decahydro-8H-isoquino[2,1-g]-[1,6]naphthyridine;

(8aS,12aR,13aR)-3-methoxy-2-methyl-5,6,8a,9,10,11,12,12a,13,13a-decahydro-8H-isoquino[2,1-g]-[1,6]naphthyridine;

(8aR,12aS,13aS)-3-ethoxy-5,6,8a,9,10,11,12,12a,13,13a-decahydro-8H-isoquino[2,1-g][1,6]-naphthyridine;

(8aS,12aR,13aR)-3-ethoxy-5,6,8a,9,10,11,12,12a,13,13a-decahydro-8H-isoquino[2,1-g][1,6]-naphthyridine;

(8aR,12aS,13aS)-3-isopropoxy-5,6,8a,9,10,11,12,12a,13,13a-decahydro-8H-isoquino[2,1-g][1,6]-naphthyridine;

(8aS,12aR,13aR)-3-isobutoxy-5,6,8a,9,10,11,12,12a,13,13a-decahydro-8H-isoquino[2,1-g][1,6]-naphthyridine;

(8aR,12aS,13aS)-3-isopropoxy-5,6,8a,9,10,11,12,12a,13,13a-decahydro-8H-isoquino[2,1-g][1,6]-naphthyridine;

(8aS,12aR,13aR)-3-isobutoxy-5,6,8a,9,10,11,12,12a,13,13a-decahydro-8H-isoquino[2,1-g][1,6]-naphthyridine;

(8aR,12aS,13aS)-3-n-hexyloxy-5,6,8a,9,10,11,12,12a,13,13a-decahydro-8H-isoquino[2,1-g][1,6]-naphthyridine;

(8aS,12aR,13aR)-3-n-hexyloxy-5,6,8a,9,10,11,12,12a,13,13a-decahydro-8H-isoquino[2,1-g][1,6]-naphthyridine;

(8aR,12aS,13aS)-3-hydroxy-5,6,8a,9,10,11,12,12a,13,13a-decahydro-8H-isoquino[2,1-g][1,6]-naphthyridine;

(8aS,12aR,13aR)-3-hydroxy-5,6,8a,9,10,11,12,12a,13,13a-decahydro-8H-isoquino[2,1-g][1,6]-naphthyridine;

(8aR,12aS,13aS)-2,3-dihydroxy-5,6,8a,9,10,11,12,12a,13,13a-decahydro-8H-isoquino[2,1-g][1,6]-naphthyridine;

(8aS,12aR,13aR)-2,3-dihydroxy-5,6,8a,9,10,11,12,12a,13,13a-decahydro-8H-isoquino[2,1-g][1,6]-

naphthyridine;

(8aR,12aS,13aS)-1,2-dimethoxy-5,6,8a,9,10,11,12,12a,13,13a-decahydro-8H-isoquino[2,1-g][1,6]-naphthyridine;

(8aS,12aR,13aR)-1,2-dimethoxy-5,6,8a,9,10,11,12,12a,13,13a-decahydro-8H-isoquino[2,1-g][1,6]-naphthyridine;

(8aR,12aS,13aS)-1,4-dimethoxy-5,6,8a,9,10,11,12,12a,13,13a-decahydro-8H-isoquino[2,1-g][1,6]-naphthyridine;

(8aS,12aR,13aR)-1,4-dimethoxy-5,6,8a,9,10,11,12,12a,13,13a-decahydro-8H-isoquino[2,1-g][1,6]-naphthyridine;

(8aR,12aS,13aS)-3,4-dimethoxy-5,6,8a,9,10,11,12,12a,13,13a-decahydro-8H-isoquino[2,1-g][1,6]-naphthyridine;

(8aS,12aR,13aR)-3,4-dimethoxy-5,6,8a,9,10,11,12,12a,13,13a-decahydro-8H-isoquino[2,1-g][1,6]-naphthyridine;

(8aR,12aS,13aS)-2,3-diethoxy-5,6,8a,9,10,11,12,12a,13,13a-decahydro-8H-isoquino[2,1-g][1,6]-naphthyridine;

(8aS,12aR,13aR)-2,3-diethoxy-5,6,8a,9,10,11,12,12a,13,13a-decahydro-8H-isoquino[2,1-g][1,6]-naphthyridine;

(8aR,12aS,13aS)-2,3-di-n-butoxy-5,6,8a,9,10,11,12,12a,13,13a-decahydro-8H-isoquino[2,1-g][1,6]-naphthyridine;

(8aS,12aR,13aR)-2,3-di-n-butoxy-5,6,8a,9,10,11,12,12a,13,13a-decahydro-8H-isoquino[2,1-g][1,6]-naphthyridine;

(8aR,12aS,13aS)-1,2-methylenedioxy-5,6,8a,9,10,11,12,12a,13,13a-decahydro-8H-isoquino[2,1-g][1,6]-naphthyridine;

(8aS,12aR,13aR)-1,2-methylenedioxy-5,6,8a,9,10,11,12,12a,13,13a-decahydro-8H-isoquino[2,1-g]-[1,6]naphthyridine;

(8aR,12aS,13aS)-2-chloro-5,6,8a,9,10,11,12,12a,13,13a-decahydro-8H-isoquino[2,1-g][1,6]-naphthyridine;

(8aS,12aR,13aR)-2-chloro-5,6,8a,9,10,11,12,12a,13,13a-decahydro-8H-isoquino[2,1-g][1,6]-naphthyridine;

(8aR,12aS,13aS)-3-chloro-5,6,8a,9,10,11,12,12a,13,13a-decahydro-8H-isoquino[2,1-g][1,6]-naphthyridine;

(8aS,12aR,13aR)-3-chloro-5,6,8a,9,10,11,12,12a,13,13a-decahydro-8H-isoquino[2,1-g][1,6]-naphthyridine;

(8aR,12aS,13aS)-4-chloro-5,6,8a,9,10,11,12,12a,13,13a-decahydro-8H-isoquino[2,1-g][1,6]-naphthyridine;

(8aS,12aR,13aR)-4-chloro-5,6,8a,9,10,11,12,12a,13,13a-decahydro-8H-isoquino[2,1-g][1,6]-naphthyridine;

(8aR,12aS,13aS)-3-bromo-5,6,8a,9,10,11,12,12a,13,13a-decahydro-8H-isoquino[2,1-g][1,6]-naphthyridine;

(8aS,12aR,13aR)-3-bromo-5,6,8a,9,10,11,12,12a,13,13a-decahydro-8H-isoquino[2,1-g][1,6]-naphthyridine;

(8aR,12aS,13aS)-3-fluoro-5,6,8a,9,10,11,12,12a,13,13a-decahydro-8H-isoquino[2,1-g][1,6]-naphthyridine;

(8aS,12aR,13aR)-3-fluoro-5,6,8a,9,10,11,12,12a,13,13a-decahydro-8H-isoquino[2,1-g][1,6]-naphthyridine;

(8aR,12aS,13aS)-2-fluoro-5,6,8a,9,10,11,12,12a,13,13a-decahydro-8H-isoquino[2,1-g][1,6]-naphthyridine;

(8aS,12aR,13aR)-2-fluoro-5,6,8a,9,10,11,12,12a,13,13a-decahydro-8H-isoquino[2,1-g][1,6]-naphthyridine.

D. Similarly, replacing (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine with other compounds of formula (XVII) and following the procedure in paragraph A above, the following exemplary compounds of formula (XVIII) were prepared:

(8aR,12aS,13aS)-3-methoxy-5,6,8a,9,10,11,12,12a,13,13a-decahydro-8H-isoquino[2,1-g][1,6]-naphthyridine;

mp 110-112 °C, $[\alpha]_D^{25}$ = +101.5 ($CHCl_3$)

(8aR,12aS,13aR)-2,3-methylenedioxy-5,6,8a,9,10,11,12,12a,13,13a-decahydro-8H-isoquino[2,1-g]-[1,6]naphthyridine,

E. Similarly, replacing (8aR,12aS,13aS)-3-methoxy-12-(1-R-phenethylaminocarbonyl)-5,6,8a,9,10,11,12,12a,13,13a-decahydro-8H-isoquino[2,1-g][1,6]naphthyridine with other compounds of formula (XVII) and following the procedure in paragraph A above, the following exemplary compounds of formula (XVIII) are prepared:

(8aR,12aS,13aR)-5,6,8a,9,10,11,12,12a,13,13a-decahydro-8H-isoquino[2,1-g][1,6]naphthyridine;

(8aR,12aS,13aR)-2,3-dimethoxy-5,6,8a,9,10,11,12,12a,13,13a-decahydro-8H-isoquino[2,1-g][1,6]-naphthyridine;

(8aR,12aS,13aR)-1,4-dimethoxy-5,6,8a,9,10,11,12,12a,13,13a-decahydro-8H-isoquino[2,1-g][1,6]-naphthyridine;

(8aR,12aS,13aR)-2,3-(ethylene-1,2-dioxy)-5,6,8a,9,10,11,12,12a,13,13a-decahydro-8H-isoquino[2,1-g]-[1,6]naphthyridine;

(8aR,12aS,13aR)-1-methyl-5,6,8a,9,10,11,12,12a,13,13a-decahydro-8H-isoquino[2,1-g][1,6]-naphthyridine;

(8aR,12aS,13aR)-2-methyl-5,6,8a,9,10,11,12,12a,13,13a-decahydro-8H-isoquino[2,1-g][1,6]-naphthyridine;

(8aR,12aS,13aR)-3-methyl-5,6,8a,9,10,11,12,12a,13,13a-decahydro-8H-isoquino[2,1-g][1,6]-naphthyridine;

(8aR,12aS,13aR)-2,3-dimethyl-5,6,8a,9,10,11,12,12a,13,13a-decahydro-8H-isoquino[2,1-g][1,6]-naphthyridine;

(8aR,12aS,13aR)-3-ethyl-5,6,8a,9,10,11,12,12a,13,13a-decahydro-8H-isoquino-[2,1-g][1,6]-naphthyridine;

(8aR,12aS,13aR)-3-isobutyl-5,6,8a,9,10,11,12,12a,13,13a-decahydro-8H-isoquino[2,1-g][1,6]-naphthyridine;

(8aR,12aS,13aR)-3-n-hexyl-5,6,8a,9,10,11,12,12a,13,13a-decahydro-8H-isoquino[2,1-g][1,6]-naphthyridine;

(8aR,12aS,13aR)-1-methoxy-5,6,8a,9,10,11,12,12a,13,13a-decahydro-8H-isoquino[2,1-g][1,6]-naphthyridine;

(8aR,12aS,13aR)-2-methoxy-5,6,8a,9,10,11,12,12a,13,13a-decahydro-8H-isoquino[2,1-g][1,6]-naphthyridine;

(8aR,12aS,13aR)-4-methoxy-5,6,8a,9,10,11,12,12a,13,13a-decahydro-8H-isoquino[2,1-g][1,6]-naphthyridine;

(8aR,12aS,13aR)-3-methoxy-2-methyl-5,6,8a,9,10,11,12,12a,13,13a-decahydro-8H-isoquino[2,1-g]-[1,6]naphthyridine;

(8aR,12aS,13aR)-3-ethoxy-5,6,8a,9,10,11,12,12a,13,13a-decahydro-8H-isoquino[2,1-g][1,6]-naphthyridine;

(8aR,12aS,13aR)-3-isopropoxy-5,6,8a,9,10,11,12,12a,13,13a-decahydro-8H-isoquino[2,1-g][1,6]-naphthyridine;

(8aR,12aS,13aR)-3-isopropoxy-5,6,8a,9,10,11,12,12a,13,13a-decahydro-8H-isoquino[2,1-g][1,6]-naphthyridine;

(8aR,12aS,13aR)-3-n-hexyloxy-5,6,8a,9,10,11,12,12a,13,13a-decahydro-8H-isoquino[2,1-g][1,6]-naphthyridine;

(8aR,12aS,13aR)-3-hydroxy-5,6,8a,9,10,11,12,12a,13,13a-decahydro-8H-isoquino[2,1-g][1,6]-naphthyridine;

(8aR,12aS,13aR)-2,3-dihydroxy-5,6,8a,9,10,11,12,12a,13,13a-decahydro-8H-isoquino[2,1-g][1,6]-naphthyridine;

(8aR,12aS,13aR)-1,2-dimethoxy-5,6,8a,9,10,11,12,12a,13,13a-decahydro-8H-isoquino[2,1-g][1,6]-naphthyridine;

(8aR,12aS,13aR)-1,4-dimethoxy-5,6,8a,9,10,11,12,12a,13,13a-decahydro-8H-isoquino[2,1-g][1,6]-naphthyridine;

(8aR,12aS,13aR)-3,4-dimethoxy-5,6,8a,9,10,11,12,12a,13,13a-decahydro-8H-isoquino[2,1-g][1,6]-naphthyridine;

(8aR,12aS,13aR)-2,3-diethoxy-5,6,8a,9,10,11,12,12a,13,13a-decahydro-8H-isoquino[2,1-g][1,6]-naphthyridine;

(8aR,12aS,13aR)-2,3-di-n-butoxy-5,6,8a,9,10,11,12,12a,13,13a-decahydro-8H-isoquino[2,1-g][1,6]-naphthyridine;

(8aR,12aS,13aR)-1,2-methylenedioxy-5,6,8a,9,10,11,12,12a,13,13a-decahydro-8H-isoquino[2,1-g]-[1,6]naphthyridine;

(8aR,12aS,13aR)-2-chloro-5,6,8a,9,10,11,12,12a,13,13a-decahydro-8H-isoquino[2,1-g][1,6]-

naphthyridine;

(8aR,12aS,13aR)-3-chloro-5,6,8a,9,10,11,12,12a,13,13a-decahydro-8H-isoquino[2,1-g][1,6]-naphthyridine;

(8aR,12aS,13aR)-4-chloro-5,6,8a,9,10,11,12,12a,13,13a-decahydro-8H-isoquino[2,1-g][1,6]-naphthyridine;

(8aR,12aS,13aR)-3-bromo-5,6,8a,9,10,11,12,12a,13,13a-decahydro-8H-isoquino[2,1-g][1,6]-naphthyridine;

(8aR,12aS,13aR)-3-fluoro-5,6,8a,9,10,11,12,12a,13,13a-decahydro-8H-isoquino[2,1-g][1,6]-naphthyridine; and

(8aR,12aS,13aR)-2-fluoro-5,6,8a,9,10,11,12,12a,13,13a-decahydro-8H-isoquino[2,1-g][1,6]-naphthyridine.

EXAMPLE 1A

Preparation of (±)-12-Methanesulfonyl-5,6,8aα,9,10,11,12,12aα,13,13aα-decahydro-8H-isoquino[2,1g][1,6]-naphthyridine hydrochloride and Related Compounds of Formula (1) and (2)

A. A solution of 0.4 g of (±)-5,6,8aα,9,10,11,12,12aα,13,13aα-decahydro-8H-isoquino[2,1-g][1,6]-naphthyridine (VII) in 15 ml of methylene chloride and 0.5 ml of triethylamine was cooled in an ice bath and 0.5 ml of methanesulfonyl chloride was added. The mixture was stirred at room temperature for 1 hour, diluted with 100 ml of ether, and extracted with dilute HCl. The aqueous HCl layer was basified with $NH_4OH$ and extracted with methylene chloride. The methylene chloride was evaporated to a residue which was dissolved in ethanol and acidified with ethanolic HCl. Crystallization was induced by adding a small amount of diethylether. Filtration afforded 0.4 g of (±)-12-methanesulfonyl-5,6,8aα,9,10,11,12,12aα,13,13aα-decahydro-8H-isoquino[2,1-g][1,6]naphthyridine hydrochloride, m.p. 234-235 °C.

B. Similarly, replacing (±)-5,6,8aα,9,10,11,12,12aα,13,13aα-decahydro-8H-isoquino[2,1-g][1,6]-naphthyridine with the appropriate compound of formula (VII) or (VIII), optionally replacing methanesulfonyl chloride with other sulfonyl halides of formula $ZSO_2R$, where Z is chlorine or bromine and R is as defined supra, and following the procedure in paragraph A above, the following compounds of formula (1) and (2) were prepared:

(±)-12-methanesulfonyl-5,6,8aβ,9,10,11,12,12aβ,13,13aα-decahydro-8H-isoquino[2,1-g][1,6]-naphthyridine hydrochloride, m.p. 230-235 °C;

(±)-12-(1-butanesulfonyl)-5,6,8aβ,9,10,11,12,12aβ,13,13aα-decahydro-8H-isoquino[2,1-g][1,6]-naphthyridine hydrochloride, m.p. 215-216 °C;

(±)-12-ethanesulfonyl-5,6,8aα,9,10,11,12,12aα,13,13aα-decahydro-8H-isoquino[2,1-g][1,6]-naphthyridine hydrochloride, m.p. 203-204 °C;

(±)-3-methoxy-12-methanesulfonyl-5,6,8aα,9,10,11,12,12aα,13,13aα-decahydro-8H-isoquino[2,1-g]-[1,6]naphthyridine hydrochloride, m.p. 265-266 °C;

(±)-3-methoxy-12-methanesulfonyl-5,6,8aβ,9,10,11,12,12aβ,13,13aα-decahydro-8H-isoquino[2,1-g]-[1,6]naphthyridine hydrochloride, m.p. 238-239 °C;

(±)-3-methoxy-12-(2-methylpropanesulfonyl)-5,6,8aα,9,10,11,12,12aα,13,13aα-decahydro-8H-isoquino-[2,1-g][1,6]naphthyridine hydrochloride, m.p. 127-130 °C;

(±)-2,3-dimethoxy-12-methanesulfonyl-5,6,8α,9,10,11,12,12aα,13,13aα-decahydro-8H-isoquino[2,1-g]-[1,6]naphthyridine hydrochloride, m.p. 175-177 °C;

(±)-1,4-dimethoxy-12-methanesulfonyl-5,6,8aα,9,10,11,12,12aα,13,13aα-decahydro-8H-isoquino[2,1-g][1,6]naphthyridine hydrochloride, m.p. 194-195 °C;

(±)-2,3-dimethoxy-12-(2-methylpropanesulfonyl)-5,6,8aα,9,10,11,12,12aα,13,13aα-decahydro-8H-isoquino[2,1-g][1,6]naphthyridine hydrochloride, m.p. 155-156 °C;

(±)-2,3-methylenedioxy-12-methanesulfonyl-5,6,8aα,9,10,11,12,12aα,13,13aα-decahydro-8H-isoquino-[2,1-g][1,6]naphthyridine hydrochloride, m.p. 279-280 °C;

(±)-2,3-(ethylene-1,2-dioxy)-12-methanesulfonyl-5,6,8aα,9,10,11,12,12aα,13,13aα-decahydro-8H-isoquino[2,1-g][1,6]naphthyridine hydrochloride, m.p. 279-280 °C;

(±)-2,3-methylenedioxy-12-(2-methylpropanesulfonyl)-5,6,8aα,9,10,11,12,12aα,13,13aα-decahydro-8H-isoquino[2,1-g][1,6]naphthyridine hydrochloride, m.p. 172-174 °C;

(±)-12-(1-butanesulfonyl)-5,6,8aα,9,10,11,12,12aα,13,13aα-decahydro-8H-isoquino[2,1-g][1,6]-naphthyridine hydrochloride, m.p. 220-222 °C;

(±)-12-(1-propanesulfonyl)-5,6,8aα,9,10,11,12,12aα,13,13aα-decahydro-8H-isoquino[2,1-g][1,6]-

naphthyridine hydrochloride, m.p. 235-236 °C;

(±)-12-(2-methylpropanesulfonyl)-5,6,8aα,9,10,11,12,12aα,13,13aα-decahydro-8H-isoquino[2,1-g][1,6]-naphthyridine hydrochloride, m.p. 220-221 °C;

(±)-12-phenylsulfonyl-5,6,8aα,9,10,11,12,12aα,13,13aα-decahydro-8H-isoquino[2,1-g][1,6]-naphthyridine hydrochloride, m.p. 247-248 °C;

(±)-12-(4-methoxyphenylsulfonyl)-5,6,8aα,9,10,11,12,12aα,13,13aα-decahydro-8H-isoquino[2,1-g][1,6]-naphthyridine hydrochloride, m.p. 256-257 °C;

(±)-12-(4-chlorophenylsulfonyl)-5,6,8aα,9,10,11,12,12aα,13,13aα-decahydro-8H-isoquino[2,1-g][1,6]-naphthyridine hydrochloride, m.p. 261-263 °C;

(±)-12-(4-fluorophenylsulfonyl)-5,6,8aα,9,10,11,12,12aα,13,13aα-decahydro-8H-isoquino[2,1-g][1,6]-naphthyridine hydrochloride, m.p. 258-259 °C;

(±)-12-(2-methoxyethanesulfonyl)-5,6,8aα,9,10,11,12,12aα,13,13aα-decahydro-8H-isoquino[2,1-g][1,6]-naphthyridine hydrochloride, m.p. 173-174 °C;

(±)-3-methoxy-12-(2-methoxyethanesulfonyl)-5,6,8aα,9,10,11,12,12aα,13,13aα-decahydro-8H-isoquino[2,1-g][1,6]naphthyridine hydrochloride, m.p. 173-174 °C; and

(±)-12-(N,N-dimethylaminosulfonyl)-5,6,8aα,9,10,11,12,12aα,13,13aα-decahydro-8H-isoquino[2,1-g]-[1,6]naphthyridine hydrochloride, m.p. 237-238 °C.

C. Similarly, replacing (±)-5,6,8aα,9,10,11,12,12aα,13,13aα-decahydro-8H-isoquino[2,1-g][1,6]-naphthyridine with other compounds of formula (VII) or (VIII), optionally replacing methanesulfonyl chloride with other sulfonyl halides of formula $ZSO_2R$ and following the procedure in paragraph A above, the following compounds of formula (1) and (2) are prepared:

(±)-12-ethanesulfonyl-5,6,8aβ,9,10,11,12,12aβ,13,13aα-decahydro-8H-isoquino[2,1-g][1,6]-naphthyridine hydrochloride;

(±)-3-methoxy-12-(2-methylpropanesulfonyl)-5,6,8aβ,9,10,11,12,12aβ,13,13aα-decahydro-8H-isoquino[2,1-g][1,6]naphthyridine hydrochloride;

(±)-2,3-dimethoxy-12-methanesulfonyl-5,6,8aβ,9,10,11,12,12aβ,13,13aα-decahydro-8H-isoquino[2,1-g][1,6]naphthyridine hydrochloride;

(±)-2,3-dimethoxy-12-(2-methylpropanesulfonyl)-5,6,8aβ,9,10,11,12,12aβ,13,13aα-decahydro-8H-isoquino[2,1-g][1,6]naphthyridine hydrochloride;

(±)-2,3-methylenedioxy-12-methanesulfonyl-5,6,8aβ,9,10,11,12,12aβ,13,13aα-decahydro-8H-isoquino-[2,1-g][1,6]naphthyridine hydrochloride;

(±)-2,3-methylenedioxy-12-(2-methylpropanesulfonyl)-5,6,8aβ,9,10,11,12,12aβ,13,13aα-decahydro-8H-isoquino[2,1-g][1,6]naphthyridine hydrochloride;

(±)-12-(2-methylpropanesulfonyl)-5,6,8aβ,9,10,11,12,12aβ,13,13aα-decahydro-8H-isoquino[2,1-g][1,6]-naphthyridine hydrochloride;

(±)-12-phenylsulfonyl-5,6,8aβ,9,10,11,12,12aβ,13,13aα-decahydro-8H-isoquino[2,1-g][1,6]-naphthyridine hydrochloride;

(±)-12-(4-methoxyphenylsulfonyl)-5,6,8aβ,9,10,11,12,12aβ,13,13aα-decahydro-8H-isoquino[2,1-g][1,6]-naphthyridine hydrochloride;

(±)-12-(4-chlorophenylsulfonyl)-5,6,8aβ,9,10,11,12,12aβ,13,13aα-decahydro-8H-isoquino[2,1-g][1,6]-naphthyridine hydrochloride;

(±)-12-(4-aminophenylsulfonyl)-5,6,8aβ,9,10,11,12,12aβ,13,13aα-decahydro-8H-isoquino[2,1-g][1,6]-naphthyridine hydrochloride;

(±)-12-(4-fluorophenylsulfonyl)-5,6,8aβ,9,10,11,12,12aβ,13,13aα-decahydro-8H-isoquino[2,1-g][1,6]-naphthyridine hydrochloride;

(±)-12-(2-methoxyethanesulfonyl)-5,6,8aβ,9,10,11,12,12aβ,13,13aα-decahydro-8H-isoquino[2,1-g][1,6]-naphthyridine hydrochloride;

(±)-12-(2-hydroxyethanesulfonyl)-5,6,8aβ,9,10,11,12,12aβ,13,13aα-decahydro-8H-isoquino[2,1-g][1,6]-naphthyridine hydrochloride;

(±)-12-(N,N-dimethylaminosulfonyl)-5,6,8aβ,9,10,11,12,12aβ,13,13aα-decahydro-8H-isoquino[2,1-g]-[1,6]naphthyridine hydrochloride;

(±)-3-methyl-12-methanesulfonyl-5,6,8aα,9,10,11,12,12aα,13,13aα-decahydro-8H-isoquino[2,1-g][1,6]-naphthyridine hydrochloride;

(±)-2-methyl-12-methanesulfonyl-5,6,8aβ,9,10,11,12,12aβ,13,13aα-decahydro-8H-isoquino[2,1-g][1,6]-naphthyridine hydrochloride;

(±)-2,3-dimethyl-12-methanesulfonyl-5,6,8aα,9,10,11,12,12aα,13,13aα-decahydro-8H-isoquino[2,1-g]-[1,6]naphthyridine hydrochloride;

(±)-2,3-dimethyl-12-methanesulfonyl-5,6,8aβ,9,10,11,12,12aβ,13,13aα-decahydro-8H-isoquino[2,1-g]-

[1,6]naphthyridine hydrochloride;

(±)-2-n-hexyl-12-methanesulfonyl-5,6,8aα,9,10,11,12,12aα,13,13aα-decahydro-8H-isoquino[2,1-g][1,6]-naphthyridine hydrochloride;

(±)-2-n-hexyl-12-methanesulfonyl-5,6,8aβ,9,10,11,12,12aβ,13,13aα-decahydro-8H-isoquino[2,1-g][1,6]-naphthyridine hydrochloride;

(±)-2-methoxy-12-methanesulfonyl-5,6,8aα,9,10,11,12,12aα,13,13aα-decahydro-8H-isoquino[2,1-g]-[1,6]naphthyridine hydrochloride;

(±)-2-methoxy-12-methanesulfonyl-5,6,8aβ,9,10,11,12,12aβ,13,13aα-decahydro-8H-isoquino[2,1-g]-[1,6]naphthyridine hydrochloride;

(±)-3-ethoxy-12-methanesulfonyl-5,6,8aα,9,10,11,12,12aα,13,13aα-decahydro-8H-isoquino[2,1-g][1,6]-naphthyridine hydrochloride;

(±)-3-ethoxy-12-methanesulfonyl-5,6,8aβ,9,10,11,12,12aβ,13,13aα-decahydro-8H-isoquino[2,1-g][1,6]-naphthyridine hydrochloride;

(±)-3-isobutoxy-12-methanesulfonyl-5,6,8aα,9,10,11,12,12aα,13,13aα-decahydro-8H-isoquino[2,1-g]-[1,6]naphthyridine hydrochloride;

(±)-3-isobutoxy-12-methanesulfonyl-5,6,8aβ,9,10,11,12,12aβ,13,13aα-decahydro-8H-isoquino[2,1-g]-[1,6]naphthyridine hydrochloride;

(±)-3-n-hexyloxy-12-methanesulfonyl-5,6,8aα,9,10,11,12,12aα,13,13aα-decahydro-8H-isoquino[2,1-g]-[1,6]naphthyridine hydrochloride;

(±)-3-n-hexyloxy-12-methanesulfonyl-5,6,8aβ,9,10,11,12,12aβ,13,13aα-decahydro-8H-isoquino[2,1-g]-[1,6]naphthyridine hydrochloride;

(±)-3-hydroxy-12-methanesulfonyl-5,6,8aα,9,10,11,12,12aα,13,13aα-decahydro-8H-isoquino[2,1-g]-[1,6]naphthyridine hydrochloride;

(±)-3-hydroxy-12-methanesulfonyl-5,6,8aβ,9,10,11,12,12aβ,13,13aα-decahydro-8H-isoquino[2,1-g]-[1,6]naphthyridine hydrochloride;

(±)-2,3-dihydroxy-12-methanesulfonyl-5,6,8aα,9,10,11,12,12aα,13,13aα-decahydro-8H-isoquino[2,1-g]-[1,6]naphthyridine hydrochloride;

(±)-2,3-dihydroxy-12-methanesulfonyl-5,6,8aβ,9,10,11,12,12aβ,13,13aα-decahydro-8H-isoquino[2,1-g]-[1,6]naphthyridine hydrochloride;

(±)-1,2-dimethoxy-12-methanesulfonyl-5,6,8aα,9,10,11,12,12aα,13,13aα-decahydro-8H-isoquino[2,1-g][1,6]naphthyridine hydrochloride;

(±)-1,2-dimethoxy-12-methanesulfonyl-5,6,8aβ,9,10,11,12,12aβ,13,13aα-decahydro-8H-isoquino[2,1-g][1,6]naphthyridine hydrochloride;

(±)-1,4-dimethoxy-12-methanesulfonyl-5,6,8aα,9,10,11,12,12aα,13,13aα-decahydro-8H-isoquino[2,1-g][1,6]naphthyridine hydrochloride;

(±)-1,4-dimethoxy-12-methanesulfonyl-5,6,8aβ,9,10,11,12,12aβ,13,13aα-decahydro-8H-isoquino[2,1-g][1,6]naphthyridine hydrochloride;

(±)-2,3-diethoxy-12-methanesulfonyl-5,6,8aα,9,10,11,12,12aα,13,13aα-decahydro-8H-isoquino[2,1-g]-[1,6]naphthyridine hydrochloride;

(±)-2,3-diethoxy-12-methanesulfonyl-5,6,8aβ,9,10,11,12,12aβ,13,13aα-decahydro-8H-isoquino[2,1-g]-[1,6]naphthyridine hydrochloride;

(±)-2,3-di-n-butoxy-12-methanesulfonyl-5,6,8aα,9,10,11,12,12aα,13,13aα-decahydro-8H-isoquino[2,1-g][1,6]naphthyridine hydrochloride;

(±)-2,3-di-n-butoxy-12-methanesulfonyl-5,6,8aβ,9,10,11,12,12aβ,13,13aα-decahydro-8H-isoquino[2,1-g][1,6]naphthyridine hydrochloride;

(±)-3,4-methylenedioxy-12-methanesulfonyl-5,6,8aα,9,10,11,12,12aα,13,13aα-decahydro-8H-isoquino-[2,1-g][1,6]naphthyridine hydrochloride;

(±)-3,4-methylenedioxy-12-methanesulfonyl-5,6,8aβ,9,10,11,12,12aβ,13,13aα-decahydro-8H-isoquino-[2,1-g][1,6]naphthyridine hydrochloride;

(±)-2-chloro-12-methanesulfonyl-5,6,8aα,9,10,11,12,12aα,13,13aα-decahydro-8H-isoquino[2,1-g][1,6]-naphthyridine hydrochloride;

(±)-2-chloro-12-methanesulfonyl-5,6,8aβ,9,10,11,12,12aβ,13,13aα-decahydro-8H-isoquino[2,1-g][1,6]-naphthyridine hydrochloride;

(±)-3-chloro-12-methanesulfonyl-5,6,8aα,9,10,11,12,12aα,13,13aα-decahydro-8H-isoquino[2,1-g][1,6]-naphthyridine hydrochloride;

(±)-3-chloro-12-methanesulfonyl-5,6,8aβ,9,10,11,12,12aβ,13,13aα-decahydro-8H-isoquino[2,1-g][1,6]-naphthyridine hydrochloride;

(±)-3-fluoro-12-methanesulfonyl-5,6,8aα,9,10,11,12,12aα,13,13aα-decahydro-8H-isoquino[2,1-g][1,6]-

naphthyridine hydrochloride;

(±)-3-fluoro-12-methanesulfonyl-5,6,8aβ,9,10,11,12,12aβ,13,13aα-decahydro-8H-isoquino[2,1-g][1,6]-naphthyridine hydrochloride;

(±)-12-aminosulfonyl-5,6,8aα,9,10,11,12,12aα,13,13aα-decahydro-8H-isoquino[2,1-g][1,6]naphthyridine hydrochloride;

(±)-12-aminosulfonyl-5,6,8aβ,9,10,11,12,12aβ,13,13aα-decahydro-8H-isoquino[2,1-g][1,6]-naphthyridine hydrochloride;

(±)-12-methylaminosulfonyl-5,6,8aα,9,10,11,12,12aα,13,13aα-decahydro-8H-isoquino[2,1-g][1,6]-naphthyridine hydrochloride;

(±)-12-methylaminosulfonyl-5,6,8aβ,9,10,11,12,12aβ,13,13aα-decahydro-8H-isoquino[2,1-g][1,6]-naphthyridine hydrochloride;

(±)-12-diethylaminosulfonyl-5,6,8aα,9,10,11,12,12aα,13,13aα-decahydro-8H-isoquino[2,1-g][1,6]-naphthyridine hydrochloride;

(±)-12-diethylaminosulfonyl-5,6,8aβ,9,10,11,12,12aβ,13,13aα-decahydro-8H-isoquino[2,1-g][1,6]-naphthyridine hydrochloride;

(±)-12-di-n-hexylaminosulfonyl-5,6,8aα,9,10,11,12,12aα,13,13aα-decahydro-8H-isoquino[2,1-g][1,6]-naphthyridine hydrochloride;

(±)-12-di-n-hexylaminosulfonyl-5,6,8aβ,9,10,11,12,12aβ,13,13aα-decahydro-8H-isoquino[2,1-g][1,6]-naphthyridine hydrochloride;

(±)-12-(1-piperazinosulfonyl)-5,6,8aα,9,10,11,12,12aα,13,13aα-decahydro-8H-isoquino[2,1-g][1,6]-naphthyridine hydrochloride;

(±)-12-(1-piperazinosulfonyl)-5,6,8aβ,9,10,11,12,12aβ,13,13aα-decahydro-8H-isoquino[2,1-g][1,6]-naphthyridine hydrochloride;

(±)-12-(1-morpholinosulfonyl)-5,6,8aα,9,10,11,12,12aα,13,13aα-decahydro-8H-isoquino[2,1-g][1,6]-naphthyridine hydrochloride;

(±)-12-(1-morpholinosulfonyl)-5,6,8aβ,9,10,11,12,12aβ,13,13aα-decahydro-8H-isoquino[2,1-g][1,6]-naphthyridine hydrochloride;

(±)-12-(1-piperidinosulfonyl)-5,6,8aα,9,10,11,12,12aα,13,13aα-decahydro-8H-isoquino[2,1-g][1,6]-naphthyridine hydrochloride;

(±)-12-(1-piperidinosulfonyl)-5,6,8aβ,9,10,11,12,12aβ,13,13aα-decahydro-8H-isoquino[2,1-g][1,6]-naphthyridine hydrochloride;

(±)-3-methyl-12-ethanesulfonyl-5,6,8aα,9,10,11,12,12aα,13,13aα-decahydro-8H-isoquino[2,1-g][1,6]-naphthyridine hydrochloride;

(±)-3-methyl-12-ethanesulfonyl-5,6,8aβ,9,10,11,12,12aβ,13,13aα-decahydro-8H-isoquino[2,1-g][1,6]-naphthyridine hydrochloride;

(±)-3-ethoxy-12-ethanesulfonyl-5,6,8aα,9,10,11,12,12aα,13,13aα-decahydro-8H-isoquino[2,1-g][1,6]-naphthyridine hydrochloride;

(±)-3-ethoxy-12-ethanesulfonyl-5,6,8aβ,9,10,11,12,12aβ,13,13aα-decahydro-8H-isoquino[2,1-g][1,6]-naphthyridine hydrochloride;

(±)-3-n-hexyloxy-12-ethanesulfonyl-5,6,8aα,9,10,11,12,12aα,13,13aα-decahydro-8H-isoquino[2,1-g]-[1,6]naphthyridine hydrochloride;

(±)-3-n-hexyloxy-12-ethanesulfonyl-5,6,8aβ,9,10,11,12,12aβ,13,13aα-decahydro-8H-isoquino[2,1-g]-[1,6]naphthyridine hydrochloride;

(±)-2,3-diethoxy-12-ethanesulfonyl-5,6,8aα,9,10,11,12,12aα,13,13aα-decahydro-8H-isoquino[2,1-g]-[1,6]naphthyridine hydrochloride;

(±)-2,3-diethoxy-12-ethanesulfonyl-5,6,8aβ,9,10,11,12,12aβ,13,13aα-decahydro-8H-isoquino[2,1-g]-[1,6]naphthyridine hydrochloride;

(±)-3-chloro-12-ethanesulfonyl-5,6,8aα,9,10,11,12,12aα,13,13aα-decahydro-8H-isoquino[2,1-g][1,6]-naphthyridine hydrochloride;

(±)-3-chloro-12-ethanesulfonyl-5,6,8aβ,9,10,11,12,12aβ,13,13aα-decahydro-8H-isoquino[2,1-g][1,6]-naphthyridine hydrochloride;

(±)-3-methyl-12-(1-n-hexanesulfonyl)-5,6,8aα,9,10,11,12,12aα,13,13aα-decahydro-8H-isoquino[2,1-g]-[1,6]naphthyridine hydrochloride;

(±)-3-methoxy-12-(1-n-hexanesulfonyl)-5,6,8aβ,9,10,11,12,12aβ,13,13aα-decahydro-8H-isoquino[2,1-g][1,6]naphthyridine hydrochloride;

(±)-3-methoxy-12-(1-n-hexanesulfonyl)-5,6,8aα,9,10,11,12,12aα,13,13aα-decahydro-8H-isoquino[2,1-g][1,6]naphthyridine hydrochloride;

(±)-2,3-dimethoxy-12-(1-n-hexanesulfonyl)-5,6,8aβ,9,10,11,12,12aβ,13,13aα-decahydro-8H-isoquino-

[2,1-g][1,6]naphthyridine hydrochloride;

(±)-2,3-dimethoxy-12-(1-n-hexanesulfonyl)-5,6,8aα,9,10,11,12,12aα,13,13aα-decahydro-8H-isoquino-[2,1-g][1,6]naphthyridine hydrochloride;

(±)-3-chloro-12-(1-n-hexanesulfonyl)-5,6,8aα,9,10,11,12,12aα,13,13aα-decahydro-8H-isoquino[2,1-g]-[1,6]naphthyridine hydrochloride;

(±)-3-chloro-12-(1-n-hexanesulfonyl)-5,6,8aβ,9,10,11,12,12aβ,13,13aα-decahydro-8H-isoquino[2,1-g]-[1,6]naphthyridine hydrochloride;

(±)-3-methoxy-12-phenylsulfonyl-5,6,8aα,9,10,11,12,12aα,13,13aα-decahydro-8H-isoquino[2,1-g][1,6]-naphthyridine hydrochloride;

(±)-3-methoxy-12-phenylsulfonyl-5,6,8aβ,9,10,11,12,12aβ,13,13aα-decahydro-8H-isoquino[2,1-g][1,6]-naphthyridine hydrochloride;

(±)-3-methyl-12-(N,N-dimethylaminosulfonyl)-5,6,8aα,9,10,11,12,12aα,13,13aα-decahydro-8H-isoquino[2,1-g][1,6]naphthyridine hydrochloride;

(±)-3-methyl-12-(N,N-dimethylaminosulfonyl)-5,6,8aβ,9,10,11,12,12aβ,13,13aα-decahydro-8H-isoquino[2,1-g][1,6]naphthyridine hydrochloride;

(±)-3-methoxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-3-methoxy-12-(N,N-dimethylaminosufonyl)-5,6,8aβ,9,10,11,12,12aβ,13,13aα-decahydro-8H-isoquino[2,1-g][1,6]naphthyridine hydrochloride;

(±)-2,3-dimethoxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-2,3-dimethoxy-12-(N,N-dimethylaminosulfonyl)-5,6,8aβ,9,10,11,12,12aβ,13,13aα-decahydro-8H-isoquino[2,1-g][1,6]naphthyridine hydrochloride;

(±)-12-(2-methoxypropanesulfonyl)-5,6,8aα,9,10,11,12,12aα,13,13aα-decahydro-8H-isoquino[2,1-g]-[1,6]naphthyridine hydrochloride;

(±)-12-(2-methoxypropanesulfonyl)-5,6,8aβ,9,10,11,12,12aβ,13,13aα-decahydro-8H-isoquino[2,1-g]-[1,6]naphthyridine hydrochloride;

(±)-12-(2-methoxyhexanesulfonyl)-5,6,8aα,9,10,11,12,12aα,13,13aα-decahydro-8H-isoquino[2,1-g]-[1,6]naphthyridine hydrochloride;

(±)-12-(2-methoxyhexanesulfonyl)-5,6,8aβ,9,10,11,12,12aβ,13,13aα-decahydro-8H-isoquino[2,1-g]-[1,6]naphthyridine hydrochloride;

(±)-2-methyl-12-(2-methoxymethanesulfonyl)-5,6,8aα,9,10,11,12,12aα,13,13aα-decahydro-8H-isoquino[2,1-g][1,6]naphthyridine hydrochloride;

(±)-2-methyl-12-(2-methoxymethanesulfonyl)-5,6,8aβ,9,10,11,12,12aβ,13,13aα-decahydro-8H-isoquino[2,1-g][1,6]naphthyridine hydrochloride;

(±)-2,3-methylenedioxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-2,3-methylenedioxy-12-(N,N-dimethylaminosulfonyl)-5,6,8aβ,9,10,11,12,12aβ,13,13aα-decahydro-8H-isoquino[2,1-g][1,6]naphthyridine hydrochloride;

(±)-3-n-hexyloxy-12-ethanesulfonyl-5,6,8aα,9,10,11,12,12aα,13,13aα-decahydro-8H-isoquino[2,1-g]-[1,6]naphthyridine hydrochloride;

(±)-3-n-hexyloxy-12-ethanesulfonyl-5,6,8aβ,9,10,11,12,12aβ,13,13aα-decahydro-8H-isoquino[2,1-g]-[1,6]naphthyridine hydrochloride;

(±)-2,3-diethoxy-12-ethanesulfonyl-5,6,8aα,9,10,11,12,12aα,13,13aα-decahydro-8H-isoquino[2,1-g]-[1,6]naphthyridine hydrochloride;

(±)-2,3-diethoxy-12-ethanesulfonyl-5,6,8aβ,9,10,11,12,12aβ,13,13aα-decahydro-8H-isoquino[2,1-g]-[1,6]naphthyridine hydrochloride;

(±)-3-chloro-12-ethanesulfonyl-5,6,8aα,9,10,11,12,12aα,13,13aα-decahydro-8H-isoquino[2,1-g][1,6]-naphthyridine hydrochloride; and

(±)-3-chloro-12-ethanesulfonyl-5,6,8aβ,9,10,11,12,12aβ,13,13aα-decahydro-8H-isoquino[2,1-g][1,6]-naphthyridine hydrochloride.

## EXAMPLE 1B

Preparation of (8aR,12aS,13aS)-3-methoxy-12-methanesulfonyl-5,6,8a,9,10,11,12,12a,13,13a-decahydro-8H-isoquino[2,1g][1,6]naphthyridine, Pharmaceutically Acceptable Salts Thereof and Related Compounds of Formula (1) and (2)

A. A solution of 0.4 g of (8aR,12aS,13aS)-3-methoxy-5,6,8a,9,10,11,12,12a,13,13a-decahydro-8H-isoquino[2,1-g][1,6]naphthyridine (XIII) in 15 ml of methylene chloride and 0.5 ml of triethylamine was cooled in an ice bath and 0.5 ml of methanesulfonyl chloride was added. The mixture was stirred at room temperature for 1 hour, diluted with 100 ml of ether, and extracted with dilute HCl. The aqueous HCl layer was basified with $NH_4OH$ and extracted with methylene chloride. The methylene chloride was evaporated to afford the title compound, mp 165-166 °C $[\alpha]_D^{25}$ = -55.5 ($CHCl_3$).

The residue was optionally dissolved in ethanol and acidified with ethanolic HCl. Crystallization was induced by adding a small amount of diethylether.

Filtration afforded 0.4 g of (8aR,12aS,13aS)-3-methoxy-12-methanesulfonyl-5,6,8a,9,10,11,12,12a,13,13a-decahydro-8H-isoquino[2,1-g][1,6]napthyridine hydrochloride,

mp 256-258 °C, $[\alpha]_D^{25}$ = +13.1 ($CHCl_3$).

Similarly, proceeding as in the optional step described above, but substituting inorganic acids such as hydrobromic acid, sulfuric acid, nitric acid, phosphoric acid and the like, or organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like, for hydrochloric acid, the corresponding salts are prepared. For example:

(8aR,12aS,13aS)-3-methoxy-12-methanesulfonyl-5,6,8a,9,10,-11,12,12a,13,13a-decahydro-8H-isoquino[2,1-g][1,6]naphthyridine hydrobromide;

(8aR,12aS,13aS)-3-methoxy-12-methanesulfonyl-5,6,8a,9,10,-11,12,12a,13,13a-decahydro-8H-isoquino[2,1-g][1,6]naphthyridinium sulfate;

(8aR,12aS,13aS)-3-methoxy-12-methanesulfonyl-5,6,8a,9,10,-11,12,12a,13,13a-decahydro-8H-isoquino[2,1-g][1,6]naphthyridinium nitrate;

(8aR,12aS,13aS)-3-methoxy-12-methanesulfonyl-5,6,8a,9,10,-11,12,12a,13,13a-decahydro-8H-isoquino[2,1-g][1,6]naphthyridinium phosphate;

(8aR,12aS,13aS)-3-methoxy-12-methanesulfonyl-5,6,8a,9,10,-11,12,12a,13,13a-decahydro-8H-isoquino[2,1-g][1,6]naphthyridinium acetate;

(8aR,12aS,13aS)-3-methoxy-12-methanesulfonyl-5,6,8a,9,10,-11,12,12a,13,13a-decahydro-8H-isoquino[2,1-g][1,6]naphthyridinium propionate;

(8aR,12aS,13aS)-3-methoxy-12-methanesulfonyl-5,6,8a,9,10,-11,12,12a,13,13a-decahydro-8H-isoquino[2,1-g][1,6]naphthyridinium glycolate;

(8aR,12aS,13aS)-3-methoxy-12-methanesulfonyl-5,6,8a,9,10,-11,12,12a,13,13a-decahydro-8H-isoquino[2,1-g][1,6]naphthyridinium pyruvate;

(8aR,12aS,13aS)-3-methoxy-12-methanesulfonyl-5,6,8a,9,10,-11,12,12a,13,13a-decahydro-8H-isoquino[2,1-g][1,6]naphthyridinium oxalate;

(8aR,12aS,13aS)-3-methoxy-12-methanesulfonyl-5,6,8a,9,10,-11,12,12a,13,13a-decahydro-8H-isoquino[2,1-g][1,6]naphthyridinium malate;

(8aR,12aS,13aS)-3-methoxy-12-methanesulfonyl-5,6,8a,9,10,-11,12,12a,13,13a-decahydro-8H-isoquino[2,1-g][1,6]naphthyridinium malonate;

(8aR,12aS,13aS)-3-methoxy-12-methanesulfonyl-5,6,8a,9,10,-11,12,12a,13,13a-decahydro-8H-isoquino[2,1-g][1,6]naphthyridinium succinate;

(8aR,12aS,13aS)-3-methoxy-12-methanesulfonyl-5,6,8a,9,10,-11,12,12a,13,13a-decahydro-8H-isoquino[2,1-g][1,6]naphthyridinium maleate;

(8aR,12aS,13aS)-3-methoxy-12-methanesulfonyl-5,6,8a,9,10,-11,12,12a,13,13a-decahydro-8H-isoquino[2,1-g][1,6]naphthyridinium fumarate;

(8aR,12aS,13aS)-3-methoxy-12-methanesulfonyl-5,6,8a,9,10,-11,12,12a,13,13a-decahydro-8H-isoquino[2,1-g][1,6]naphthyridinium tartrate;

(8aR,12aS,13aS)-3-methoxy-12-methanesulfonyl-5,6,8a,9,10,-11,12,12a,13,13a-decahydro-8H-isoquino[2,1-g][1,6]naphthyridinium citrate;

(8aR,12aS,13aS)-3-methoxy-12-methanesulfonyl-5,6,8a,9,10,-11,12,12a,13,13a-decahydro-8H-isoquino[2,1-g][1,6]naphthyridinium benzoate;

(8aR,12aS,13aS)-3-methoxy-12-methanesulfonyl-5,6,8a,9,10,-11,12,12a,13,13a-decahydro-8H-isoquino[2,1-g][1,6]-naphthyridinium cinnamate;

(8aR,12aS,13aS)-3-methoxy-12-methanesulfonyl-5,6,8a,9,10,-11,12,12a,13,13a-decahydro-8H-isoquino[2,1-g][1,6]-naphthyridinium mandelate;

(8aR,12aS,13aS)-3-methoxy-12-methanesulfonyl-5,6,8a,9,10,-11,12,12a,13,13a-decahydro-8H-isoquino[2,1-g][1,6]-naphthyridinium methanesulfonate;

(8aR,12aS,13aS)-3-methoxy-12-methanesulfonyl-5,6,8a,9,10,-11,12,12a,13,13a-decahydro-8H-isoquino[2,1-g][1,6]-naphthyridinium ethanesulfonate;

(8aR,12aS,13aS)-3-methoxy-12-methanesulfonyl-5,6,8a,9,10,-11,12,12a,13,13a-decahydro-8H-isoquino[2,1-g][1,6]-naphthyridinium p-toluenesulfonate; and

(8aR,12aS,13aS)-3-methoxy-12-methanesulfonyl-5,6,8a,9,10,-11,12,12a,13,13a-decahydro-8H-isoquino[2,1-g][1,6]-naphthyridinium salicylate.

B. Similarly, optionally replacing (8aR,12aS,13aS)-3-methoxy-5,6,8aα,9,10,11,12,12a,13,13a-decahydro-8H-isoquino[2,1-g][1,6]naphthyridine with other compounds of formula (XIII), optionally replacing methanesulfonyl chloride with other sulfonyl halides of formula $ZSO_2R$ and following the procedure in paragraph A above, the following compounds of formula (1) were prepared:

(8aR,12aS,13aS)-3-methoxy-12-N,N-dimethylaminosulfonyl-5,6,8a,9,10,11,12,12a,13,13a-decahydro-8H-isoquino[2,1-g][1,6]naphthyridine hydrochloride,
mp 242-243°C, $[\alpha]_D^{25}$ = +8.21 ($CH_3OH$).

(8aR,12aS,13aS)-12-methanesulfonyl-5,6,8a,9,10,11,12,12a,13,13a-decahydro-8H-isoquino[2,1-g][1,6]-naphthyridine hydrochloride,
mp 266-267°C, $[\alpha]_D^{25}$ = +0.1 ($H_2O$)

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrochloride,
mp 263-265°C, $[\alpha]_D^{25}$ = -17.0($CH_3OH$)

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine;

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrobromide;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium sulfate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium nitrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium phosphate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium acetate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium propionate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium glycolate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium pyruvate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium oxalate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium malate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium malonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium succinate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium maleate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium fumarate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium tartrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium citrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium benzoate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium cinnamate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium mandelate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium methanesulfonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium ethanesulfonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium p-toluenesulfonate; and

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium salicylate.

C. Similarly, optionally replacing (8aR,12aS,13aS)-3-methoxy-5,6,8a,9,10,11,12,12a,13,13a-decahydro-8H-isoquino[2,1-g][1,6]naphthyridine with compounds of formula (XVIII), optionally replacing methanesulfonyl chloride with other sulfonyl halides of formula $ZSO_2R$ and following the procedure in paragraph A above, the following compounds of formula (2) were prepared:

(8aR,12aS,13aS)-3-methoxy-12-methanesulfonyl-5,6,8a,9,10,11,12,12a,13,13a-decahydro-8H-isoquino[2,1-g][1,6]naphthyridine,
mp 140-143°C, $[\alpha]_D^{25} = +17.6$ ($CHCl_3$).

(8aR,12aS,13aR)-2,3-methylenedioxy-12-methanesulfonyl-5,6,8a,9,10,11,12,12a,13,13a-decahydro-8H-isoquino[2,1-g][1,6]naphthyridine,
mp 219-220°C, $[\alpha]_D^{25} = +45.3$ ($CHCl_3$)

D. Similarly, optionally replacing (8aR,12aS,13aS)-3-methoxy-5,6,8aα,9,10,11,12,12a,13,13a-decahydro-8H-isoquino[2,1-g][1,6]naphthyridine with other compounds of formula (XIII), optionally replacing methanesulfonyl chloride with other sulfonyl halides of formula $ZSO_2R$ and following the procedure in paragraph A above, the following compounds of formula (1) are prepared:

(8aR,12aS,13aS)-12-ethanesulfonyl-5,6,8a,9,10,11,12,12a,13,13a-decahydro-8H-isoquino[2,1-g][1,6]-naphthyridine hydrochloride;

(8aR,12aS,13aS)-12-(1-propanesulfonyl)-5,6,8a,9,10,11,12,12a,13,13a-decahydro-8H-isoquino[2,1-g]-[1,6]naphthyridine hydrochloride;

(8aR,12aS,13aS)-12-(1-butanesulfonyl)-5,6,8a,9,10,11,12,12a,13,13a-decahydro-8H-isoquino[2,1-g]-[1,6]naphthyridine hydrochloride;

(8aR,12aS,13aS)-12-(2-methylpropanesulfonyl)-5,6,8a,9,10,11,12,12a,13,13a-decahydro-8H-isoquino-[2,1-g][1,6]naphthyridine hydrochloride;

(8aR,12aS,13aS)-12-phenylsulfonyl-5,6,8a,9,10,11,12,12a,13,13a-decahydro-8H-isoquino[2,1-g][1,6]-naphthyridine hydrochloride;

(8aR,12aS,13aS)-12-(4-methoxyphenylsulfonyl)-5,6,8a,9,10,11,12,12a,13,13a-decahydro-8H-isoquino-[2,1-g][1,6]naphthyridine hydrochloride;

(8aR,12aS,13aS)-12-(4-chlorophenylsulfonyl)-5,6,8a,9,10,11,12,12a,13,13a-decahydro-8H-isoquino-[2,1-g][1,6]naphthyridine hydrochloride;

(8aR,12aS,13aS)-12-(4-fluorophenylsulfonyl)-5,6,8a,9,10,11,12,12a,13,13a-decahydro-8H-isoquino[2,1-g][1,6]naphthyridine hydrochloride;

(8aR,12aS,13aS)-12-(2-methoxyethanesulfonyl)-5,6,8a,9,10,11,12,12a,13,13a-decahydro-8H-isoquino-[2,1-g][1,6]naphthyridine hydrochloride;

(8aR,12aS,13aS)-3-methoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(1-propanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-piperazinosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-morpholinosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-piperidinosulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-1,4-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-dimethoxy-12-(2-methylpropanesulfonyl)-5,6,8a,9,10,11,12,12a,13,13a-decahydro-8H-isoquino[2,1-g][1,6]naphthyridinehydrochloride;

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-(ethylene-1,2-dioxy)-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-methylenedioxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(2-methoxyethanesulfonyl)-5,6,8a,9,10,11,12,12a,13,13a-decahydro-8H-isoquino[2,1-g][1,6]naphthyridine hydrochloride;

(8aR,12as,13aS)-12-(4-aminophenylsulfonyl)-5,6,8a,9,10,11,12,12a,13,13a-decahydro-8H-isoquino[2,1-g][1,6]naphthyridine hydrochloride;

(8aR,12aS,13aS)-12-(2-hydroxyethanesulfonyl)-5,6,8a,9,10,11,12,12a,13,13a-decahydro-8H-isoquino-[2,1-g][1,6]naphthyridine hydrochloride;

(8aR,12aS,13aS)-1-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-ethyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2,3-dimethyl-12-methanesulfonyl-5,6,8a,9,10,11,12,12a,13,13a-decahydro-8H-isoquino[2,1-g][1,6]naphthyridine hydrochloride;

(8aR,12aS,13aS)-3-isobutyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2-n-hexyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-methoxy-2-methyl-12-methanesulfonyl-5,6,8a,9,10,11,12,12a,13,13a-decahydro-8H-isoquino[2,1-g][1,6]naphthyridine hydrochloride;

(8aR,12aS,13aS)-1-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-4-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-ethoxy-12-methanesulfonyl-5,6,8a,9,10,11,12,12a,13,13a-decahydro-8H-isoquino-[2,1-g][1,6]naphthyridine hydrochloride;

(8aR,12aS,13aS)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-isopropoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-isobutoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-n-hexyloxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-hydroxy-12-methanesulfonyl-5,6,8a,9,10,11,12,12a,13,13a-decahydro-8H-isoquino-[2,1-g][1,6]naphthyridine hydrochloride;

(8aR,12aS,13aS)-2,3-dihydroxy-12-methanesulfonyl-5,6,8a,9,10,11,12,12a,13,13a-decahydro-8H-isoquino[2,1-g][1,6]naphthyridine hydrochloride;

(8aR,12aS,13aS)-1,2-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-diethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-di-n-butoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3,4-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aS)-2-chloro-12-methanesulfonyl,5,6,8a,9,10,11,12,12a,13,13a-decahydro-8H-isoquino-[2,1-g][1,6]naphthyridine hydrochloride;

(8aR,12aS,13aS)-3-chloro-12-methanesulfonyl-5,6,8a,9,10,11,12,12a,13,13a-decahydro-8H-isoquino-[2,1-g][1,6]naphthyridine hydrochloride;

(8aR,12aS,13aS)-2-fluoro-12-methanesulfonyl-5,6,8a,9,10,11,12,12a,13,13a-decahydro-8H-isoquino-[2,1-g][1,6]naphthyridine hydrochloride;

(8aR,12aS,13aS)-3-fluoro-12-methanesulfonyl-5,6,8a,9,10,11,12,12a,13,13a-decahydro-8H-isoquino-[2,1-g][1,6]naphthyridine hydrochloride;

(8aR,12aS,13aS)-12-aminosulfonyl-5,6,8a,9,10,11,12,12a,13,13a-decahydro-8H-isoquino[2,1-g][1,6]-naphthyridine hydrochloride;

(8aR,12aS,13aS)-3-methoxy-12-aminosulfonyl-5,6,8a,9,10,11,12,12a,13,13a-decahydro-8H-isoquino-[2,1-g][1,6]naphthyridine hydrochloride;

(8aR,12aS,13aS)-12-methylaminosulfonyl-5,6,8a,9,10,11,12,12a,13,13a-decahydro-8H-isoquino[2,1-g]-[1,6]naphthyridine hydrochloride;

(8aR,12aS,13aS)-12-diethylaminosulfonyl-5,6,8a,9,10,11,12,12a,13,13a-decahydro-8H-isoquino[2,1-g]-[1,6]naphthyridine hydrochloride;

(8aR,12aS,13aS)-12-di-n-hexylaminosulfonyl-5,6,8a,9,10,11,12,12a,13,13a-decahydro-8H-isoquino-[2,1-g][1,6]naphthyridine hydrochloride;

(8aR,12aS,13aS)-12-(1-piperazinosulfonyl)-5,6,8a,9,10,11,12,12a,13,13a-decahydro-8H-isoquino[2,1-g][1,6]naphthyridine hydrochloride;

(8aR,12aS,13aS)-12-(1-morpholinosulfonyl)-5,6,8a,9,10,11,12,12a,13,13a-decahydro-8H-isoquino[2,1-g][1,6]naphthyridine hydrochloride;

(8aR,12aS,13aS)-12-(1-piperidinosulfonyl)-5,6,8a,9,10,11,12,12a,13,13a-decahydro-8H-isoquino[2,1-g][1,6]naphthyridine hydrochloride;

(8aR,12aS,13aS)-3-methyl-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-n-hexyloxy-12-ethanesulfonyl-5,6,8a,9,10,11,12,12a,13,13a-decahydro-8H-isoquino[2,1-g][1,6]naphthyridine hydrochloride;

(8aR,12aS,13aS)-2,3-diethoxy-12-ethanesulfonyl-5,6,8a,9,10,11,12,12a,13,13a-decahydro-8H-isoquino[2,1-g][1,6]naphthyridine hydrochloride;

(8aR,12aS,13aS)-3-methyl-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(N,N-dimethylaminosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(t-butylaminosulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-(N,N-dimethylaminosulfonyl)-5,6,8a,9,10,11,12,12a,13,13a-decahydro-8H-isoquino[2,1-g][1,6]naphthyridine hydrochloride;

(8aR,12aS,13aS)-2,3-methylenedioxy-12-(N,N-dimethylaminosulfonyl)-5,6,8a,9,10,11,12,12a,13,13a-decahydro-8H-isoquino[2,1-g][1,6]naphthyridine hydrochloride; and

(8aR,12aS,13aS)-2-methyl-12-(2-methoxymethanesulfonyl)-5,6,8a,9,10,11,12,12a,13,13a-decahydro-8H-isoquino[2,1-g][1,6]naphthyridine hydrochloride.

E. Similarly, optionally replacing (8aR,12aS,13aS)-3-methoxy-5,6,8a$\alpha$,9,10,11,12,12a,13,13a-decahydro-8H-isoquino[2,1-g][1,6]naphthyridine with other compounds of formula (XVIII), optionally replacing methanesulfonyl chloride with other sulfonyl halides of formula $ZSO_2R$ and following the procedure in paragraph A above, the following compounds of formula (2) are prepared:

(8aR,12aS,13aR)-12-ethanesulfonyl-5,6,8a,9,10,11,12,12a,13,13a-decahydro-8H-isoquino[2,1-g][1,6]-naphthyridine hydrochloride;

(8aR,12aS,13aR)-12-(1-propanesulfonyl-5,6,8a,9,10,11,12,12a,13,13a-decahydro-8H-isoquino[2,1-g]-[1,6]naphthyridine hydrochloride;

(8aR,12aS,13aR)-12-(1-butanesulfonyl)-5,6,8a,9,10,11,12,12a,13,13a-decahydro-8H-isoquino[2,1-g]-[1,6]naphthyridine hydrochloride;

(8aR,12aS,13aR)-12-(2-methylpropanesulfonyl)-5,6,8a,9,10,11,12,12a,13,13a-decahydro-8H-isoquino-[2,1-g][1,6]naphthyridine hydrochloride;

(8aR,12aS,13aR)-12-phenylsulfonyl-5,6,8a,9,10,11,12,12a,13,13a-decahydro-8H-isoquino[2,1-g][1,6]-naphthyridine hydrochloride;

(8aR,12aS,13aR)-12-(4-methoxyphenylsulfonyl)-5,6,8a,9,10,11,12,12a,13,13a-decahydro-8H-isoquino-[2,1-g][1,6]naphthyridine hydrochloride;

(8aR,12aS,13aR)-12-(4-chlorophenylsulfonyl)-5,6,8a,9,10,11,12,12a,13,13a-decahydro-8H-isoquino-[2,1-g][1,6]naphthyridine hydrochloride;

(8aR,12aS,13aR)-12-(4-fluorophenylsulfonyl)-5,6,8a,9,10,11,12,12a,13,13a-decahydro-8H-isoquino[2,1-g][1,6]naphthyridine hydrochloride;

(8aR,12aS,13aR)-12-(2-methoxyethanesulfonyl)-5,6,8a,9,10,11,12,12a,13,13a-decahydro-8H-isoquino-[2,1-g][1,6]naphthyridine hydrochloride;

(8aR,12aS,13aR)-3-methoxy-12-ethanesulfonyl-5,6,8a,9,10,11,12,12a,13,13a-decahydro-8H-isoquino-[2,1-g][1,6]naphthyridine hydrochloride;

(8aR,12aS,13aR)-3-methoxy-12-(1-propanesulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aR)-3-methoxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(1-piperazinosulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(1-morpholinosulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(1-piperidinosulfonyl)-5,6,8a,9,10,11,12,12a,13,13a-decahydro-8H-isoquino[2,1-g][1,6]naphthyridine hydrochloride;

(8aR,12aS,13aR)-2,3-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aR)-1,4-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aR)-2,3-dimethoxy-12-(2-methylpropanesulfonyl)-5,6,8a,9,10,11,12,12a,13,13a-decahydro-8H-isoquino[2,1-g][1,6]naphthyridinehydrochloride;

(8aR,12aS,13aR)-2,3-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aR)-2,3-(ethylene-1,2-dioxy)-12-methanesulfonyl-5,6,8a,9,10,11,12,12a,13,13a-decahydro-8H-isoquino[2,1-g][1,6]naphthyridine hydrochloride;

(8aR,12aS,13aR)-2,3-methylenedioxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(2-methoxyethanesulfonyl)-5,6,8a,9,10,11,12,12a,13,13a-decahydro-8H-isoquino[2,1-g][1,6]naphthyridine hydrochloride;

(8aR,12aS,13aR)-12-(4-aminophenylsulfonyl)-5,6,8a,9,10,11,12,12a,13,13a-decahydro-8H-isoquino-[2,1-g][1,6]naphthyridine hydrochloride;

(8aR,12aS,13aR)-12-(2-hydroxyethanesulfonyl)-5,6,8a,9,10,11,12,12a,13,13a-decahydro-8H-isoquino-[2,1-g][1,6]naphthyridine hydrochloride;

(8aR,12aS,13aR)-1-methyl-12-methanesulfonyl-5,6,8a,9,10,11,12,12a,13,13a-decahydro-8H-isoquino-[2,1-g][1,6]naphthyridine hydrochloride;

(8aR,12aS,13aR)-2-methyl-12-methanesulfonyl-5,6,8a,9,10,11,12,12a,13,13a-decahydro-8H-isoquino-[2,1-g][1,6]naphthyridine hydrochloride;

(8aR,12aS,13aR)-3-methyl-12-methanesulfonyl-5,6,8a,9,10,11,12,12a,13,13a-decahydro-8H-isoquino-[2,1-g][1,6]naphthyridine hydrochloride;

(8aR,12aS,13aR)-3-ethyl-12-methanesulfonyl-5,6,8a,9,10,11,12,12a,13,13a-decahydro-8H-isoquino-[2,1-g][1,6]naphthyridine hydrochloride;

(8aR,12aS,13aR)-2,3-dimethyl-12-methanesulfonyl-5,6,8a,9,10,11,12,12a,13,13a-decahydro-8H-isoquino[2,1-g][1,6]naphthyridine hydrochloride;

(8aR,12aS,13aR)-3-isobutyl-12-methanesulfonyl-5,6,8a,9,10,11,12,12a,13,13a-decahydro-8H-isoquino-[2,1-g][1,6]naphthyridine hydrochloride;

(8aR,12aS,13aR)-2-n-hexyl-12-methanesulfonyl-5,6,8a,9,10,11,12,12a,13,13a-decahydro-8H-isoquino-[2,1-g][1,6]naphthyridine hydrochloride;

(8aR,12aS,13aR)-3-methoxy-2-methyl-12-methanesulfonyl-5,6,8a,9,10,11,12,12a,13,13a-decahydro-8H-isoquino[2,1-g][1,6]naphthyridine hydrochloride;

(8aR,12aS,13aR)-1-methoxy-12-methanesulfonyl-5,6,8a,9,10,11,12,12a,13,13a-decahydro-8H-isoquino[2,1-g][1,6]naphthyridine hydrochloride;

(8aR,12aS,13aR)-2-methoxy-12-methanesulfonyl-5,6,8a,9,10,11,12,12a,13,13a-decahydro-8H-isoquino[2,1-g][1,6]naphthyridine hydrochloride;

(8aR,12aS,13aR)-4-methoxy-12-methanesulfonyl-5,6,8a,9,10,11,12,12a,13,13a-decahydro-8H-isoquino[2,1-g][1,6]naphthyridine hydrochloride;

(8aR,12aS,13aR)-3-ethoxy-12-methanesulfonyl-5,6,8a,9,10,11,12,12a,13,13a-decahydro-8H-isoquino-[2,1-g][1,6]naphthyridine hydrochloride;

(8aR,12aS,13aR)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aR)-3-isopropoxy-12-methanesulfonyl-5,6,8a,9,10,11,12,12a,13,13a-decahydro-8H-isoquino[2,1-g][1,6]naphthyridine hydrochloride;

(8aR,12aS,13aR)-3-isobutoxy-12-methanesulfonyl-5,6,8a,9,10,11,12,12a,13,13a-decahydro-8H-isoquino[2,1-g][1,6]naphthyridine hydrochloride;

(8aR,12aS,13aR)-3-n-hexyloxy-12-methanesulfonyl-5,6,8a,9,10,11,12,12a,13,13a-decahydro-8H-isoquino[2,1-g][1,6]naphthyridine hydrochloride;

(8aR,12aS,13aR)-3-hydroxy-12-methanesulfonyl-5,6,8a,9,10,11,12,12a,13,13a-decahydro-8H-isoquino-[2,1-g][1,6]naphthyridine hydrochloride;

(8aR,12aS,13aR)-2,3-dihydroxy-12-methanesulfonyl-5,6,8a,9,10,11,12,12a,13,13a-decahydro-8H-isoquino[2,1-g][1,6]naphthyridine hydrochloride;

(8aR,12aS,13aR)-1,2-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aR)-2,3-diethoxy-12-methanesulfonyl-5,6,8a,9,10,11,12,12a,13,13a-decahydro-8H-isoquino[2,1-g][1,6]naphthyridine hydrochloride;

(8aR,12aS,13aR)-2,3-di-n-butoxy-12-methanesulfonyl-5,6,8a,9,10,11,12,12a,13,13a-decahydro-8H-isoquino[2,1-g][1,6]naphthyridine hydrochloride;

(8aR,12aS,13aR)-3,4-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aR)-2-chloro-12-methanesulfonyl-5,6,8a,9,10,11,12,12a,13,13a-decahydro-8H-isoquino-[2,1-g][1,6]naphthyridine hydrochloride;

(8aR,12aS,13aR)-3-chloro-12-methanesulfonyl-5,6,8a,9,10,11,12,12a,13,13a-decahydro-8H-isoquino-[2,1-g][1,6]naphthyridine hydrochloride;

(8aR,12aS,13aR)-2-fluoro-12-methanesulfonyl-5,6,8a,9,10,11,12,12a,13,13a-decahydro-8H-isoquino-[2,1-g][1,6]naphthyridine hydrochloride;

(8aR,12aS,13aR)-3-fluoro-12-methanesulfonyl-5,6,8a,9,10,11,12,12a,13,13a-decahydro-8H-isoquino-[2,1-g][1,6]naphthyridine hydrochloride;

(8aR,12aS,13aR)-12-aminosulfonyl-5,6,8a,9,10,11,12,12a,13,13a-decahydro-8H-isoquino[2,1-g][1,6]-naphthyridine hydrochloride;

(8aR,12aS,13aR)-3-methoxy-12-aminosulfonyl-5,6,8a,9,10,11,12,12a,13,13a-decahydro-8H-isoquino-[2,1-g][1,6]naphthyridine hydrochloride;

(8aR,12aS,13aR)-12-methylaminosulfonyl-5,6,8a,9,10,11,12,12a,13,13a-decahydro-8H-isoquino[2,1-g]-[1,6]naphthyridine hydrochloride;

(8aR,12aS,13aR)-12-diethylaminosulfonyl-5,6,8a,9,10,11,12,12a,13,13a-decahydro-8H-isoquino[2,1-g]-[1,6]naphthyridine hydrochloride;

(8aR,12aS,13aR)-12-di-n-hexylaminosulfonyl-5,6,8a,9,10,11,12,12a,13,13a-decahydro-8H-isoquino-[2,1-g][1,6]naphthyridine hydrochloride;

(8aR,12aS,13aR)-12-(1-piperazinosulfonyl)-5,6,8a,9,10,11,12,12a,13,13a-decahydro-8H-isoquino[2,1-g][1,6]naphthyridine hydrochloride;

(8aR,12aS,13aR)-12-(1-morpholinosulfonyl)-5,6,8a,9,10,11,12,12a,13,13a-decahydro-8H-isoquino[2,1-g][1,6]naphthyridine hydrochloride;

(8aR,12aS,13aR)-12-(1-piperidinosulfonyl)-5,6,8a,9,10,11,12,12a,13,13a-decahydro-8H-isoquino[2,1-g][1,6]naphthyridine hydrochloride;

(8aR,12aS,13aR)-3-methyl-12-ethanesulfonyl-5,6,8a,9,10,11,12,12a,13,13a-decahydro-8H-isoquino-[2,1-g][1,6]naphthyridine hydrochloride;

(8aR,12aS,13aR)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aR)-3-n-hexyloxy-12-ethanesulfonyl-5,6,8a,9,10,11,12,12a,13,13a-decahydro-8H-isoquino[2,1-g][1,6]naphthyridine hydrochloride;

(8aR,12aS,13aR)-2,3-diethoxy-12-ethanesulfonyl-5,6,8a,9,10,11,12,12a,13,13a-decahydro-8H-isoquino[2,1-g][1,6]naphthyridine hydrochloride;

(8aR,12aS,13aR)-3-methyl-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aR)-2,3-dimethoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aR)-3-methoxy-12-(N,N-dimethylaminosulfonyl)-5,6,8a,9,10,11,12,12a,13,13a-decahydro-8H-isoquino[2,1-g][1,6]naphthyridine hydrochloride;

(8aR,12aS,13aR)-3-methoxy-12-(t-butylaminosulfonyl)-5,6,8a,9,10,11,12,12a,13,13a-decahydro-8H-isoquino[2,1-g][1,6]naphthyridine hydrochloride;

(8aR,12aS,13aR)-2,3-dimethoxy-12-(N,N-dimethylaminosulfonyl)-5,6,8a,9,10,11,12,12a,13,13a-decahydro-8H-isoquino[2,1-g][1,6]naphthyridine hydrochloride;

(8aR,12aS,13aR)-2,3-methylenedioxy-12-(N,N-dimethylaminosulfonyl)-5,6,8a,9,10,11,12,12a,13,13a-decahydro-8H-isoquino[2,1-g][1,6]naphthyridine hydrochloride; and

(8aR,12aS,13aR)-2-methyl-12-(2-methoxymethanesulfonyl)-5,6,8a,9,10,11,12,12a,13,13a-decahydro-8H-isoquino[2,1-g][1,6]naphthyridine hydrochloride.

## EXAMPLE 1C

Alternative Preparation of (±)-12-Methanesulfonyl-5,6,8aα,9,10,11,12,12a, α,13,13a α-decahydro-8H-isoquino-[2,1-g][1,6]naphthyridine hydrochloride and Related Compounds of Formula (1)

A. A mixture of 1.14 g of (±)-12-methanesulfonyl-5,6,8aβ,9,10,11,12,12aβ,13,13aα-decahydro-8H-isoquino[2,1-g][1,6]naphthyridine (a compound of formula (2)) and 4.53 g of mercuric acetate in 20 ml of acetic acid and 2 ml of water was stirred at 105 °C for 1 hour. The mixture was filtered and hydrogen sulfide was bubbled through the filtrate for 5 minutes. The mixture was filtered again and the filtrate was concentrated under reduced pressure. Ethanol (50 ml) was added and the resulting solution was cooled to -20 °C and treated with 0.5 g of sodium borohydride. The solution was allowed to warm to room temperature and acidified with aqueous HCl. After washing with ethyl acetate, the aqueous layer was basified with $NH_4OH$ and extracted with ethyl acetate. The ethyl acetate was washed with brine, dried over anhydrous sodium sulfate and evaporated. Chromatography of the residue on silica gel, eluting with 1% methanol in methylene chloride, afforded the crystalline (±)-12-methanesulfonyl-5,6,8aα,9,10,11,12,12aα,13,13aα-decahydro-8H-isoquino[2,1-g][1,6]naphthyridine, m.p. XX °C. The hydrochloride salt was crystallized from ethanol: yield 0.4 g : m.p. 234-235 °C.

B. Similarly, replacing (±)-12-methanesulfonyl-5,6,8aβ,9,10,11,12,12aβ,13,13aα-decahydro-8H-isoquino-[2,1-g][1,6]naphthyridine with other compounds of formula (2) and following the procedure in paragraph A above, the following compounds of formula (1) are prepared:

(±)-12-ethanesulfonyl-5,6,8aα,9,10,11,12,12aα,13,13aα-decahydro-8H-isoquino[2,1-g][1,6]-naphthyridine;

(±)-3-methoxy-12-methanesulfonyl-5,6,8aα,9,10,11,12,12aα,13,13a,α-decahydro-8H-isoquino[2,1-g]-[1,6]naphthyridine;

(±)-3-methoxy-12-(2-methylpropanesulfonyl)-5,6,8aα,9,10,11,12,12aα,13,13aα-decahydro-8H-isoquino-[2,1-g][1,6]naphthyridine;

(±)-2,3-dimethoxy-12-methanesulfonyl-5,6,8aα,9,10,11,12,12aα,13,13aα-decahydro-8H-isoquino[2,1-g][1,6]naphthyridine;

(±)-1,4-dimethoxy-12-methanesulfonyl-5,6,8aα,9,10,11,12,12aα,13,13aα-decahydro-8H-isoquino[2,1-g][1,6]naphthyridine;

(±)-2,3-dimethoxy-12-(2-methylpropanesulfonyl)-5,6,8aα,9,10,11,12,12aα,13,13aα-decahydro-8H-isoquino[2,1-g][1,6]naphthyridine;

(±)-2,3-methylenedioxy-12-methanesulfonyl-5,6,8aα,9,10,11,12,12aα,13,13aα-decahydro-8H-isoquino-

[2,1-g][1,6]naphthyridine;

(±)-2,3-methylenedioxy-12-(2-methylpropanesulfonyl)-5,6,-8aα,9,10,11,12,12aα,13,13aα-decahydro-8H-isoquino[2,1-g][1,6]naphthyridine;

(±)-12-(1-butanesulfonyl)-5,6,8aα,9,10,11,12,12aα,13,13aα-decahydro-8H-isoquino[2,1-g][1,6]-naphthyridine;

(±)-12-(1-propanesulfonyl)-5,6,8aα,9,10,11,12,12aα,13,13aα-decahydro-8H-isoquino[2,1-g][1,6]-naphthyridine;

(±)-12-(2-methylpropanesulfonyl)-5,6,8aα,9,10,11,12,12aα,13,13aα-decahydro-8H-isoquino[2,1-g][1,6]-naphthyridine;

(±)-12-phenylsulfonyl-5,6,8aα,9,10,11,12,12aα,13,13aα-decahydro-8H-isoquino[2,1-g][1,6]-naphthyridine;

(±)-12-(4-methoxyphenylsulfonyl)-5,6,8aα,9,10,11,12,12aα,13,13aα-decahydro-8H-isoquino[2,1-g][1,6]-naphthyridine;

(±)-12-(4-chlorophenylsulfonyl)-5,6,8aα,9,10,11,12,12aα,13,13aα-decahydro-8H-isoquino[2,1-g][1,6]-naphthyridine;

(±)-12-(4-aminophenylsulfonyl)-5,6,8aα,9,10,11,12,12aα,13,13aα-decahydro-8H-isoquino[2,1-g]1,6]-naphthyridine;

(±)-12-(4-fluorophenylsulfonyl)-5,6,8aα,9,10,11,12,12aα,13,13aα-decahydro-8H-isoquino[2,1-g][1,6]-naphthyridine;

(±)-12-(2-methoxyethanesulfonyl)-5,6,8aα,9,10,11,12,12aα,13,13aα-decahydro-8H-isoquino[2,1-g][1,6]-naphthyridine;

(±)-12-(2-hydroxyethanesulfonyl)-5,6,8aα,9,10,11,12,12aα,13,13aα-decahydro-8H-isoquino[2,1-g][1,6]-naphthyridine; and

(±)-12-(N,N-dimethylaminosulfonyl)-5,6,8aα,9,10,11,12,12aα,13,13aα-decahydro-8H-isoquino[2,1-g]-[1,6]naphthyridine;

(±)-3-methyl-12-methanesulfonyl-5,6,8aα,9,10,11,12,12aα,13,13aα-decahydro-8H-isoquino[2,1-g][1,6]-naphthyridine hydrochloride;

(±)-2,3-dimethyl-12-methanesulfonyl-5,6,8aα,9,10,11,12,12aα,13,13aα-decahydro-8H-isoquino[2,1-g]-[1,6]naphthyridine hydrochloride;

(±)-2-n-hexyl-12-methanesulfonyl-5,6,8aα,9,10,11,12,12aα,13,13aα-decahydro-8H-isoquino[2,1-g][1,6]-naphthyridine hydrochloride;

(±)-2-methoxy-12-methanesulfonyl-5,6,8aα,9,10,11,12,12aα,13,13aα-decahydro-8H-isoquino[2,1-g]-[1,6]naphthyridine hydrochloride;

(±)-3-ethoxy-12-methanesulfonyl-5,6,8aα,9,10,11,12,12aα-13,13aα-decahydro-8H-isoquino[2,1-g][1,6]-naphthyridine hydrochloride;

(±)-3-isobutoxy-12-methanesulfonyl-5,6,8aα,9,10,11,12,12aα,13,13aα-decahydro-8H-isoquino[2,1-g]-[1,6]naphthyridine hydrochloride;

(±)-3-n-hexyloxy-12-methanesulfonyl-5,6,8aα,9,10,11,12,12aα,13,13aα-decahydro-8H-isoquino[2,1-g]-[1,6]naphthyridine hydrochloride;

(±)-1,2-dimethoxy-12-methanesulfonyl-5,6,8aα,9,10,11,12,12aα,13,13aα-decahydro-8H-isoquino[2,1-g][1,6]naphthyridine hydrochloride;

(±)-2,3-diethoxy-12-methanesulfonyl-5,6,8aα,9,10,11,12,12aα,13,13aα-decahydro-8H-isoquino[2,1-g]-[1,6]naphthyridine hydrochloride;

(±)-2,3-di-n-butoxy-12-methanesulfonyl-5,6,8aα,9,10,11,12,12aα,13,13aα-decahydro-8H-isoquino[2,1-g][1,6]naphthyridine hydrochloride;

(±)-3,4-methylenedioxy-12-methanesulfonyl-5,6,8aα,9,10,11,12,12aα,13,13aα-decahydro-8H-isoquino-[2,1-g][1,6]naphthyridine hydrochloride;

(±)-2-chloro-12-methanesulfonyl-5,6,8aα,9,10,11,12,12aα,13,13aα-decahydro-8H-isoquino[2,1-g][1,6]-naphthyridine hydrochloride;

(±)-3-chloro-12-methanesulfonyl-5,6,8aα,9,10,11,12,12aα,13,13aα-decahydro-8H-isoquino[2,1-g][1,6]-naphthyridine hydrochloride;

(±)-3-fluoro-12-methanesulfonyl-5,6,8aα,9,10,11,12,12aα,13,13aα-decahydro-8H-isoquino[2,1-g][1,6]-naphthyridine hydrochloride;

(±)-12-aminosulfonyl-5,6,8aα,9,10,11,12,12aα,13,13aα-decahydro-8H-isoquino[2,1-g][1,6]naphthyridine hydrochloride;

(±)-12-methylaminosulfonyl-5,6,8aα,9,10,11,12,12aα,13,13aα-decahydro-8H-isoquino[2,1-g][1,6]-naphthyridine hydrochloride;

(±)-12-diethylaminosulfonyl-5,6,8aα,9,10,11,12,12aα,13,13aα-decahydro-8H-isoquino[2,1-g][1,6]-

naphthyridine hydrochloride;

(±)-12-di-n-hexylaminosulfonyl-5,6,8aα,9,10,11,12,12aα,13,13aα-decahydro-8H-isoquino[2,1-g][1,6]-naphthyridine hydrochloride;

(±)-12-(1-piperazinosulfonyl)-5,6,8aα,9,10,11,12,12aα,13,13aα-decahydro-8H-isoquino[2,1-g][1,6]-naphthyridine hydrochloride;

(±)-12-(1-morpholinosulfonyl)-5,6,8aα,9,10,11,12,12aα,13,13aα-decahydro-8H-isoquino[2,1-g][1,6]-naphthyridine hydrochloride;

(±)-12-(1-piperidinosulfonyl)-5,6,8aα,9,10,11,12,12aα,13,13aα-decahydro-8H-isoquino[2,1-g][1,6]-naphthyridine hydrochloride;

(±)-3-methyl-12-ethanesulfonyl-5,6,8aα,9,10,11,12,12aα,13,13aα-decahydro-8H-isoquino[2,1-g][1,6]-naphthyridine hydrochloride;

(±)-3-ethoxy-12-ethanesulfonyl-5,6,8aα,9,10,11,12,12aα,13,13aα-decahydro-8H-isoquino[2,1-g][1,6]-naphthyridine hydrochloride;

(±)-3-n-hexyloxy-12-ethanesulfonyl-5,6,8aα,9,10,11,12,12aα,13,13aα-decahydro-8H-isoquino[2,1-g]-[1,6]naphthyridine hydrochloride;

(±)-2,3-diethoxy-12-ethanesulfonyl-5,6,8aα,9,10,11,12,12aα,13,13aα-decahydro-8H-isoquino[2,1-g]-[1,6]naphthyridine hydrochloride;

(±)-3-chloro-12-ethanesulfonyl-5,6,8aα,9,10,11,12,12aα,13,13aα-decahydro-8H-isoquino[2,1-g][1,6]-naphthyridine hydrochloride;

(±)-3-methyl-12-(1-n-hexanesulfonyl)-5,6,8aα,9,10,11,12,12aα,13,13aα-decahydro-8H-isoquino[2,1-g]-[1,6]naphthyridine hydrochloride;

(±)-3-methoxy-12-(1-n-hexanesulfonyl)-5,6,8aα,9,10,11,12,12aα,13,13aα-decahydro-8H-isoquino[2,1-g][1,6]naphthyridine hydrochloride;

(±)-2,3-dimethoxy-12-(1-n-hexanesulfonyl)-5,6,8aα,9,10,11,-12,12aα,13,13aα-decahydro-8H-isoquino-[2,1-g][1,6]naphthyridine hydrochloride;

(±)-3-chloro-12-(1-n-hexanesulfonyl)-5,6,8aα,9,10,11,12,12aα,13,13aα-decahydro-8H-isoquino[2,1-g]-[1,6]naphthyridine hydrochloride;

(±)-3-methoxy-12-phenylsulfonyl-5,6,8aα,9,10,11,12,12aα,13,13aα-decahydro-8H-isoquino[2,1-g][1,6]-naphthyridine hydrochloride;

(±)-3-methyl-12-(N,N-dimethylaminosulfonyl)-5,6,8aα,9,10,11,12,12aα,13,13aα-decahydro-8H-isoquino[2,1-g][1,6]naphthyridine hydrochloride;

(±)-3-methoxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-2,3-dimethoxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-2,3-methylenedioxy-12-(N,N-dimethylaminosulfonyl)-5,6,8aα,9,10,11,12,12aα,13,13aα-decahydro-8H-isoquino[2,1-g][1,6]naphthyridine hydrochloride;

(±)-3-n-hexyloxy-12-ethanesulfonyl-5,6,8aα,9,10,11,12,12aα,13,13aα-decahydro-8H-isoquino[2,1-g]-[1,6]naphthyridine hydrochloride;

(±)-2,3-diethoxy-12-ethanesulfonyl-5,6,8aα,9,10,11,12,12aα,13,13aα-decahydro-8H-isoquino[2,1-g]-[1,6]naphthyridine hydrochloride; and

(±)-3-chloro-12-ethanesulfonyl-5,6,8aα,9,10,11,12,12aα,13,13aα-decahydro-8H-isoquino[2,1-g][1,6]-naphthyridine hydrochloride.

## EXAMPLE 1D

### Alternative Preparation of (8aR,12aS,13aS)-3-methoxy-12-Methanesulfonyl-5,6,8a,9,10,11,12,12a,13,13a-decahydro-8H-isoquino[2,1g][1,6]naphthyridine hydrochloride and Related Compounds of Formula (1)

A. A solution of 1.45 g of (8aR,12aS,13aR)-3-methoxy-12-methanesulfonyl-5,6,8a,9,10,11,12,12a,13,13a-decahydro-8H-isoquino[2,1-g][1,6]naphthyridine (a compound of formula (2)) was dissolved in 50 ml of chloroform, cooled to 0°C and 0.825 g of 80% m-chloroperbenzoic acid added. The solution was allowed to warm to room temperature, and a further 200 mg of m-chloroperbenzoic acid added. The mixture was then recooled to 0°C and 3.2 ml of trifluoroacetic anhydride added, and the solution stirred for 20 minutes. The solution was allowed to warm to room temperature, stirred for 20 minutes, then the solvent removed under reduced pressure at room temperature. The residue was dissolved in 50 ml of ethanol, cooled to 0°C and sodium borohydride slowly added until the solution was basic. Solvent was removed under reduced pressure and the residue partitioned between ethyl acetate and dilute hydrochloric acid. The aqueous solution was separated and basified with ammonium hydroxide, then extracted three times with methylene chloride. Solvent was removed from the combined extracts and the residue chromatographed on silica gel, eluting with ethyl acetate, giving 780 mg of (8aR,12aS,13aS)-3-methoxy-12-methanesulfonyl-5,6,8a,9,10,11,12,12a,13,13a-decahydro-8H-isoquino[2,1-g][1,6]naphthyridine, mp 165-166 °C, $[\alpha]_D^{25}$ = -55.5 ($CHCl_3$).

B. Alternatively, the conversion of a compound of formula (2) to a compound of formula (1) may be accomplished by the following procedure.

A mixture of 1.14 g of (8aR,12aS,13aR)-3-methoxy-12-methanesulfonyl-5,6,8a,9,10,11,12,12a,13,13a-decahydro-8H-isoquino[2,1-g][1,6]naphthyridine (a compound of formula (2)) and 4.53 g of mercuric acetate in 20 ml of acetic acid and 2 ml of water is stirred at 105 °C for 1 hour. The mixture is filtered and hydrogen sulfide is bubbled through the filtrate for 5 minutes. The mixture is filtered again and the filtrate is concentrated under reduced pressure. Ethanol (50 ml) is added and the resulting solution is cooled to -20 °C and treated with 0.5 g of sodium borohydride. The solution is allowed to warm to room temperature and acidified with aqueous HCl. After washing with ethyl acetate, the aqueous layer is basified with $NH_4OH$ and extracted with ethyl acetate. The ethyl acetate is washed with brine, dried over anhydrous sodium sulfate and evaporated. Chromatography of the residue on silica gel, eluting with 1% methanol in methylene chloride, affords (8aR,12aS,13aS)-3-methoxy-12-methanesulfonyl-5,6,8a,9,10,11,12,12a,13,13a-decahydro-8H-isoquino[2,1-g][1,6]naphthyridine.

C. Similarly, replacing (8aR,12aS,13aR)-3-methoxy-12-methanesulfonyl-5,6,8a,9,10,11,12,12a,13,13a-decahydro-8H-isoquino[2,1-g][1,6]naphthyridinewith other compounds of formula (2) and following the procedure of paragraph A or B above, the following compounds of formula (1) are prepared:

(8aR,12aS,13aS)-12-methanesulfonyl-5,6,8a,9,10,11,12,12a,13,13a-decahydro-8H-isoquino[2,1-g][1,6]-naphthyridine hydrochloride;

(8aR,12aS,13aS)-12-ethanesulfonyl-5,6,8a,9,10,11,12,12a,13,13a-decahydro-8H-isoquino[2,1-g][1,6]-naphthyridine hydrochloride;

(8aR,12aS,13aS)-12-(1-propanesulfonyl)-5,6,8a,9,10,11,12,12a,13,13a-decahydro-8H-isoquino[2,1-g]-[1,6]naphthyridine hydrochloride;

(8aR,12aS,13aS)-12-(1-butanesulfonyl)-5,6,8a,9,10,11,12,12a,13,13a-decahydro-8H-isoquino[2,1-g]-[1,6]naphthyridine hydrochloride;

(8aR,12aS,13aS)-12-(2-methylpropanesulfonyl)-5,6,8a,9,10,11,12,12a,13,13a-decahydro-8H-isoquino-[2,1-g][1,6]naphthyridine hydrochloride;

(8aR,12aS,13aS)-12-phenylsulfonyl-5,6,8a,9,10,11,12,12a,13,13a-decahydro-8H-isoquino[2,1-g][1,6]-naphthyridine hydrochloride;

(8aR,12aS,13aS)-12-(4-methoxyphenylsulfonyl)-5,6,8a,9,10,11,12,12a,13,13a-decahydro-8H-isoquino-[2,1-g][1,6]naphthyridine hydrochloride;

(8aR,12aS,13aS)-12-(4-chlorophenylsulfonyl)-5,6,8a,9,10,11,12,12a,13,13a-decahydro-8H-isoquino-[2,1-g][1,6]naphthyridine hydrochloride;

(8aR,12aS,13aS)-12-(4-fluorophenylsulfonyl)-5,6,8a,9,10,11,12,12a,13,13a-decahydro-8H-isoquino[2,1-g][1,6]naphthyridine hydrochloride;

(8aR,12aS,13aS)-12-(2-methoxyethanesulfonyl)-5,6,8a,9,10,11,12,12a,13,13a-decahydro-8H-isoquino-[2,1-g][1,6]naphthyridine hydrochloride;

(8aR,12aS,13aS)-3-methoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(1-propanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-piperazinosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-morpholinosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-piperidinosulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-1,4-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-dimethoxy-12-(2-methylpropanesulfonyl)-5,6,8a,9,10,11,12,12a,13,13a-decahydro-8H-isoquino[2,1-g][1,6]naphthyridinehydrochloride;

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-(ethylene-1,2-dioxy)-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-methylenedioxy-12-(2-methylpropanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(2-methoxyethanesulfonyl)-5,6,8a,9,10,11,12,12a,13,13a-decahydro-8H-isoquino[2,1-g][1,6]naphthyridine hydrochloride;

(8aR,12aS,13aS)-12-(4-aminophenylsulfonyl)-5,6,8a,9,10,11,12,12a,13,13a-decahydro-8H-isoquino-[2,1-g][1,6]naphthyridine hydrochloride;

(8aR,12aS,13aS)-12-(2-hydroxyethanesulfonyl)-5,6,8a,9,10,11,12,12a,13,13a-decahydro-8H-isoquino-[2,1-g][1,6]naphthyridine hydrochloride;

(8aR,12aS,13aS)-1-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-methyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-ethyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2,3-dimethyl-12-methanesulfonyl-5,6,8a,9,10,11,12,12a,13,13a-decahydro-8H-isoquino[2,1-g][1,6]naphthyridine hydrochloride;

(8aR,12aS,13aS)-3-isobutyl-12-methanesulfonyl-5,6,8a,9,10,11,12,12a,13,13a-decahydro-8H-isoquino-[2,1-g][1,6]naphthyridine hydrochloride;

(8aR,12aS,13aS)-2-n-hexyl-12-methanesulfonyl-5,6,8a,9,10,11,12,12a,13,13a-decahydro-8H-isoquino-[2,1-g][1,6]naphthyridine hydrochloride;

(8aR,12aS,13aS)-3-methoxy-2-methyl-12-methanesulfonyl-5,6,8a,9,10,11,12,12a,13,13a-decahydro-8H-isoquino[2,1-g][1,6]naphthyridine hydrochloride;

(8aR,12aS,13aS)-1-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-4-methoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-ethoxy-12-methanesulfonyl-5,6,8a,9,10,11,12,12a,13,13a-decahydro-8H-isoquino-[2,1-g][1,6]naphthyridine hydrochloride;

(8aR,12aS,13aS)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-isopropoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-isobutoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-n-hexyloxy-12-methanesulfonyl-5,6,8a,9,10,11,12,12a,13,13a-decahydro-8H-isoquino[2,1-g][1,6]naphthyridine hydrochloride;

(8aR,12aS,13aS)-3-hydroxy-12-methanesulfonyl-5,6,8a,9,10,11,12,12a,13,13a-decahydro-8H-isoquino-[2,1-g][1,6]naphthyridine hydrochloride;

(8aR,12aS,13aS)-2,3-dihydroxy-12-methanesulfonyl-5,6,8a,9,10,11,12,12a,13,13a-decahydro-8H-isoquino[2,1-g][1,6]naphthyridine hydrochloride;

(8aR,12aS,13aS)-1,2-dimethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-diethoxy-12-methanesulfonyl-5,6,8a,9,10,11,12,12a,13,13a-decahydro-8H-isoquino[2,1-g][1,6]naphthyridine hydrochloride;

(8aR,12aS,13aS)-2,3-di-n-butoxy-12-methanesulfonyl-5,6,8a,9,10,11,12,12a,13,13a-decahydro-8H-isoquino[2,1-g][1,6]naphthyridine hydrochloride;

(8aR,12aS,13aS)-3,4-methylenedioxy-12-methanesulfonyl-5,6,8a,9,10,11,12,12a,13,13a-decahydro-8H-isoquino[2,1-g][1,6]naphthyridine hydrochloride;

(8aR,12aS,13aS)-2-chloro-12-methanesulfonyl-5,6,8a,9,10,11,12,12a,13,13a-decahydro-8H-isoquino-[2,1-g][1,6]naphthyridine hydrochloride;

(8aR,12aS,13aS)-3-chloro-12-methanesulfonyl-5,6,8a,9,10,11,12,12a,13,13a-decahydro-8H-isoquino-[2,1-g][1,6]naphthyridine hydrochloride;

(8aR,12aS,13aS)-2-fluoro-12-methanesulfonyl-5,6,8a,9,10,11,12,12a,13,13a-decahydro-8H-isoquino-[2,1-g][1,6]naphthyridine hydrochloride;

(8aR,12aS,13aS)-3-fluoro-12-methanesulfonyl-5,6,8a,9,10,11,12,12a,13,13a-decahydro-8H-isoquino-[2,1-g][1,6]naphthyridine hydrochloride;

(8aR,12aS,13aS)-12-aminosulfonyl-5,6,8a,9,10,11,12,12a,13,13a-decahydro-8H-isoquino[2,1-g][1,6]-naphthyridine hydrochloride;

(8aR,12aS,13aS)-3-methoxy-12-aminosulfonyl-5,6,8a,9,10,11,12,12a,13,13a-decahydro-8H-isoquino-[2,1-g][1,6]naphthyridine hydrochloride;

(8aR,12aS,13aS)-12-methylaminosulfonyl-5,6,8a,9,10,11,12,12a,13,13a-decahydro-8H-isoquino[2,1-g]-[1,6]naphthyridine hydrochloride;

(8aR,12aS,13aS)-12-diethylaminosulfonyl-5,6,8a,9,10,11,12,12a,13,13a-decahydro-8H-isoquino[2,1-g]-[1,6]naphthyridine hydrochloride;

(8aR,12aS,13aS)-12-di-ni-hexylaminosulfonyl-5,6,8a,9,10,11,12,12a,13,13a-decahydro-8H-isoquino-[2,1-g][1,6]naphthyridine hydrochloride;

(8aR,12aS,13aS)-12-(1-piperazinosulfonyl)-5,6,8a,9,10,11,12,12a,13,13a-decahydro-8H-isoquino[2,1-g][1,6]naphthyridine hydrochloride;

(8aR,12aS,13aS)-12-(1-morpholinosulfonyl)-5,6,8a,9,10,11,12,12a,13,13a-decahydro-8H-isoquino[2,1-g][1,6]naphthyridine hydrochloride;

(8aR,12aS,13aS)-12-(1-piperidinosulfonyl)-5,6,8a,9,10,11,12,12a,13,13a-decahydro-8H-isoquino[2,1-g][1,6]naphthyridine hydrochloride;

(8aR,12aS,13aS)-3-methyl-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-ethoxy-12-ethanesulfonyl-5,6,8a,9,10,11,12,12a,13,13a-decahydro-8H-isoquino-[2,1-g][1,6]naphthyridine hydrochloride;

(8aR,12aS,13aS)-3-n-hexyloxy-12-ethanesulfonyl-5,6,8a,9,10,11,12,12a,13,13a-decahydro-8H-isoquino[2,1-g][1,6]naphthyridine hydrochloride;

(8aR,12aS,13aS)-2,3-diethoxy-12-ethanesulfonyl-5,6,8a,9,10,11,12,12a,13,13a-decahydro-8H-isoquino[2,1-g][1,6]naphthyridine hydrochloride;

(8aR,12aS,13aS)-3-methyl-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-(1-n-hexanesulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-phenylsulfonyl-5,6,8a,9,10,11,12,12a,13,13a-decahydro-8H-isoquino-[2,1-g][1,6]naphthyridine hydrochloride;

(8aR,12aS,13aS)-3-methoxy-12-(N,N-dimethylaminosulfonyl)-5,6,8a,9,10,11,12,12a,13,13a-decahydro-8H-isoquino[2,1-g][1,6]naphthyridine hydrochloride;

(8aR,12aS,13aS)-3-methoxy-12-(t-butylaminosulfonyl)-5,6,8a,9,10,11,12,12a,13,13a-decahydro-8H-isoquino[2,1-g][1,6]naphthyridine hydrochloride;

(8aR,12aS,13aS)-2,3-dimethoxy-12-(N,N-dimethylaminosulfonyl)-5,6,8a,9,10,11,12,12a,13,13a-decahydro-8H-isoquino[2,1,-g][1,6]naphthyridine hydrochloride;

(8aR,12aS,13aS)-2,3-methylenedioxy-12-(N,N-dimethylaminosulfonyl)-5,6,8a,9,10,11,12,12a,13,13a-decahydro-8H-isoquino[2,1-g][1,6]naphthyridine hydrochloride; and

(8aR,12aS,13aS)-2-methyl-12-(2-methoxymethanesulfonyl)-5,6,8a,9,10,11,12,12a,13,13a-decahydro-8H-isoquino[2,1-g][1,6]naphthyridine hydrochloride.

EXAMPLE 2

Preparation of (±)-12-(2-Hydroxyethanesulfonyl-5,6,8aα,9,10,11,12,12aα,13,13aα-decahydro-8H-isoquino[2,1-g][1,6]naphthyridine hydrochloride

A. A solution of 0.62 g of (±)-12-(2-methoxyethanesulfonyl)-5,6,8aα,9,10,11,12,12aα,13,13aα-decahydro-8H-isoquino[2,1-g][1,6]naphthyridine in 25 ml of methylene chloride was cooled to -60 °C and treated with 2 ml of 1M $BBr_3$ in methylene chloride. The mixture was allowed to warm to room temperature and 50 ml of water was then added. The mixture was basified with $NH_4OH$ and extracted several times with methylene chloride. The methylene chloride extracts were evaporated and the residue was purified by silica gel chromatography to give the free base, which was treated with ethanolic HCl-ether to give (±)-12-(2-hydroxyethanesulfonyl)-5,6,8aα,9,10,11,12,12aα,13,13aα-decahydro-8H-isoquino[2,1-g][1,6]-naphthyridine hydrochloride, (0.45 g), m.p. 215-216 °C.

B. Similarly, replacing (±)-12-(2-methoxyethanesulfonyl)-5,6,8aα,9,10,11,12,12aα,13,13aα-decahydro-8H-isoquino[2,1-g][1,6]naphthyridine with the appropriate alkoxy-substituted compounds of formula (1) or (2) and following the procedure in paragraph A above, the following compounds of formula (1) and (2) are prepared:

(±)-12-(2-hydroxyethanesulfonyl)-5,6,8aβ,9,10,11,12,12aβ,13,13aα-decahydro-8H-isoquino[2,1-g][1,6]-naphthyridine hydrochloride

(±)-12-(2-hydroxypropanesulfonyl)-5,6,8aα,9,10,11,12,12aα,13,13aα-decahydro-8H-isoquino[2,1-g]-[1,6]naphthyridine hydrochloride;

(±)-12-(2-hydroxyhexanesulfonyl)-5,6,8aα,9,10,11,12,12aα,13,13aα-decahydro-8H-isoquino[2,1-g][1,6]-naphthyridine hydrochloride; and

(±)-2-methyl-12-(2-hydroxymethanesulfonyl)-5,6,8aα,9,10,11,12,12aα,13,13aα-decahydro-8H-isoquino-[2,1-g][1,6]naphthyridine hydrochloride.

EXAMPLE 3

Preparation of (±)-12-(4-Aminophenylsulfonyl)-5,6,8aα,9,10,11,12,12aα,13,13aα-decahydro-8H-isoguino[2,1-g][1,6]naphthyridine hydrochloride

A. A solution of 0.44 g of (±)-5,6,8aα,9,10,11,12,12aα,13,13aα-decahydro-8H-isoquino[2,1-g][1,6]-naphthyridine (VII) in 10 ml of methylene chloride and 1 ml of triethylamine was treated with 0.4 g of 4-nitrobenzenesulfonyl chloride and the resulting mixture was stirred at room temperature for 1 hour. The mixture was diluted with methylene chloride, washed with aqueous $NH_4OH$, and evaporated. The residue was dissolved in 25 ml of ethanol and hydrogenated at 50 psi (3.45 bar) with 0.2 g of 10% Pd-C for 6 hours. The catalyst was removed by filtration and the filtrate was concentrated under reduced pressure to a residue which was dissolved in ethanolic HCl. Addition of ether gave a precipitate which was filtered off and dried under vacuum to give (±)-12-(4-aminophenylsulfonyl)-5,6,8aα,9,10,11,12,12aα,13,13aα-decahydro-8H-isoquino[2,1-g][1,6]naphthyridine dihydrochloride: yield 0.27 g,
m.p. 245-247 °C.

B. Similarly, replacing (±)-5,6,8aα,9,10,11,12,12aα,13,13aα-decahydro-8H-isoquino[2,1-g][1,6]-naphthyridine with the appropriate compounds of formula (VII) or (VIII) and following the procedure in paragraph A above, the following compounds of formula (1) and (2) are prepared:

(±)-12-(4-aminophenylsulfonyl)-5,6,8aβ,9,10,11,12,12aβ,13,13aα-decahydro-8H-isoquino[2,1-g][1,6]-naphthyridine hydrochloride;

(±)-3-methoxy-12-(4-aminophenylsulfonyl)-5,6,8aα,9,10,11,12,12aα,13,13aα-decahydro-8H-isoquino-[2,1-g][1,6]naphthyridine hydrochloride;

(±)-3-hydroxy-12-(4-aminophenylsulfonyl)-5,6,8aα,9,10,11,12,12aα,13,13aα-decahydro-8H-isoquino-[2,1-g][1,6]naphthyridine hydrochloride;

(±)-2,3-dimethoxy-12-(4-aminophenylsulfonyl)-5,6,8aα,9,10,11,12,12aα,13,13aα-decahydro-8H-isoquino[2,1-g][1,6]naphthyridine hydrochloride;

(±)-2,3-methylenedioxy-12-(4-aminophenylsulfonyl)-5,6,8aα,9,10,11,12,12aα,13,13aα-decahydro-8H-isoquino[2,1-g][1,6]naphthyridine hydrochloride;

EXAMPLE 4A

Conversion of (±)-12-methanesulfonyl-5,6,8aα,9,10,11,12,12aα,13,13aα-decahydro-8H-isoquino[2,1-g][1,6]-naphthyridine to its hydrochloride

Excess 3% hydrogen chloride in methanol is added to a solution of (±)-12-methanesulfonyl-5,6,8aα,9,10,11,12,12aα,13,13aα-decahydro-8H-isoquino[2,1-g][1,6]naphthyridine in 20 ml methanol. Diethyl ether is added until precipitation is complete. The product is filtered, washed with ether, air dried and recrystallized from methanol/acetone to yield (±)-12-methanesulfonyl-5,6,8aα,9,10,11,12,12aα,13,13aα-decahydro-8H-isoquino[2,1-g][1,6]naphthyridine hydrochloride.

In a similar manner, all compounds of formula (1) and (2) in free base form may be converted to the acid addition salts by treatment with the appropriate acid, for example, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

EXAMPLE 4B

Conversion of (8aR,12aS,13aS)-3-methoxy-12-methanesulfonyl-5,6,8a,9,10,11,12,12a,13,13a-decahydro-8H-isoquino[2,1g][1,6]naphthyridine to its hydrochloride

Excess 3% hydrogen chloride in methanol is added to a solution of (8aR,12aS,13aS)-3-methoxy-12-methanesulfonyl-5,6,8a,9,10,11,12,12a,13,13a-decahydro-8H-isoquino[2,1-g][1,6]naphthyridine in 20 ml methanol. Diethyl ether is added until precipitation is complete. The product is filtered, washed with ether, air dried and recrystallized from methanol/acetone to yield (8aR,12aS,13aS)-3-methoxy-12-methanesulfonyl-5,6,8a,9,10,11,12,12a,13,13a-decahydro-8H-isoquino[2,1-g][1,6]-naphthyridine hydrochloride, mp 256-258 °C, $[\alpha]_D^{25}$ = +13.1 ($CHCl_3$).

In a similar manner, all compounds of formula (1) including (8aR,12aS,13aS)-3-methoxy-12-methanesulfonyl-5,6,8a,9,10,11,12,12a,13,13a-decahydro-8H-isoquino-[2,1-g][1,6]naphthyridine and (8aR,12aS,13aS)-2,3-methylenedioxy-12-methanesulfonyl-5,6,8a,9,10,11,12,12a, 13,13a-decahydro-8H-isoquino-[2,1-g][1,6]naphthyridine in free base form may be converted to the acid addition salts by treatment with the appropriate acid, for example, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, and the like.

For example:

(8aR,12aS,13aS)-3-methoxy-12-methanesulfonyl-5,6,8a,9,10,-11,12,12a,13,13a-decahydro-8H-isoquino-[2,1-g][1,6]naphthyridine hydrobromide;

(8aR,12aS,13aS)-3-methoxy-12-methanesulfonyl-5,6,8a,9,10,-11,12,12a,13,13a-decahydro-8H-isoquino-[2,1-g][1,6]naphthyridinium sulfate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium nitrate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium phosphate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium acetate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium propionate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium glycolate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium pyruvate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium oxalate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium malate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium malonate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium succinate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium maleate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium fumarate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium tartrate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium citrate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium benzoate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium cinnamate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium mandelate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium methanesulfonate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium ethanesulfonate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium p-toluenesulfonate;

(8aR,12aS,13aS)-3-methoxy-12-methanesulfonyl-5,6,8a,9,10,-11,12,12a,13,13a-decahydro-8H-isoquino-[2,1-g][1,6]naphthyridinium salicylate;

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrochloride,
mp 263-265 °C, $[\alpha]_D^{25}$ = -17.0 ($CH_3OH$)

(8aR,12aS,13aS)-2,3-methylenedioxy-12-methanesulfonyl-5,6,8a,9,10,11,12,12a,13,13a-decahydro-8H-isoquino[2,1-g][1,6]naphthyridine hydrobromide;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium sulfate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium nitrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium phosphate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium acetate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium propionate;

(8aR,12aS,13aS)-2,3-methylenedioxy-12-methanesulfonyl-5,6,8a,9,10,11,12,12a,13,13a-decahydro-8H-isoquino[2,1-g][1,6]-naphthyridinium glycolate;

(8aR,12aS,13aS)-2,3-methylenedioxy-12-methanesulfonyl-5,6,8a,9,10,11,12,12a,13,13a-decahydro-8H-isoquino[2,1-g][1,6]-naphthyridinium pyruvate;

(8aR,12aS,13aS)-2,3-methylenedioxy-12-methanesulfonyl-5,6,8a,9,10,11,12,12a,13,13a-decahydro-8H-isoquino[2,1-g][1,6]-naphthyridinium oxalate;

(8aR,12aS,13aS)-2,3-methylenedioxy-12-methanesulfonyl-5,6,8a,9,10,11,12,12a,13,13a-decahydro-8H-isoquino[2,1-g][1,6]-naphthyridinium malate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium malonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium succinate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium maleate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium fumarate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium tartrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium citrate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium benzoate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium cinnamate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium mandelate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium methanesulfonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium ethanesulfonate;

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium p-toluenesulfonate; and

(8aR,12aS,13aS)-2,3-methylenedioxy-12-methanesulfonyl-5,6,8a,9,10,-11,12,12a,13,13a-decahydro-8H-isoquino[2,1-g][1,6]-naphthyridinium salicylate.

EXAMPLE 5A

Conversion of a salt of (±)-12-methanesulfonyl-5,6,8aα,9,10,11,12,12aα,13,13aα-decahydro-8H-isoquino[2,1-g][1,6]naphthyridine to free base.

(±)-12-methanesulfonyl-5,6,8aα,9,10,11,12,12aα,13,13aα-decahydro-8H-isoquino[2,1-g][1,6]naphthyridine hydrochloride suspended in 50 ml of ether is stirred with excess dilute aqueous potassium carbonate solution until the salt is completely dissolved. The organic layer is then separated, washed twice with water, dried over magnesium sulfate and evaporated to yield (±)-12-methanesulfonyl-5,6,8aα,9,10,11,12,12aα,13,13aα-decahydro-8H-isoquino[2,1-g][1,6]naphthyridine, m.p. 176-177 °C.

In a similar manner the acid addition salts of all compounds of formula (1) and (2) may be converted to the corresponding compounds in free base form.

EXAMPLE 5B

Conversion of a salt of (8aR,12aS,13aS)-3-methoxy-12-methanesulfonyl-5,6,8a,9,10,11,12,12a,13,13a-decahydro-8H-isoquino[2,1-g][1,6]naphthyridine to free base.

(8aR,12aS,13aS)-3-methoxy-12-methanesulfonyl-5,6,8a,9,10,11,12,12a,13,13a-decahydro-8H-isoquino-[2,1-g][1,6]naphthyridine hydrochloride suspended in 50 ml of ether is stirred with excess dilute aqueous potassium carbonate solution until the salt is completely dissolved. The organic layer is then separated, washed twice with water, dried over magnesium sulfate and evaporated to yield (8aR,12aS,13aS)-3-methoxy-12-methanesulfonyl-5,6,8a,9,10,11,12,12a,13,13a-decahydro-8H-isoquino[2,1-g][1,6]naphthyridine, m.p. 165-166 °C.

In a similar manner the acid addition salts of all compounds of formula (1) may be converted to the corresponding compounds in free base form.

EXAMPLE 6A

Direct interchange of acid addition salts of (±)-12-methanesulfonyl-5,6,8aα,9,10,11,12,12aα,13,13aα-decahydro-8H-isoquino[2,1-g][1,6]naphthyridine

(±)-12-Methanesulfonyl-5,6,8aα,9,10,11,12,12aα,13,13aα-decahydro-8H-isoquino[2,1-g][1,6]naphthyridine acetate (1.0 g) is dissolved in 50 ml 5N aqueous hydrochloric acid, and the solution evaporated to dryness. The product is suspended in ethyl acetate and filtered, air dried and recrystallized from methanol/acetone to yield (±)-12-methanesulfonyl-5,6,8aα,9,10,11,12,12aα,-13,13aα-decahydro-8H-isoquino[2,1-g][1,6]-naphthyridine hydrochloride.

In a similar manner, substituting for hydrochloric acid other acids, such as sulfuric acid, nitric acid, phosphoric acid and the like, other acid addition salts of all compounds of formula (1) and (2) are prepared.

EXAMPLE 6B

Direct interchange of acid addition salts of (8aR,12aS,13aS)-3-methoxy-12-methanesulfonyl-5,6,8a,9,10,11,12,12a,13,13a-decahydro-8H-isoquino[2,1-g][1,6]naphthyridine

(8aR,12aS,13aS)-3-methoxy-12-methanesulfonyl-5,6,8a,9,10,11,12,12a,13,13a-decahydro-8H-isoquino-[2,1-g][1,6]naphthyridine acetate (1.0 g) is dissolved in 50 ml 5N aqueous hydrochloric acid, and the solution evaporated to dryness. The product is suspended in ethyl acetate and filtered, air dried and recrystallized from methanol/acetone to yield (8aR,12aS,13aS)-3-methoxy-12-methanesulfonyl-5,6,8a,9,10,11,12,12a,13,13a-decahydro-8H-isoquino[2,1-g][1,6]naphthyridine hydrochloride.

In a similar manner, substituting for hydrochloric acid other acids, such as sulfuric acid, nitric acid, phosphoric acid and the like, other acid addition salts of all compounds of formula (1) are prepared.

In Examples 7 through 12 the active ingredient is (8aR,12aS,13aS)-3-methoxy-12-methanesulfonyl-5,6,8a,9,10,11,12,12a,13,13a-decahydro-8H-isoquino[2,1-g][1,6]naphthyridine hydrochloride. Other compounds of formula (I), (1) or (2) or the pharmaceutically acceptable salts thereof may be substituted therein.

EXAMPLE 7

Composition for Oral Administration

| The composition contains: | % wt./wt. |
|---|---|
| Active ingredient | 20% |
| Lactose | 80% |

The two ingredients are milled, mixed and dispensed into capsules containing 100 mg each; one capsule would approximate a total daily dosage.

EXAMPLE 8

Composition for Oral Administration

| The composition contains: | % wt./wt. |
|---|---|
| Active ingredient | 20.0% |
| Magnesium stearate | 0.9% |
| Starch | 8.6% |
| Lactose | 79.6% |
| PVP (polyvinylpyrrolidine) | 0.9% |

The above ingredients are combined and granulated using methanol as solvent. The formulation is then dried and formed into tablets (containing 20 mg of active compound) with an appropriate tableting machine.

EXAMPLE 9

Parenteral Formulation (IV)

| The composition contains: | % wt./wt. |
|---|---|
| Active ingredient | 0.25 g |
| Propylene glycol | 20. g |
| Polyethylene glycol 400 | 20. g |
| Polysorbate 80 | 1. g |
| 0.9% Saline solution qs ad | 100 ml |

The active ingredient is dissolved in propylene glycol, polyethylene glycol 400 and polysorbate 80. A sufficient quantity of 0.9% saline solution is then added with stirring to provide 100 ml. of the I.V. solution which is filtered through a 0.2 micron membrane filter and packaged under sterile conditions.

EXAMPLE 10

Suppository Formulation

| The composition contains: | % wt./wt. |
|---|---|
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

EXAMPLE 11

Composition for Topical Administration to the Eye

| The composition contains: | % wt/vol |
|---|---|
| Active ingredient | 0.10 |
| Benzalkonium chloride | 0.02 |
| EDTA | 0.01 |
| Phenylethanol | 0.25 |
| Boric acid | 1.62 |
| | to adjust pH |
| water qs and | 100 ml |

The first four ingredients are dissolved in less than the required total volume of water, and the pH adjusted to 7.4. The volume is then brought to 100 ml with additional water.

EXAMPLE 12

Topical Formulation

| Ingredients | | grams |
|---|---|---|
| Active compound | | 0.2-2 |
| Span 60 | | 2 |
| Tween 60 | | 2 |
| Mineral oil | | 5 |
| Petrolatum | | 10 |
| Methyl paraben | | 0.15 |
| Propyl paraben | | 0.05 |
| BHA (butylated hydroxy anisole) | | 0.01 |
| Water | q.s. | 100 |

All of the above ingredients, except water, are combined and heated to 60 °C with stirring. A sufficient quantity of water at 60 °C is then added with vigorous stirring to emulsify the ingredients, and water then added q.s. 100 g.

EXAMPLE 13

Assay for pre- and post-synaptic α-adrenoceptor blockade

Protocol:

(According to Caroon, J.M. et al., J. Med. Chem., 1982, Vol. 25, 666.)

Contralateral, prostatic and epididymal portions of the rat isolated vas deferens were suspended in separate organ baths containing oxygenated Krebs - bicarbonate solution at 37 °C. The test compound was added to the Krebs - bicarbonate solution bathing the epididymal and prostatic portions of vas deferens. The contralateral portions served as control tissues. All tissues were then allowed to equilibrate with the bathing solution for 30 minutes.

Pre-synaptic α-adrenoceptor blockade was determined using the prostatic portions of vas deferens. Following the equilibration period, dose-response curves for the inhibitory effect of xylazine on the contractile response of the vas deferens to single pulse nerve stimulation were obtained.

Post-synaptic α-adrenoceptor blockade was determined using the epididymal portions of rat vas deferens. Following the equilibration period, dose-response curves for the contractile effects of phenylephrine on the vas deferens were obtained.

EXAMPLE 14

Determination of Platelet Aggregation Inhibition

Protocol:

Blood platelets are collected in the standard manner, and incubated in an Aggregation Module Incubator-Cuvette in the presence of either the inhibitor to be tested, or without said inhibitor as a control. The aggregation of the platelets is observed after the addition of an inducer, and the samples are evaluated for the presence of a lag period and the slope of the aggregation curve, as well as the maximum height of the aggregation curve in comparison to the control. $IC_{50}$ values i.e. the concentration of inhibitor required for 50% inhibition can be calculated from the inflection point on the appropriate dose response curve.

EXAMPLE 15

Determination of Effect on Intraoccular Pressure

Protocol:

The compound to be tested is dissolved in saline, and applied topically to the eye. The intraoccular pressure is measured immediately before application, and at specified time intervals thereafter, by means of a probe which measures the force necessary to flatten a small area of corneal surface, according to the method described by Moses, R. A., Tr. Am. Acad. Opth. and Otol., Jan-Feb 1962: 88-95.

In Examples 16 through 19 Compound 1 is 3-methoxy-12-methanesulfonyl-5,6,8a,9,10,11,12,12a,13,13a-decahydro-8H-isoquino[2,1-g][1,6]naphthyridine hydrochloride.

EXAMPLE 16

Determination of Effect on Rat Sexual Behavior

"Sexual Behaviour in Developing Male Rats", P. Sodersten, D. A Dammassa and E. R. Smith, Hormones and Behaviour, Vol. 8, pp 320-334 (1977).

Protocol:

Sexually naive, male rats, weighing 200 to 250 gms were housed 2 per cage in a normal light cycle room (lights on 5:00 a.m., lights off 7:00 p.m.).

The animals were weight grouped after a 10 day acclimatization period and tested on either the 12th or 13th day. Compound was administered 30 minutes before testing for sexual activity.

Tests for sexual behaviour were done in hemi-hexagonal observation cages with plexiglass fronts.

Stimulus female Sprague-Dawley rats, housed in a reverse light cycle room (lights off 10:00 a.m., lights on 8:00 p.m.), were brought into sexual receptivity by injection with 20 ug Estradiol Benzoate in 0.1 ml sesame seed oil 48 hours prior to the test, and with 1 mg Progesterone in 0.1 ml sesame oil 4-6 hrs prior to testing.

Each treated female rat was placed in an observation cage and allowed to acclimatize for ten minutes. A stimulus female was introduced into the arena and the behaviour pattern of the male was recorded on an Esterline Angus event recorder. The behaviours recorded were Mounts, Intromissions and Ejaculations. Intromission Latency (time from the start of the test to the first intromission), Ejaculation Latency (time from the first intromission to ejaculation), and Post Ejaculatory Interval (PEI, time from ejaculation to the first following intromission) were also recorded. The time from ejaculation to the first sign of active interest displayed by the male towards the female, to the first post ejaculatory mount and the number of post ejaculatory mounts were also recorded. Tests were terminated if the Intromission Latency was longer than 15 minutes, the Ejaculation Latency longer than 30 minutes or the Post Ejaculatory Interval was in excess of 15 minutes.

Results:

Behaviour scores were assigned to each animal as follows:
0 = no sexual activity, 1 = mounting behaviour only, 2 = mounting and intromitting activity and 3 = mounting, intromitting and ejaculatory behaviour observed. These data are presented in Table 1. A single oral dose of 0.4 mg/kg of Compound 1, administered 30 minutes prior to testing for sexual activity, significantly increased the mean behaviour score ($p<.05$) of naive male rats compared to the vehicle group tested at the same time period.

Conclusion:

In summary, 0.4mg/kg of Compound 1, administered 30 minutes before testing, significantly enhanced sexual activity in the naive male rat. This enhanced activity was measured by an increase in the behaviour score.

TABLE 1

Rat Sex Behavior Study

| Treatment | N | Rats Mounting (%) | Rats Intromitting (%) | Rats Ejaculating (%) | Mean Behavior Score | sem | SL* |
|---|---|---|---|---|---|---|---|
| Vehicle | 18 | 9 (50%) | 8 (44%) | 4 (22%) | 1.16 | 0.31 | |
| Cmpd 1 | 18 | 15 (83%) | 15 (83%) | 9 (50%) | 2.17 | 0.26 | $p < .05$ |

* Significance levels are compared to the Vehicle group using Fishings LSD multiple comparison test following a ranked one-way ANOVA (Kruskal-Wallace).

EXAMPLE 17

Toxicology

| | |
|---|---|
| SPECIES: | Charles River CD Sprague Dawley Rats. |
| COMPOUND: | Compound 1 |
| DOSE NUMBERS: | 50 and 100 mg/kg/day, 10 males/group. |
| DURATION OF DOSING: | Two weeks. |
| CONCLUSION: | No serious toxicological effects were observed. |

EXAMPLE 18

Weight-loss Stimulation Assay

| | |
|---|---|
| SPECIES: | Charles River CD Sprague Dawley Rats. |
| COMPOUND: | Compound 1 |
| DOSE AND NUMBERS: | 50 and 100 mg/kg/day, 10 males/group. |
| DURATION OF DOSING: | Two weeks. |
| CONCLUSION: | 50 and 100 mg/kg/day dosed rats gained 14% less bodyweight then controls. |

EXAMPLE 19

Irritable-Bowel Syndrome Assay

Protocol:

The test used is a modification of the method of Macht and Barba-Gose (Macht, D.T. and Barba-Gose, J. (1931): J. Amer. Pharm. Ass. 20, 558), which traces the transit of a charcoal meal through the intestine as an index of transit time. In the present model, intestinal transit in conscious mice (15-20g) was accelerated with an oral dose of barium chloride (300 mg/kg) administered at the same time as the charcoal meal. The

animals were sacrificed 10 min. later and the distance travelled by the charcoal measured.

Antagonist compound (Compound 1) was given as a 15 min. oral pretreatment and its effects on barium-stimulated intestinal transit of the charcoal meal was calculated.

EFFECTS OF COMPOUND 1 ON $Ba^{2+}$-STIMULATED INTESTINAL TRANSIT IN CONSCIOUS MICE (n=6-9/group)

| COMPOUND | DOSE (mg/kg p.o.) 15 min | % INHIBITION OF $Ba^{2+}$-EFFECT |
|---|---|---|
| Compound 1 | 10 | -26%* |

* p 0.05 significantly different from $Ba^{2+}$ control

EXAMPLE 20

Antidepressant Assay

Protocol:

Antidepressant utility was assessed by the ability of Compound 1 to down-regulate β-adrenoreceptors in rat cerebral cortex after chronic dosing; down-regulation of β-adrenoreceptors may be an index of antidepressant effectiveness (Clark, Michel and Whiting, 1986, Progress in Medicinal Chemistry, Vol. 23, 1 - 39). The number of β-adrenoreceptors was measured using an adaptation of the method of Bylund and Snyder (1976, Molecular Pharmacology, 12, 568) and expressed as $B_{max}$ (fmol/mg protein). Chronic dosing with Compound 1 reduced the number of β-adrenoreceptors in rat cerebral cortex without changing the affinity of the receptors (Kd).

Male Sprague-Dawley rats were dosed with 0.5 mg/kg of Compound 1 o.d. p.o. for 14 days. The animals were sacrificed 24 hours after the last dose.

Tris washed cortical membranes were prepared from the rat brains and incubated with [3H]-dihydroalprenolol (0.1-4.0 nM) for 30 mins. at 25 °C. The incubation was terminated by rapid filtration over Whatman GF/B filters in a Brandel Cell Harvester. Bound radioactivity was defined as the amount of ligand bound in the presence of 0.5 mM isoprenaline.

14 Day Down-regulation Study; Once Daily Oral Dosing

| Group | n | Kd (nM) | Bmax (fmoles/mg) |
|---|---|---|---|
| Control | 9 | 0.41 ± 0.03 | 70.69 ± 2.82 |
| Compound 1 | 10 | 0.34 ± 0.03 | 51.17 ± 4.44* |

* $p < 0.002$

EXAMPLE 21

Hypoglycaemic Assay

Compound 1 was administered to groups of 10 male mice (30 mg/kg, intraperitoneally) and found to reduce blood glucose from 220 ± 10 mg/deciL to 150 ± 41 mg/deciL; these hypoglycaemic effects indicate potential utility as an antidiabetic agent.

EXAMPLE 22

Antihypertensive Assay

Compound 1 has antihypertensive activity in that the compound lowered blood pressure in conscious spontaneously hypertensive rats when administered intravenously in a dose of 100 μ/kg. Mean blood pressure, monitored by an indwelling catheter in the tail artery, fell by 72 mm Hg (average fall in 3 experiments).

EXAMPLE 23

Anxiolytic Assay

Protocol:

The method used was that described by Crawley and Goodwin ("Preliminary behaviour model for the anxiolytic effects of benzodiazepines." Pharmac. Biochem. Behaviour 1980;13:167-170.). This method involves placing naive mice in a novel test environment which comprises a box divided by a partition into a dark area and a light area. Mice are allowed to shuttle between the dark and light area for a period of 10 mins. During this time the number of shuttles, total locomotor activity and total time spent in the dark were monitored. Compound 1 (0.3 mg/kg) was examined 30 min. after ip administration. The prescribed test parameters: # of shuttles, time dark area, and total locomotor activity were compared to control data using a standard Student's t-test and the percent of control response were calculated.

Results:

In the test apparatus, Crawley and Goodwin (1980) demonstrated that anxiolytic compounds decrease time spent in the dark area, increase the total amount of shuttle activity, and either not effect or increase total locomotor activity. Present data was evaluated against that profile.

Compound 1 displayed a profile which is generally associated with an anxiolytic agent within this model. The compound produced a significant effect on the total amount of locomotor activity and shuttle crossings at the 0.3 mg/kg dose ($p<.05$). It also significantly reduced the amount of time spent in the dark area at this dosage level.

| | | Shuttles | | Time Dark | | Locomotor | |
|---|---|---|---|---|---|---|---|
| Cmpd | Dose mg/kg | Mean | % Con | Mean | % Con | Mean | % Con |
| Cmpd 1 | 0.3 | 27.25 ± 1.70 | 141.56 | 4.63 ± 0.24 | 75.47 | 199.25 ± 30.0 | 136.24 |
| Cntrl | | 19.25 | ± 1.70 | 6.13 | ± 0.258 | 146.25 | ± 15.1 |

## Claims

### Claims for the following Contracting States : AT, BE, CH, DE, FR, GB, IT, LI, LU, NL, SE

**1.** A compound of the formula (I):

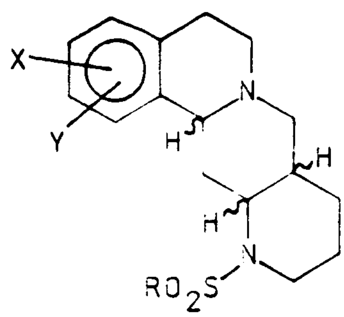

(I)

in which:

X and Y are independently hydrogen, hydroxy, lower alkyl of one to six carbon atoms, lower alkoxy of one to six carbon atoms or halo, or X and Y taken together is methylenedioxy or ethylene-1,2-dioxy; and

R is lower alkyl of one to six carbon atoms; phenyl optionally substituted by one or two substituents chosen from halo or amino groups or lower alkyl or lower alkoxy groups of one to four carbon atoms; $-(CH_2)_mOR^1$; or $-NR^1R^2$ wherein m is an integer of 1 to 6 and $R^1$ and $R^2$ are independently hydrogen or lower alkyl of one to six carbon atoms; or the group $-NR^1R^2$ forms a heterocycle of the formula:

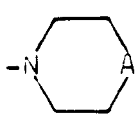

wherein A is $-CH_2-$, $-NR^1-$ or oxygen, wherein $R^1$ has the above meaning and the wavy lines indicate that the hydrogen atom attached thereto is in either the α- or β-position; or a pharmaceutically acceptable salt thereof.

**2.** A compound of claim 1 of the formula (1) or (2):

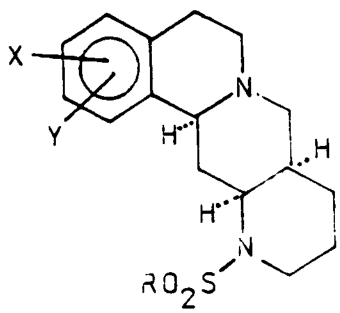

(1)

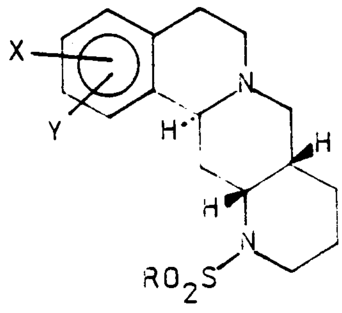

(2)

in which:

X, Y and R are as defined in claim 1; or a pharmaceutically acceptable salt thereof.

**3.** A compound of claim 2 which is a compound of formula (1); or a pharmaceutically acceptable salt thereof.

**4.** A compound of claim 3 in which X and Y are independently hydrogen or lower alkoxy having one to four carbon atoms, or X and Y taken together is methylenedioxy; or a pharmaceutically acceptable salt thereof.

**5.** A compound of claim 4 in which R is lower alkyl having one to six carbon atoms; or a pharmaceutically acceptable salt thereof.

**6.** A compound of claim 5, namely (±)-2,3-methylenedioxy-12-methanesulfonyl-5,6,8aα,9,10,11,12,12aα,13,13aα-decahydro-8H-isoquino-[2,1-g][1,6]naphthyridine;
(8aR,12aS,13aS)-2,3-methylenedioxy-12-methanesulfonyl-5,6,8a,9,10,11,12,12a,13,13a-decahydro-8H-isoquino[2,1-g][1,6]naphthyridine;
(±)-2,3-methylenedioxy-12-(2-methylpropanesulfonyl-5,6,8aα,9,10,11,12,12aα,13,13aα-decahydro-8H-isoquino[2,1-g][1,6]naphthyridine;
(8aR,12aS,13aS)-2,3-methylenedioxy-12-(2-methylpropanesulfonyl)-5,6,8a,9,10,11,12,12a,13,13a-decahydro-8H-isoquino[2,1-g][1,6]naphthyridine;
(±)-3-methoxy-12-methanesulfonyl-5,6,8aα,9,10,11,12,12aα,13,13aα-decahydro-8H-isoquino[2,1-g][1,6]-naphthyridine;
(8aR,12aS,13aS)-3-methoxy-12-methanesulfonyl-5,6,8a,9,10,11,12,12a,13,13a-decahydro-8H-isoquino-[2,1-g][1,6]naphthyridine;
(±)-3-methoxy-12-(2-methylpropanesulfonyl)-5,6,8aα,9,10,11,12,12aα,13,13aα-decahydro-8H-isoquino-[2,1-g][1,6]naphthyridine;
(8aR,12aS,13aS)-3-methoxy-12-(2-methylpropanesulfonyl)-5,6,8a,9,10,11,12,12a,13,13a-decahydro-8H-isoquino[2,1-g][1,6]naphthyridine;
(±)-2,3-dimethoxy-12-methanesulfonyl-5,6,8aα,9,10,11,12,12aα,13,13aα-decahydro-8H-isoquino[2,1-g]-[1,6]naphthyridine;
(8aR,12aS,13aS)-2,3-dimethoxy-12-methanesulfonyl-5,6,8a,9,10,11,12,12a,13,13a-decahydro-8H-isoquino[2,1-g][1,6]naphthyridine;
(±)-12-methanesulfonyl-5,6,8aα,9,10,11,12,12aα,13,13aα-decahydro-8H-isoquino[2,1-g][1,6]-naphthyridine;
(8aR,12aS,13aS)-12-methanesulfonyl-5,6,8a,9,10,11,12,12a,13,13a-decahydro-8H-isoquino[2,1-g][1,6]-naphthyridine;
(±)-12-(2-methylpropanesulfonyl)-5,6,8aα,9,10,11,12,12aα,13,13aα-decahydro-8H-isoquino[2,1-g][1,6]-naphthyridine; or
(8aR,12aS,13aS)-12-(2-methylpropanesulfonyl)-5,6,8a,9,10,11,12,12a,13,13a-decahydro-8H-isoquino-[2,1-g][1,6]naphthyridine;
or a pharmaceutically acceptable salt thereof, especially the hydrochloride salt .

**7.** A compound of claim 4 in which R is $-NR^1R^2$, or a pharmaceutically acceptable salt thereof.

**8.** A compound of claim 7 in which $R^1$ and $R^2$ are independently hydrogen or lower alkyl of one to four carbon atoms, or a pharmaceutically acceptable salt thereof.

**9.** A compound of claim 8, namely (±)-12-(N,N-dimethylaminosulfonyl)-5,6,8aα,9,10,11,12,-12aα,13,13aα-decahydro-8H-isoquino[2,1-g][1,6]-naphthyridine; (8aR,12aS,13aS)-3-methoxy-12-N,N-dimethylamino-sulfonyl-5,6,8a,9,10,11,12,12a,13,13a-decahydro-8H-isoquino[2,1-g][1,6]naphthyridine; or a pharmaceutically acceptable salt thereof.

**10.** A compound of claim 4 in which R is $-(CH_2)_mOR^1$; or a pharmaceutically acceptable salt thereof.

**11.** A compound of claim 10 in which m is 2 and $R^1$ is methyl; or a pharmaceutically acceptable salt thereof.

**12.** A compound of claim 11, namely (±)-12-(2-methoxyethanesulfonyl)-5,6,8aα,9,10,11,12,12aα13,13aα-decahydro-8H-isoquino[2,1-g][1,6]-naphthyridine; (8aR,12aS,13aS)-12-(2-methoxyethanesulfonyl)-5,6,8a,9,10,11,12,12a,13,13a-decahydro-8H-isoquino[2,1-g][1,6]-naphthyridine;
(±)-3-methoxy-12-(2-methoxyethanesulfonyl)-5,6,8aα,9,10,11,12,12aα,13,13aα-decahydro-8H-isoquino-[2,1-g][1,6]naphthyridine; and
(8aR,12aS,13aS)-3-methoxy-12-(2-methoxyethanesulfonyl)-5,6,8a,9,10,11,12,12a,13,13a-decahydro-8H-isoquino[2,1-g][1,6]naphthyridine; or a pharmaceutically acceptable salt thereof.

**13.** A compound of claim 2 which is a compound of formula (2), or a pharmaceutically acceptable salt thereof.

**14.** A compound of claim 13 in which X and Y are independently hydrogen or lower alkoxy having one to four carbon atoms or X and Y taken together is methylenedioxy, or a pharmaceutically acceptable salt thereof.

**15.** A compound of claim 14 in which R is lower alkyl having one to four carbon atoms or $-NR^1R^2$, or a pharmaceutically acceptable salt thereof.

**16.** A pharmaceutical composition comprising a pharmaceutically acceptable non-toxic carrier and a therapeutically effective amount of a compound of one of the preceding claims.

**17.** A pharmaceutical composition of claim 16 suitable for administration to a mammal having a disease-state which is alleviated by treatment with an $\alpha_2$-blocker.

**18.** A pharmaceutical composition of claim 16 wherein the compound of one of the preceding claims is
(8aR,12aS,13aS)-2,3-methlenedioxy-12-methanesulfonyl-5,6,8a,9,10,11,12,12a,13,13a-decahydro-8H-isoquino[2,1-g][1,6]naphthyridine; or
(8aR,12aS,13aS)-3-methoxy-12-methanesulfonyl-5,6,8a,9,10,11,12,12a,13,13a-decahydro-8H-isoquino-[2,1-g][1,6]naphthyridine; or a pharmaceutically acceptable salt thereof, especially the hydrochloride salt.

**19.** A pharmaceutical composition of claim 17 or 18, wherein said disease-state is comprised of depression, excessive platelet aggregation, diabetes, elevated intraoccular pressure, male impotence, irritable-bowel syndrome, hypertension, anxiety, cyclic mood disturbances in women and/or obesity.

**20.** The use of a compound of any one of claims 1 to 15 in the manufacture of a medicament for treating a disease-state which is alleviated by treatment with an $\alpha_2$-blocker.

**21.** A compound of the formula

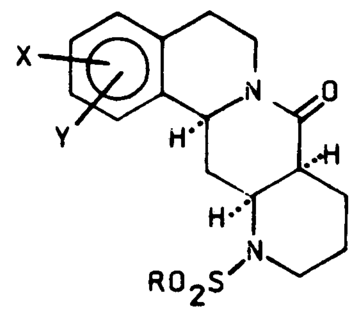

in which:
X, Y and R are as defined in claim 1.

**22.** A compound of the formula

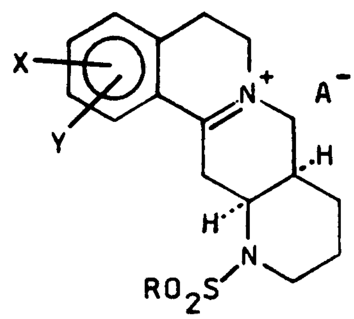

in which:
X, Y and R are as defined in claim 1 and A is representative of an organic or inorganic anion.

**23.** A compound of the formula

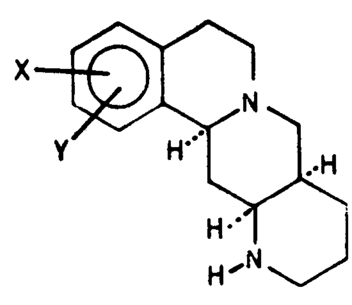

in which:
X and Y are as defined in claim 1.

**24.** The compound of claim 23 wherein at least one of X and Y is not hydrogen.

**25.** A compound of claim 24 wherein X is lower alkoxy of one to six carbons and Y is hydrogen, or X and Y taken together is methylenedioxy or ethylene-1,2-dioxy.

**26.** A process for the preparation of a compound of claim 1 said process comprising

(a) sulfonylating a compound of the formula

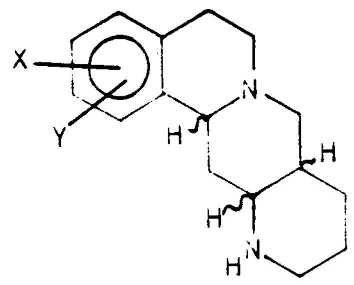

in which:

X, Y and the wavy lines are are as defined above at claim 1; with a sulfonic acid of the formula $RO_2SOH$ or an activated form thereof, wherein R is as defined above; or

(b) reducing a compound of the formula

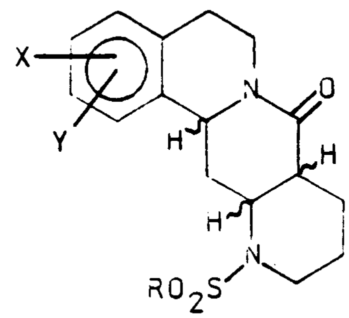

in which:

X, Y, R and the wavy lines are as defined above at claim 1; or

(c) alkylating a compound of the formula (I) in which:

at least one of X and Y is hydroxy and R and the wavy lines are as defined above at claim 1, to give a compound of the formula (I)

wherein:

at least one of X and Y is lower alkoxy of one to six carbon atoms or X and Y taken together is methylenedioxy or ethylene-1,2-dioxy; or

(d) oxidizing a compound of the formula

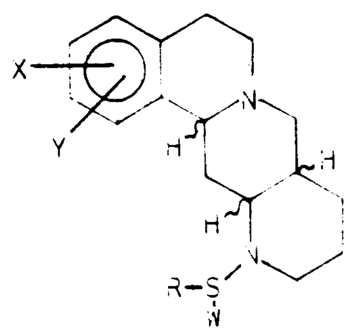

in which X, Y, R and the wavy lines are as defined above at claim 1 and W is an electron pair or an oxygen atom; or

(e) reducing a compound of the formula

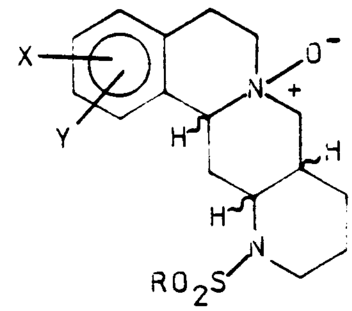

in which X, Y, R and the wavy lines are as defined above at claim 1; or
(f) epimerizing a compound of the formula

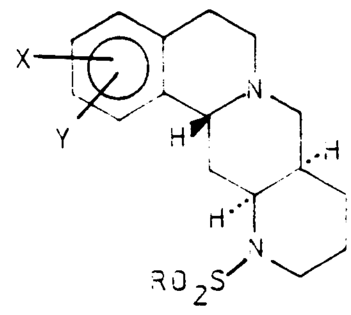

in which X, Y and R are as defined above at claim 1 to a compound of the formula

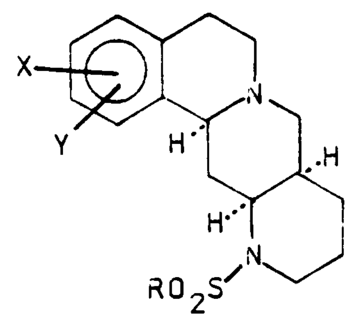

in which X, Y and R are as defined above at claim 1; or
(g) reducing a compound of the formula

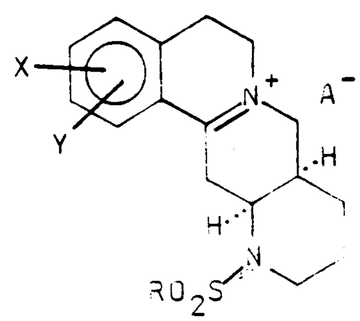

in which X, Y and R are as defined above at claim 1 and A is representative of an organic or inorganic anion, to give a compound of the formula

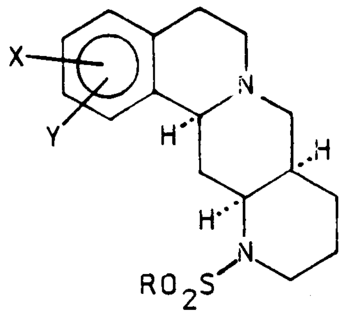

in which X, Y and R are as defined above at claim 1; or

(h) reducing a compound of the formula (I) in which:

X, Y and the wavy lines are as defined above at claim 1 and R is phenyl substituted by one or two nitro groups,

to give a compound of the formula (I) in which:

X, Y and the wavy lines are as defined above at claim 1 and R is phenyl substituted by one or two amino groups; or

(i) dealkylating a compound of the formula (I) in which:

X, Y and the wavy lines are as defined above at claim 1 and R is $-(CH_2)_mOR^1$ wherein m is an integer of 1 to 6 and $R^1$ is lower alkyl,

to give a compound of the formula (I) in which:

X, Y and the wavy lines are as defined above at claim 1 and R is $-(CH_2)_mOH$ wherein m is as defined above at claim 1; or

(j) alkylating a compound of the formula (I) in which:

X, Y and the wavy lines are as defined above at claim 1 and R is $-(CH_2)_mOH$ wherein m is an integer of 1 to 6,

to give a compound of the formula (I) in which:

X, Y and the wavy lines are as defined above at claim 1 and R is $-(CH_2)_mOR^1$ wherein m is as defined above at claim 1 and $R^1$ is lower alkyl; or

(k) converting a compound of the formula (I) to a salt thereof; or

(l) converting a salt of a compound of the formula (I) to the free compound of the formula (I); or

(m) converting a salt of a compound of the formula (I), preferably a soluble salt, to another salt of the compound of the formula (I), preferably less soluble than said soluble salt; or

(n) resolving a racemic or non-racemic mixture of the compounds of the formulae (1) and (3)

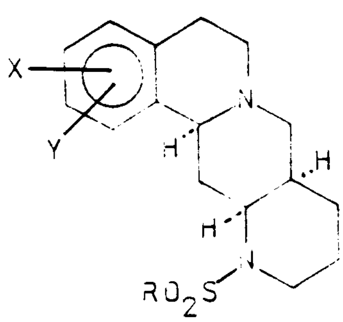

(1)

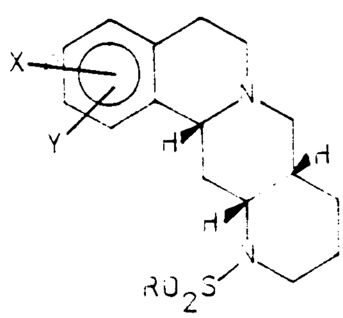

(3)

in which:

x, Y and R are as defined above at claim 1, to give an optically enriched compound of the formula (1).

**27.** The process of claim 26 wherein (±)-2,3-methylenedioxy-12-methanesulfonyl-5,6,8aα,9,10,11,12,12aα,13,13aα-decahydro-8H-isoquino-[2,1-g][1,6]naphthyridine;
(8aR,12aS,13aS)-2,3-methylenedioxy-12-methanesulfonyl-5,6,8a,9,10,11,12,12a,13,13a-decahydro-8H-isoquino[2,1-g][1,6]naphthyridine;
(±)-3-methoxy-12-methanesulfonyl-5,6,8aα,9,10,11,12,12aα,13,13aα-decahydro-8H-isoquino[2,1-g][1,6]-naphthyridine;
(8aR,12aS,13aS)-3-methoxy-12-methanesulfonyl-5,6,8a,9,10,11,12,12a,13,13a-decahydro-8H-isoquino-[2,1-g][1,6]naphthyridine; or a pharmaceutically acceptable salt thereof, especially the hydrochloride salt , is prepared.

**28.** A method of preparing a pharmaceutical composition wherein a compound of any one of claims 1 to 15 or a compound which has been prepared by the process of claim 26 or 27 is combined with a pharmaceutically acceptable non-toxic carrier.

**29.** A single enantiomer of the formula:

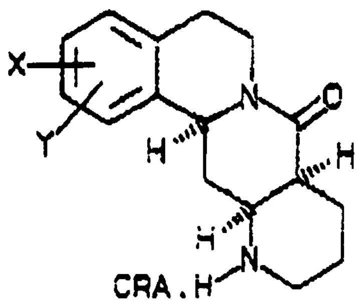

wherein:
X and Y are independently hydrogen; hydroxy; lower alkyl of one to six carbon atoms; lower alkoxy of one of six carbon atoms; or halo; or X and Y when adjacent and taken together are methylenedioxy or ethylene-1,2-dioxy; and CRA indicates an optically active acid.

**Claims for the following Contracting States : ES, GR**

**1.** A process for the preparation of a compound of the formula (I)

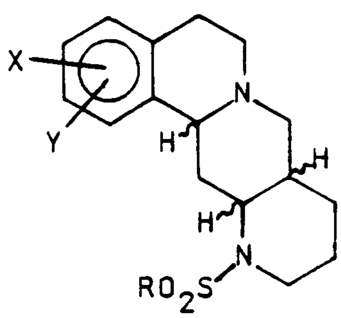

(I)

in which:

X and Y are independently hydrogen, hydroxy, lower alkyl of one to six carbon atoms, lower alkoxy of one to six carbon atoms or halo, or X and Y taken together is methylenedioxy or ethylene-1,2-dioxy; and

R is lower alkyl of one to six carbon atoms; phenyl optionally substituted by one or two substituents chosen from halo or amino groups or lower alkyl or lower alkoxy groups of one to four carbon atoms; $-(CH_2)_mOR^1$; or $-NR^1R^2$ wherein m is an integer of 1 to 6 and

$R^1$ and $R^2$ are independently hydrogen or lower alkyl of one to six carbon atoms; or the group $-NR^1R^2$ forms a heterocycle of the formula:

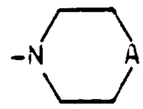

wherein A is $-CH_2-$, $-NR^1-$ or oxygen, wherein $R^1$ has the above meaning and the wavy lines indicate that the hydrogen atom attached thereto is either in the α- or β-position; or a pharmaceutically acceptable salt thereof; said process comprising

(a) sulfonylating a compound of the formula

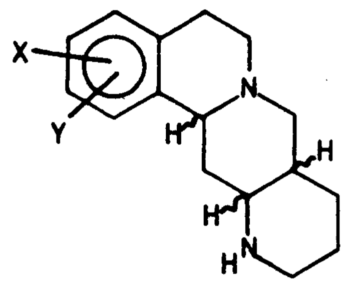

in which:

X, Y and the wavy lines are are as defined above; with a sulfonic acid of the formula $RO_2SOH$ or an activated form thereof, wherein R is as defined above; or

(b) reducing a compound of the formula

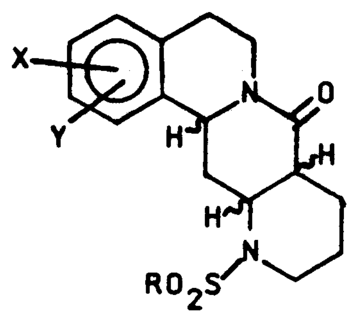

in which:

X, Y, R and the wavy lines are as defined above; or

(c) alkylating a compound of the formula (I) in which:

at least one of X and Y is hydroxy and R and the wavy lines are as defined above, to give a compound of the formula (I)

wherein:

at least one of X and Y is lower alkoxy of one to six carbon atoms or X and Y taken together is methylenedioxy or ethylene-1,2-dioxy; or

(d) oxidizing a compound of the formula

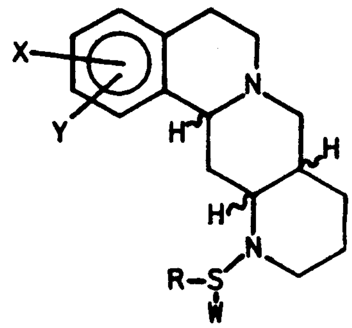

in which X, Y, R and the wavy lines are as defined above and W is an electron pair or an oxygen atom; or

(e) reducing a compound of the formula

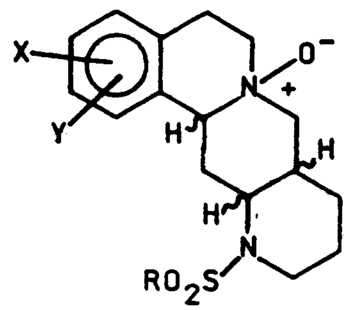

in which X, Y, R and the wavy lines are as defined above; or

(f) epimerizing a compound of the formula

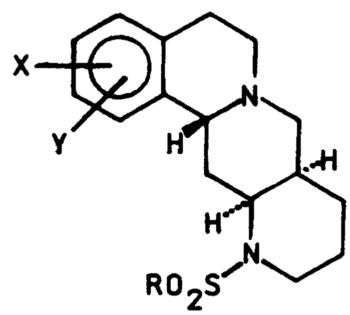

in which X, Y and R are as defined above to a compound of the formula

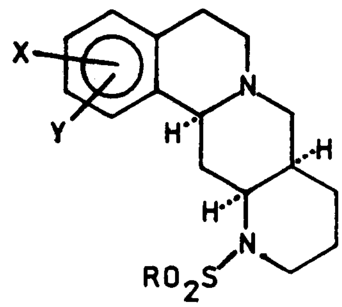

in which X, Y and R are as defined above; or

(g) reducing a compound of the formula

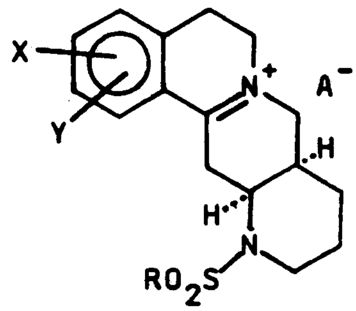

in which X, Y and R are as defined above and A is representative of an organic or inorganic anion, to give a compound of the formula

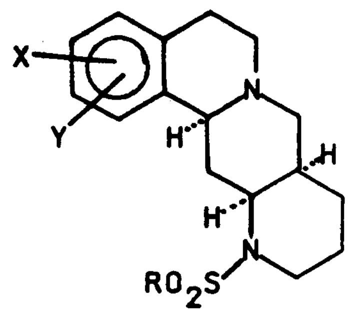

in which X, Y and R are as defined above; or

(h) reducing a compound of the formula (I) in which:

X, Y and the wavy lines are as defined above and R is phenyl substituted by one or two nitro groups,

to give a compound of the formula (I) in which:

X, Y and the wavy lines are as defined above and R is phenyl substituted by one or two amino groups; or

(i) dealkylating a compound of the formula (I) in which:

X, Y and the wavy lines are as defined above and R is $-(CH_2)_mOR^1$ wherein m is an integer of 1 to 6 and $R^1$ is lower alkyl,

to give a compound of the formula (I) in which:

X, Y and the wavy lines are as defined above and R is $-(CH_2)_mOH$ wherein m is as defined above; or

(j) alkylating a compound of the formula (I) in which:

X, Y and the wavy lines are as defined above and R is $-(CH_2)_mOH$ wherein m is an integer of 1 to 6,

to give a compound of the formula (I) in which:

X, Y and the wavy lines are as defined above and R is $-(CH_2)_mOR^1$ wherein m is as defined above and $R^1$ is lower alkyl; or

(k) converting a compound of the formula (I) to a salt thereof; or

(l) converting a salt of a compound of the formula (I) to the free compound of the formula (I); or

(m) converting a salt of a compound of the formula (I), preferably a soluble salt, to another salt of the compound of the formula (I), preferably less soluble than said soluble salt; or

(n) resolving a racemic or non-racemic mixture of the compounds of the formulae (1) and (3)

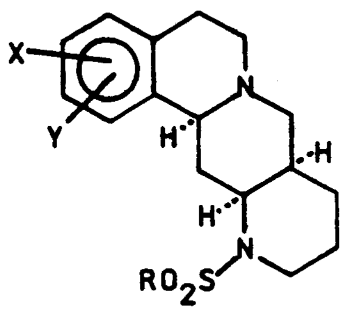

(1)

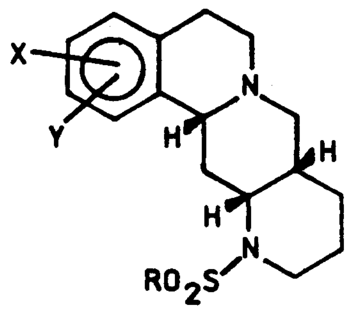

(3)

in which:

X, Y and R are as defined above,

to give an optically enriched compound of the formula (1).

**2.** A process of claim 1 in which a compound of the formula (1) or (2):

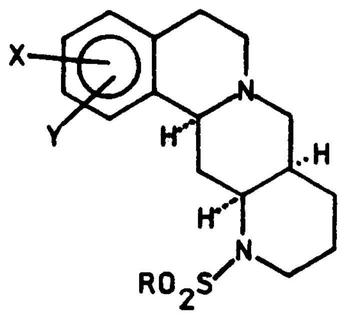

(1)

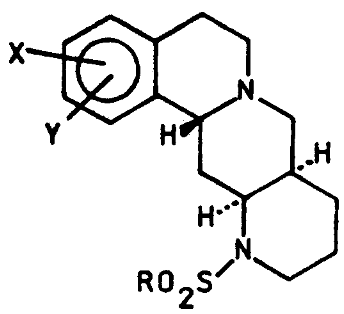

(2)

in which:

X, Y and R are as defined above; or a pharmaceutically acceptable salt thereof, is prepared.

**3.** A process of claim 2 in which a compound of formula (1); or a pharmaceutically acceptable salt thereof, is prepared.

**4.** A process of claim 3 in which X and Y are independently hydrogen or lower alkoxy having one to four carbon atoms, or X and Y taken together is methylenedioxy.

**5.** A process of claim 4 in which R is lower alkyl having one to six carbon atoms.

**6.** A process of claim 5 in which (±)-2,3-methylenedioxy-12-methanesulfonyl-5,6,8aα,9,10,11,12,12aα,13,13aα-decahydro-8H-isoquino-[2,1-g][1,6]naphthyridine;
(8aR,12aS,13aS)-2,3-methylenedioxy-12-methanesulfonyl-5,6,8a,9,10,11,12,12a,13,13a-decahydro-8H-isoquino-[2,1-g][1,6]naphthyridine;
(±)-2,3-methylenedioxy-12-(2-methylpropanesulfonyl)-5,6,8aα,9,10,11,12,12aα,13,13aα-decahydro-8H-isoquino[2,1-g][1,6]naphthyridine;
(8aR,12aS,13aS)-2,3-methylenedioxy-12-(2-methylpropanesulfonyl)-5,6,8a,9,10,11,12,12a,13,13a-decahydro-8H-isoquino-[2,1-g][1,6]naphthyridine;
(±)-3-methoxy-12-methanesulfonyl-5,6,8aα,9,10,11,12,12aα,13,13aα-decahydro-8H-isoquino[2,1-g][1,6]-naphthyridine;
(8aR,12aS,13aS)-3-methoxy-12-methanesulfonyl-5,6,8a,9,10,11,12,12a,13,13a-decahydro-8H-isoquino-

[2,1-g][1,6]naphthyridine;
(±)-3-methoxy-12-(2-methylpropanesulfonyl)-5,6,8aα,9,10,11,12,12aα,13,13aα-decahydro-8H-isoquino-[2,1-g][1,6]naphthyridine;
(8aR,12aS,13aS)-3-methoxy-12-(2-methylpropanesulfonyl)-5,6,8a,9,10,11,12,12a,13,13a-decahydro-8H-isoquino-[2,1-g][1,6]naphthyridine;
(±)-2,3-dimethoxy-12-methanesulfonyl-5,6,8aα,9,10,11,12,12aα,13,13aα-decahydro-8H-isoquino[2,1-g]-[1,6]naphthyridine;
(8aR,12aS,13aS)-2,3-dimethoxy-12-methanesulfonyl-5,6,8a,9,10,11,12,12a,13,13a-decahydro-8H-isoquino-[2,1-g][1,6]naphthyridine;
(±)-12-methanesulfonyl-5,6,8aα,9,10,11,12,12aα,13,13aα-decahydro-8H-isoquino[2,1-g][1,6]-naphthyridine;
(8aR,12aS,13aS)-12-methanesulfonyl-5,6,8a,9,10,11,12,12a,13,13a-decahydro-8H-isoquino[2,1-g][1,6]-naphthyridine;
(±)-12-(2-methylpropanesulfonyl)-5,6,8aα,9,10,11,12,12aα,13,13aα-decahydro-8H-isoquino[2,1-g][1,6]-naphthyridine;
(8aR,12aS,13aS)-12-(2-methylpropanesulfonyl)-5,6,8a,9,10,11,12,12a,13,13a-decahydro-8H-isoquino-[2,1-g][1,6]naphthyridine;
or a pharmaceutically acceptable salt thereof, especially the hydrochloride salt , is prepared.

**7.** A process of claim 4 in which R is $-NR^1R^2$ .

**8.** A process of claim 7 in which $R^1$ and $R^2$ are independently hydrogen or lower alkyl of one to four carbon atoms.

**9.** A process of claim 8 in which (±)-12-(N,N-dimethylaminosulfonyl)-5,6,8aα,9,10,11,12,-12aα,13,13aα-decahydro-8H-isoquino[2,1-g][1,6]-naphthyridine; or
(8aR,12aS,13aS)-3-methoxy-12-N,N-dimethylamino-sulfonyl-5,6,8a,9,10,11,12,12a,13,13a-decahydro-8H-isoquino[2,1-g][1,6]naphthyridine; or a pharmaceutically acceptable salt thereof, is prepared.

**10.** A process of claim 4 in which R is $-(CH_2)_mOR^1$.

**11.** A process of claim 10 in which m is 2 and $R^1$ is methyl.

**12.** A process of claim 11 in which (±)-12-(2-methoxyethanesulfonyl)-5,6,8aα,9,10,11,12,12aα,13,13aα-decahydro-8H-isoquino[2,1-g][1,6]-naphthyridine;
(8aR,12aS,13aS)-12-(2-methoxyethanesulfonyl)-5,6,8a,9,10,11,12,12a,13,13a-decahydro-8H-isoquino-[2,1-g][1,6]naphthyridine;
(±)-3-methoxy-12-(2-methoxyethanesulfonyl)-5,6,8aα,9,10,11,12,12aα,13,13aα-decahydro-8H-isoquino-[2,1-g][1,6]naphthyridine; or
(8aR,12aS,13aS)-3-methoxy-12-(2-methoxyethanesulfonyl-5,6,8a,9,10,11,12,12a,13,13a-decahydro-8H-isoquino[2,1-g][1,6]naphthyridine; or a pharmaceutically acceptable salt thereof, is prepared.

**13.** A process of claim 2 in which a compound of formula (2), or a pharmaceutically acceptable salt thereof, is prepared.

**14.** A process of claim 13 in which X and Y are independently hydrogen or lower alkoxy having one to four carbon atoms or X and Y taken together is methylenedioxy.

**15.** A process of claim 14 in which R is lower alkyl having one to four carbon atoms or $-NR^1R^2$.

**16.** A method of preparing a pharmaceutical composition wherein a therapeutically effective amount of a compound prepared by the process of any one of the preceding claims or a pharmaceutically acceptable salt thereof is combined with a pharmaceutically acceptable non-toxic carrier.

**17.** A method of claim 16 in which the pharmaceutical composition is suitable for administration to a mammal having a disease-state which is alleviated by treatment with an $\alpha_2$-blocker.

**18.** A method of claim 16 in which the compound is (8aR,12aS,13aS)-2,3-methylenedioxy-12-methanesulfonyl-5,6,8a,9,10,11,12,12a,13,13a-decahydro-8H-isoquino[2,1-g][1,6]naphthyridine; or (8aR,12aS,13aS)-3-methoxy-12-methanesulfonyl-5,6,8a,9,10,11,12,12a,13,13a-decahydro-8H-isoquino-[2,1-g][1,6]naphthyridine; or a pharmaceutically acceptable salt thereof, especially the hydrochloride salt.

**19.** A method of claim 17 or 18, in which said disease-state is comprised of depression, excessive platelet aggregation, diabetes, elevated intraoccular pressure, male impotence, irritable-bowel syndrome, hypertension, anxiety, cyclic mood disturbances in women or obesity.

**20.** The use of a compound as prepared by the process of any one of claims 1 to 15 in the manufacture of a medicament for treating a disease-state which is alleviated by treatment with an $\alpha_2$-blocker.

**21.** A method for the production of a single enantiomer of the formula:

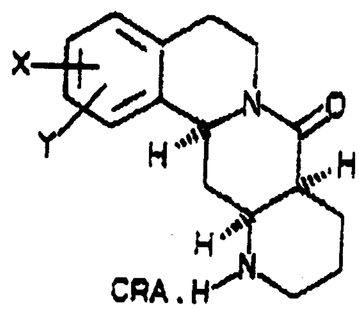

wherein:
X and Y are independently hydrogen; hydroxy; lower alkyl of one to six carbon atoms; lower alkoxy of one to six carbon atoms; or halo; or X and Y when adjacent and taken together are methylenedioxy or ethylene-1,2-dioxy; and CRA is an optically active acid;
the method comprising separating the diastereomeric salts formed by the reaction of a racemic mixture of a compound of formula (V), wherein X and Y are as defined above.

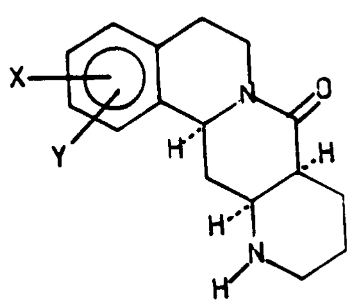

(V)

with an optically active acid, at a temperature between 0 ° C and the reflux temperature of the solvent employed for fractional crystallisation.

**Patentansprüche**

**Patentansprüche für folgende Vertragsstaaten : AT, BE, CH, DE, FR, GB, IT, LI, LU, NL, SE**

1. Verbindung der Formel (I):

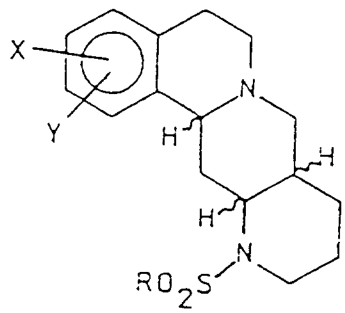

(I)

in der :

X und Y unabhängig für Wasserstoff, Hydroxy, Niederalkyl mit 1 bis 6 Kohlenstoffatomen, Niederalkoxy mit 1 bis 6 Kohlenstoffatomen oder Halogen stehen oder X und Y zusammengenommen Methylendioxy oder Ethylen-1,2-dioxy sind; und

R für Niederalkyl mit 1 bis 6 Kohlenstoffatomen; Phenyl, gegebenenfalls durch ein oder zwei Substituenten substituiert, die aus Halogen- oder Aminogruppen oder Niederalkyl- oder Niederalkoxygruppen mit 1 bis 4 Kohlenstoffatomen ausgewählt sind; $-(CH_2)_mOR^1$; oder $-NR^1R^2$ steht, worin m eine ganze Zahl von 1 bis 6 ist und $R^1$ und $R^2$ unabhängig Wasserstoff oder Niederalkyl mit 1 bis 6 Kohlenstoffatomen sind; oder die Gruppe $-NR^1R^2$ einen Heterocyclus der Formel:

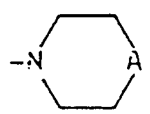

bildet, worin A $-CH_2-$, $-NR^1-$ oder Sauerstoff ist, worin $R^1$ die obige Bedeutung hat, und die Wellenlinien anzeigen, daß sich das daran angefügte Wasserstoffatom entweder in der $\alpha$- oder $\beta$-Stellung befindet; oder ein pharmazeutisch annehmbares Salz derselben.

2. Verbindung nach Anspruch 1 der Formel (1) oder (2):

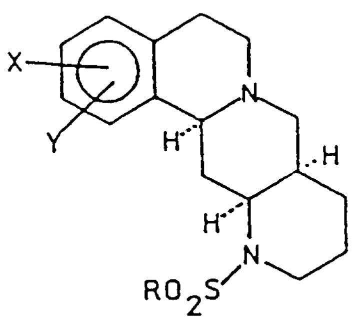

(1)

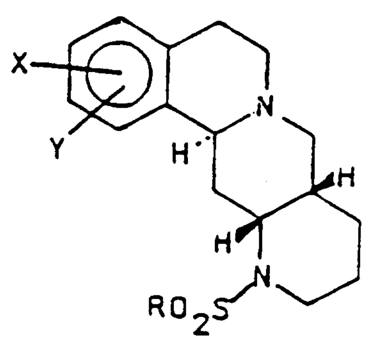

(2)

worin:

X, Y und R wie in Anspruch 1 definiert sind; oder ein pharmazeutisch annehmbares Salz derselben.

3. Verbindung nach Anspruch 2, die eine Verbindung der Formel (1) ist; oder ein pharmazeutisch annehmbares Salz derselben.

4. Verbindung nach Anspruch 3, in der X und Y unabhängig Wasserstoff oder Niederalkoxy mit 1 bis 4 Kohlenstoffatomen sind oder X und Y zusammengenommen Methylendioxy sind; oder ein pharmazeutisch annehmbares Salz derselben.

5. Verbindung nach Anspruch 4, in der R Niederalkyl mit 1 bis 6 Kohlenstoffatomen ist; oder ein pharmazeutisch annehmbares Salz derselben.

6. Verbindung nach Anspruch 5, nämlich (±)-2,3-Methylendioxy-12-methansulfonyl-5,6,8aα,9,10,11,12,12aα,13,13aα-decahydro-8H-isochino[2,1-g][1,6]naphthyridin;
(8aR,12aS,13aS)-2,3-Methylendioxy-12-methansulfonyl-5,6,8a,9,10,11,12,12a,13,13a-decahydro-8H-isochino[2,1-g][1,6]naphthyridin;
(±)-2,3-Methylendioxy-12-(2-methylpropansulfonyl)-5,6,8aα,9,10,11,12,12aα,13,13aα-decahydro-8H-isochino[2,1-g][1,6]naphthyridin;
(8aR,12aS,13aS)-2,3-Methylendioxy-12-(2-methylpropansulfonyl)-5,6,8a,9,10,11,12,12a,13,13a-decahydro-8H-isochino-[2,1-g][1,6]-naphthyridin;
(±)-3-Methoxy-12-methansulfonyl-5,6,8aα,9,10,11,12,12aα,13,13aα-decahydro-8H-isochino[2,1-g][1,6]-naphthyridin;
(8aR,12aS,13aS)-3-Methoxy-12-methansulfonyl-5,6,8a,9,10,11,12,12a,13,13a-decahydro-8H-isochino[2,1-g][1,6]naphthyridin;
(±)-3-Methoxy-12-(2-methylpropansulfonyl)-5,6,8aα,9,10,11,12,12aα,13,13aα-decahydro-8H-isochino[2,1-g][1,6]naphthyridin;
(8aR,12aS,13aS)-3-Methoxy-12-(2-methylpropansulfonyl)-5,6,8a,9,10,11,12,12a,13,13a-decahydro-8H-isochino[2,1-g][1,6]naphthyridin;
(±)-2,3-Dimethoxy-12-methansulfonyl-5,6,8aα,9,10,11,12,12aα,13,13aα-decahydro-8H-isochino[2,1-g]-[1,6]naphthyridin;
(8aR,12aS,13aS)-2,3-Dimethoxy-12-methansulfonyl-5,6,8a,9,10,11,12,12a,13,13a-decahydro-8H-isochino[2,1-g][1,6]naphthyridin;
(±)-12-Methansulfonyl-5,6,8aα,9,10,11,12,12aα,13,13aα-decahydro-8H-isochino[2,1-g][1,6]naphthyridin;
(8aR,12aS,13aS)-12-Methansulfonyl-5,6,8a,9,10,11,12,12a,13,13a-decahydro-8H-isochino[2,1-g][1,6]-naphthyridin;
(±)-12-(2-Methylpropansulfonyl)-5,6,8aα,9,10,11,12,12aα,13,13aα-decahydro-8H-isochino [2,1-g][1,6]-naphthyridin; oder
(8aR,12aS,13aS)-12-(2-Methylpropansulfonyl)-5,6,8a,9,10,11,12,12a,13,13a-decahydro-8H-isochino[2,1-g][1,6]naphthyridin;
oder ein pharmazeutisch annehmbares Salz derselben, insbesondere das Hydrochloridsalz.

7. Verbindung nach Anspruch 4, in der R $-NR^1R^2$ ist, oder ein pharmazeutisch annehmbares Salz derselben.

8. Verbindung nach Anspruch 7, in der $R^1$ und $R^2$ unabhängig Wasserstoff oder Niederalkyl mit 1 bis 4 Kohlenstoffatomen sind, oder ein pharmazeutisch annehmbares Salz derselben.

9. Verbindung nach Anspruch 8, nämlich (±)-12-(N,N-Dimethylaminosulfonyl)-5,6,8aα,9,10,11,12,12aα,13,13aα-decahydro-8H-isochino[2,1-g][1,6]naphthyridin; (8aR,12aS,13aS)-3-Methoxy-12-N,N-dimethylaminosulfonyl-5,6,8a,9,10,11,12,12a,13,13a-decahydro-8H-isochino[2,1-g][1,6]-naphthyridin; oder ein pharmazeutisch annehmbares Salz derselben.

10. Verbindung nach Anspruch 4, in der R $-(CH_2)_mOR^1$ ist; oder ein pharmazeutisch annehmbares Salz derselben.

11. Verbindung nach Anspruch 10, in der m 2 ist und $R^1$ Methyl ist; oder ein pharmazeutisch annehmbares Salz derselben.

12. Verbindung nach Anspruch 11, nämlich (±)-12-(2-Methoxyethansulfonyl)-5,6,8aα,9,10,11,12,12aα,13,13aα-decahydro-8H-isochino[2,1-g][1,6]naphthyridin;

(8aR,12aS,13aS)-12-(2-Methoxyethansulfonyl)-5,6,8a,9,10,11,12,12a,13,13a-decahydro-8H-isochino [2,1-g][1,6]naphthyridin;
(±)-3-Methoxy-12-(2-methoxyethansulfonyl)-5,6,8aα,9,10,11,12,12aα,13,13aα-decahydro-8H-isochino[2,1-g][1,6]naphthyridin; und
(8aR,12aS,13aS)-3-Methoxy-12-(2-methoxyethansulfonyl)-5,6,8a,9,10,11,12,12a,13,13a-decahydro-8H-isochino[2,1-g][1,6]naphthyridin; oder ein pharmazeutisch annehmbares Salz derselben.

**13.** Verbindung nach Anspruch 2, die eine Verbindung der Formel (2) ist, oder ein pharmazeutisch annehmbares Salz derselben.

**14.** Verbindung nach Anspruch 13, in der X und Y unabhängig Wasserstoff oder Niederalkoxy mit 1 bis 4 Kohlenstoffatomen sind oder X und Y zusammengenommen Methylendioxy sind, oder ein pharmazeutisch annehmbares Salz derselben.

**15.** Verbindung nach Anspruch 14, in der R Niederalkyl mit 1 bis 4 Kohlenstoffatomen oder $-NR^1R^2$ ist, oder ein pharmazeutisch annehmbares Salz derselben.

**16.** Pharmazeutische Zusammensetzung, umfassend einen pharmazeutisch annehmbaren, nicht-toxischen Träger und eine therapeutisch wirksame Menge einer Verbindung nach einem der vorangehenden Ansprüche.

**17.** Pharmazeutische Zusammensetzung nach Anspruch 16, die zur Verabreichung an einen Säuger mit einem Krankheitszustand, welcher durch Behandlung mit einem $\alpha_2$-Blocker gemildert wird, geeignet ist.

**18.** Pharmazeutische Zusammensetzung nach Anspruch 16, in der die Verbindung nach einem der vorangehenden Ansprüche (8aR,12aS,13aS)-2,3-Methylendioxy-12-methansulfonyl-5,6,8a,9,10,11,12,12a,13,13a-decahydro-8H-isochino[2,1-g][1,6]naphthyridin; oder
(8aR,12aS,13aS)-3-Methoxy-12-methansulfonyl-5,6,8a,9,10,11,12,12a,13,13a-decahydro-8H-isochino[2,1-g][1,6]naphthyridin; oder ein pharmazeutisch annehmbares Salz davon, insbesondere das Hydrochloridsalz, ist.

**19.** Pharmazeutische Zusammensetzung nach Anspruch 17 oder 18, in der der Krankheitszustand Depression, übermäßige Blutplättchenaggregation, Diabetes, erhöhten intraoccularen Druck, männliche Impotenz, Reizdarmsyndrom, Hochdruck, Unruhe, zyklische Stimmungsstörungen bei Frauen und/oder Fettleibigkeit umfaßt.

**20.** Verwendung einer Verbindung nach irgendeinem der Ansprüche 1 bis 15 bei der Herstellung eines Medikaments zur Behandlung eines Krankheitszustandes, der durch Behandlung mit einem $\alpha_2$-Blocker gemildert wird.

**21.** Verbindung der Formel

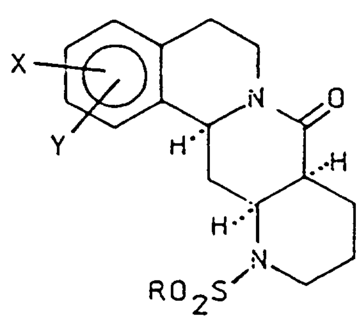

in der
X, Y und R wie in Anspruch 1 definiert sind.

**22.** Verbindung der Formel

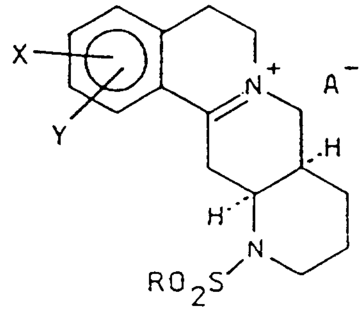

in der
X, Y und R wie in Anspruch 1 definiert sind und A ein organisches oder anorganisches Anion darstellt.

**23.** Verbindung der Formel

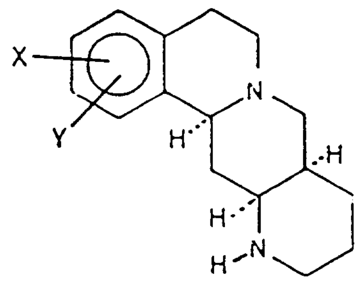

in der
X und Y wie in Anspruch 1 definiert sind.

**24.** Verbindung nach Anspruch 23, in der mindestens eines von X und Y nicht Wasserstoff ist.

**25.** Verbindung nach Anspruch 24, worin X Niederalkoxy mit 1 bis 6 Kohlenstoffatomen ist und Y Wasserstoff ist oder X und Y zusammengenommen Methylendioxy oder Ethylen-1,2-dioxy sind.

**26.** Verfahren zur Herstellung einer Verbindung nach Anspruch 1, umfassend
(a) das Sulfonylieren einer Verbindung der Formel

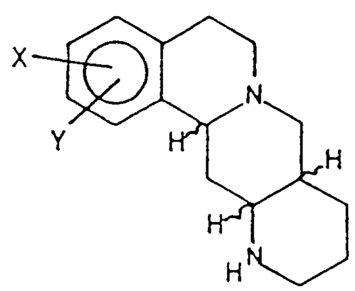

in der:
X, Y und die Wellenlinien wie oben in Anspruch 1 definiert sind; mit einer Sulfonsäure der Formel $RO_2SOH$ oder einer aktivierten Form derselben, worin R wie oben definiert ist; oder

(b) das Reduzieren einer Verbindung der Formel

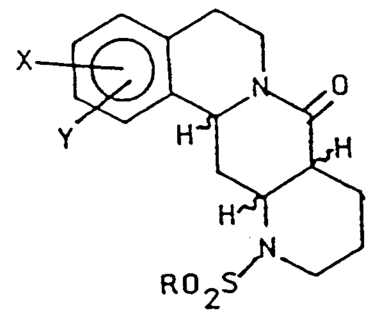

in der:
X, Y, R und die Wellenlinien wie oben in Anspruch 1 definiert sind; oder
(c) das Alkylieren einer Verbindung der Formel (I), in der:
mindestens eines von X und Y Hydroxy ist und R und die Wellenlinien wie oben in Anspruch 1 definiert sind,
um eine Verbindung der Formel (I) zu ergeben, in der:
mindestens eines von X und Y Niederalkoxy mit 1 bis 6 Kohlenstoffatomen ist oder X und Y zusammengenommen Methylendioxy oder Ethylen-1,2-dioxy sind; oder
(d) das Oxidieren einer Verbindung der Formel

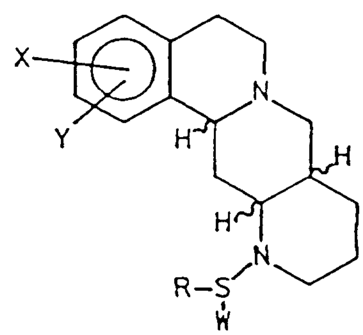

in der X, Y, R und die Wellenlinien wie oben in Anspruch 1 definiert sind und W ein Elektronenpaar oder ein Sauerstoffatom ist; oder
(e) das Reduzieren einer Verbindung der Formel

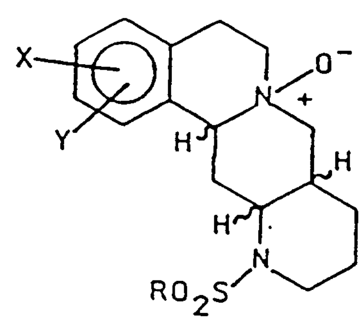

in der X, Y, R und die Wellenlinien wie oben in Anspruch 1 definiert sind; oder

(f) das Epimerisieren einer Verbindung der Formel

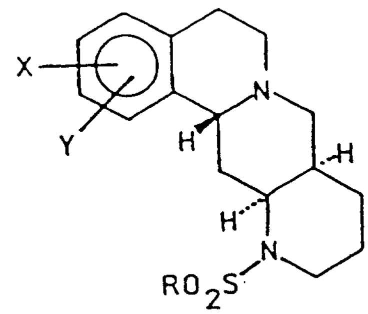

in der X, Y und R wie oben in Anspruch 1 definiert sind, zu einer Verbindung der Formel

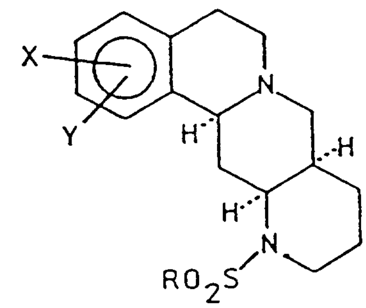

in der X, Y und R wie oben in Anspruch 1 definiert sind; oder
(g) das Reduzieren einer Verbindung der Formel

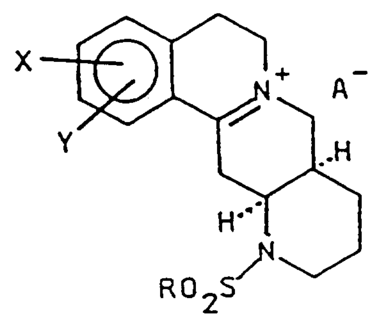

in der X, Y und R wie oben in Anspruch 1 definiert sind und A ein organisches oder anorganisches Anion darstellt, um eine Verbindung der Formel

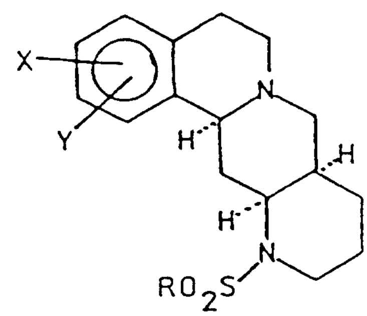

zu ergeben, in der X, Y und R wie oben in Anspruch 1 definiert sind; oder
(h) das Reduzieren einer Verbindung der Formel (I), in der:
X, Y und die Wellenlinien wie oben in Anspruch 1 definiert sind und R durch ein oder zwei Nitrogruppen substituiertes Phenyl ist,
um eine Verbindung der Formel (I) zu ergeben, in der:
X, Y und die Wellenlinien wie oben in Anspruch 1 definiert sind und R durch ein oder zwei Aminogruppen substituiertes Phenyl ist; oder
(i) das Desalkylieren einer Verbindung der Formel (I), in der:
X, Y und die Wellenlinien wie oben in Anspruch 1 definiert sind und R $-(CH_2)_mOR^1$ ist, worin m eine ganze Zahl von 1 bis 6 ist und $R^1$ Niederalkyl ist,
um eine Verbindung der Formel (I) zu ergeben, in der :
X, Y und die Wellenlinien wie oben in Anspruch 1 definiert sind und R $-(CH_2)_mOH$ ist, worin m wie oben in Anspruch 1 definiert ist; oder
(j) das Alkylieren einer Verbindung der Formel (I), in der:
X, Y und die Wellenlinien wie oben in Anspruch 1 definiert sind und R $-(CH_2)_mOH$ ist, worin m eine ganze Zahl von 1 bis 6 ist,
um eine Verbindung der Formel (I) zu ergeben, in der:
X, Y und die Wellenlinien wie oben in Anspruch 1 definiert sind und R $-(CH_2)_mOR^1$ ist, worin m wie oben in Anspruch 1 definiert ist und $R^1$ Niederalkyl ist; oder
(k) das Überführen einer Verbindung der Formel (I) in ein Salz derselben; oder
(l) das Überführen eines Salzes einer Verbindung der Formel (I) in die freie Verbindung der Formel (I); oder
(m) das Überführen eines Salzes einer Verbindung der Formel (I), vorzugsweise eines löslichen Salzes, in ein anderes Salz der Verbindung der Formel (I), das vorzugsweise weniger löslich als das lösliche Salz ist; oder
(n) das Auftrennen einer racemischen oder nicht-racemischen Mischung der Verbindungen der Formeln (1) und (3)

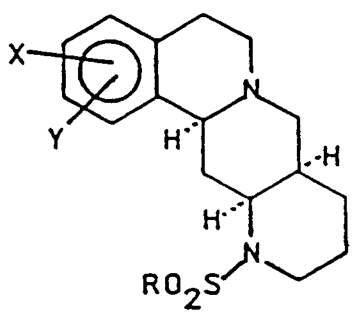

(1)

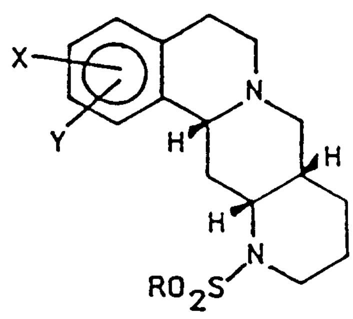

(3)

in denen:
X, Y und R wie oben in Anspruch 1 definiert sind, um eine optisch angereicherte Verbindung der Formel (1) zu ergeben.

**27.** Verfahren nach Anspruch 26, in dem (±)-2,3-Methylendioxy-12-methansulfonyl-5,6,8aα,9,10,11,12,12aα,13,13aα-decahydro-8H-isochino[2,1-g][1,6]naphthyridin;
(8aR,12aS,13aS)-2,3-Methylendioxy-12-methansulfonyl-5,6,8a,9,10,11,12,12a,13,13a-decahydro-8H-isochino[2,1-g][1,6]naphthyridin;
(±)-3-Methoxy-12-methansulfonyl-5,6,8aα,9,10,11,12,12aα,13,13aα-decahydro-8H-isochino[2,1-g][1,6]-naphthyridin;
(8aR,12aS,13aS)-3-Methoxy-12-methansulfonyl-5,6,8a,9,10,11,12,12a,13,13a-decahydro-8H-isochino[2,1-g][1,6]naphthyridin; oder ein pharmazeutisch annehmbares Salz derselben, inbesondere das Hydrochloridsalz, hergestellt wird.

**28.** Verfahren zur Herstellung einer pharmazeutischen Zusammensetzung, in dem eine Verbindung nach irgendeinem der Ansprüche 1 bis 15 oder eine Verbindung, die durch das Verfahren nach Anspruch 26

oder 27 hergestellt worden ist, mit einem pharmazeutisch annehmbaren, nicht-toxischen Träger vereinigt wird.

**29.** Einzelnes Enantiomer der Formel:

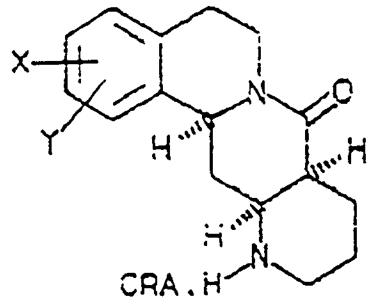

in der:
X und Y unabhängig Wasserstoff; Hydroxy; Niederalkyl mit 1 bis 6 Kohlenstoffatomen; Niederalkoxy mit 1 bis 6 Kohlenstoffatomen; oder Halogen sind; oder X und Y, wenn sie benachbart sind und zusammengenommen werden, Methylendioxy oder Ethylen-1,2-dioxy sind; und CRA eine optisch aktive Säure anzeigt.

**Patentansprüche für folgende Vertragsstaaten : ES, GR**

**1.** Verfahren zur Herstellung einer Verbindung der Formel (I):

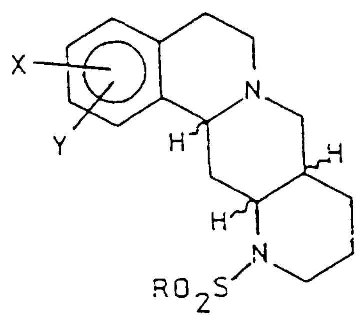

(I)

in der :
X und Y unabhängig für Wasserstoff, Hydroxy, Niederalkyl mit 1 bis 6 Kohlenstoffatomen, Niederalkoxy mit 1 bis 6 Kohlenstoffatomen oder Halogen stehen oder X und Y zusammengenommen Methylendioxy oder Ethylen-1,2-dioxy sind; und
R für Niederalkyl mit 1 bis 6 Kohlenstoffatomen; Phenyl, gegebenenfalls durch ein oder zwei Substituenten substituiert, die aus Halogen- oder Aminogruppen oder Niederalkyl- oder Niederalkoxygruppen mit 1 bis 4 Kohlenstoffatomen ausgewählt sind; $-(CH_2)_mOR^1$; oder $-NR^1R^2$ steht, worin m eine ganze Zahl von 1 bis 6 ist und $R^1$ und $R^2$ unabhängig Wasserstoff oder Niederalkyl mit 1 bis 6 Kohlenstoffatomen sind; oder die Gruppe $-NR^1R^2$ einen Heterocyclus der Formel:

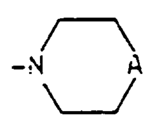

bildet, worin A $-CH_2-$, $-NR^1-$ oder Sauerstoff ist, worin $R^1$ die obige Bedeutung hat, und

die Wellenlinien anzeigen, daß sich das daran angefügte Wasserstoffatom entweder in der α- oder β-Stellung befindet; oder eines pharmazeutisch annehmbaren Salzes derselben;
wobei das Verfahren umfaßt

(a) das Sulfonylieren einer Verbindung der Formel

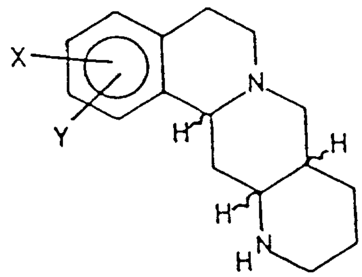

in der:
X, Y und die Wellenlinien wie oben definiert sind; mit einer Sulfonsäure der Formel $RO_2SOH$ oder einer aktivierten Form derselben, worin R wie oben definiert ist; oder

(b) das Reduzieren einer Verbindung der Formel

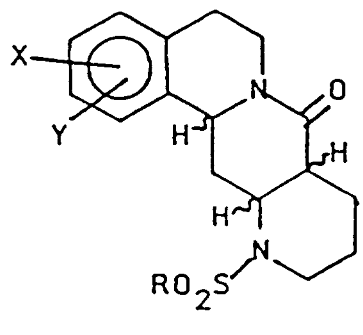

in der:
X, Y, R und die Wellenlinien wie oben definiert sind; oder

(c) das Alkylieren einer Verbindung der Formel (I), in der: mindestens eines von X und Y Hydroxy ist und R und die Wellenlinien wie oben definiert sind,
um eine Verbindung der Formel (I) zu ergeben, in der:
mindestens eines von X und Y Niederalkoxy mit 1 bis 6 Kohlenstoffatomen ist oder X und Y zusammengenommen Methylendioxy oder Ethylen-1,2-dioxy sind; oder

(d) das Oxidieren einer Verbindung der Formel

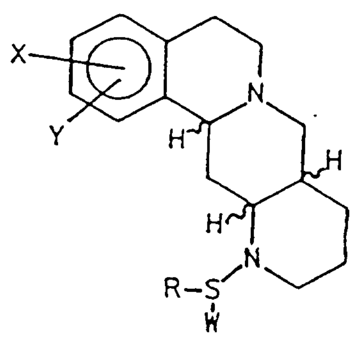

in der X, Y, R und die Wellenlinien wie oben definiert sind und W ein Elektronenpaar oder ein Sauerstoffatom ist; oder

(e) das Reduzieren einer Verbindung der Formel

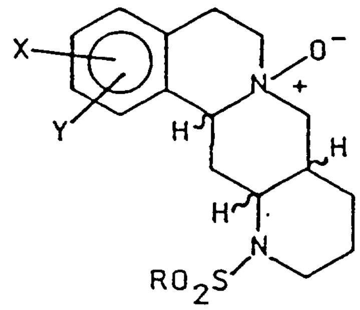

in der X, Y, R und die Wellenlinien wie oben definiert sind; oder
(f) das Epimerisieren einer Verbindung der Formel

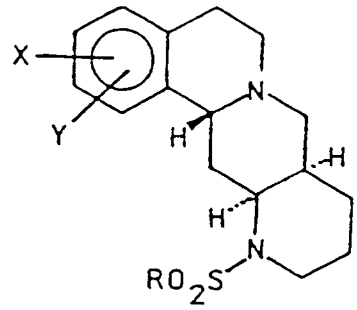

in der X, Y und R wie oben definiert sind, zu einer Verbindung der Formel

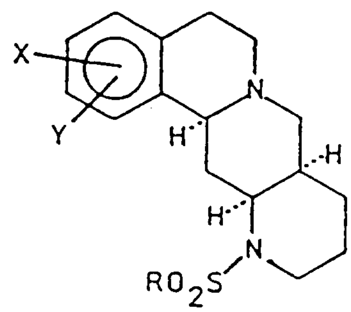

in der X, Y und R wie oben definiert sind; oder
(g) das Reduzieren einer Verbindung der Formel

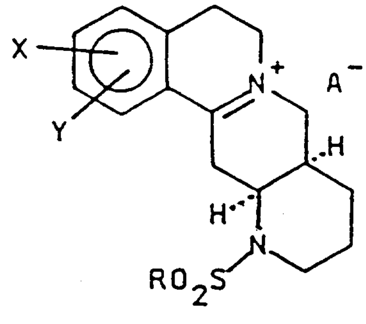

in der X, Y und R wie oben definiert sind und A ein organisches oder anorganisches Anion darstellt, um eine Verbindung der Formel

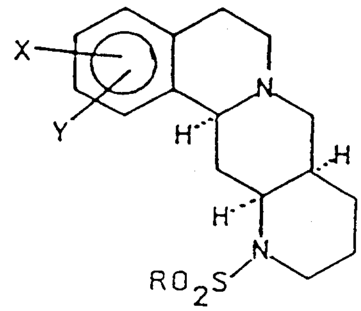

zu ergeben, in der X, Y und R wie oben definiert sind; oder

(h) das Reduzieren einer Verbindung der Formel (I), in der:
X, Y und die Wellenlinien wie oben definiert sind und R durch ein oder zwei Nitrogruppen substituiertes Phenyl ist,
um eine Verbindung der Formel (I) zu ergeben, in der:
X, Y und die Wellenlinien wie oben definiert sind und R mit ein oder zwei Aminogruppen substituiertes Phenyl ist; oder

(i) das Desalkylieren einer Verbindung der Formel (I), in der:
X, Y und die Wellenlinien wie oben definiert sind und R $-(CH_2)_mOR^1$ ist, worin m eine ganze Zahl von 1 bis 6 ist und $R^1$ Niederalkyl ist,
um eine Verbindung der Formel (I) zu ergeben, in der :
X, Y und die Wellenlinien wie oben definiert sind und R $-(CH_2)_mOH$ ist, worin m wie oben definiert ist; oder

(j) das Alkylieren einer Verbindung der Formel (I), in der:
X, Y und die Wellenlinien wie oben definiert sind und R $-(CH_2)_mOH$ ist, worin m eine ganze Zahl von 1 bis 6 ist,
um eine Verbindung der Formel (I) zu ergeben, in der:
X, Y und die Wellenlinien wie oben definiert sind und R $-(CH_2)_mOR^1$ ist, worin m wie oben definiert ist und $R^1$ Niederalkyl ist; oder

(k) das Überführen einer Verbindung der Formel (I) in ein Salz derselben; oder

(l) das Überführen eines Salzes einer Verbindung der Formel (I) in die freie Verbindung der Formel (I); oder

(m) das Überführen eines Salzes einer Verbindung der Formel (I), vorzugsweise eines löslichen Salzes, in ein anderes Salz der Verbindung der Formel (I), das vorzugsweise weniger löslich als das lösliche Salz ist; oder

(n) das Auftrennen einer racemischen oder nicht-racemischen Mischung der Verbindungen der Formeln (1) und (3)

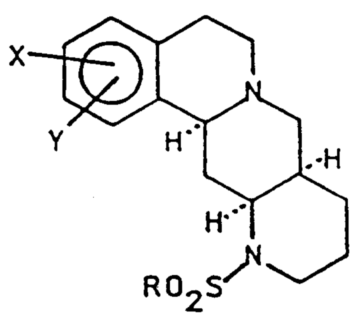

(1)

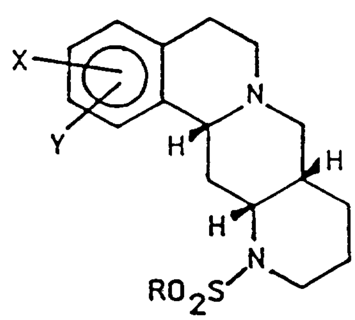

(3)

in denen:

X, Y und R wie oben definiert sind, um eine optisch angereicherte Verbindung der Formel (1) zu ergeben.

**2.** Verfahren nach Anspruch 1, in dem eine Verbindung der Formel (1) oder (2):

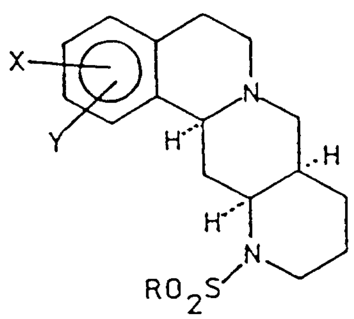
(1)

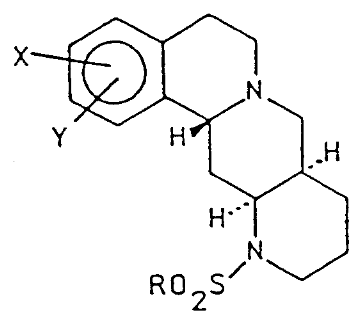
(2)

worin:
X, Y und R wie oben definiert sind; oder ein pharmazeutisch annehmbares Salz derselben hergestellt wird.

**3.** Verfahren nach Anspruch 2, in dem eine Verbindung der Formel (1); oder ein pharmazeutisch annehmbares Salz derselben hergestellt wird.

**4.** Verfahren nach Anspruch 3, in dem X und Y unabhängig Wasserstoff oder Niederalkoxy mit 1 bis 4 Kohlenstoffatomen sind oder X und Y zusammengenommen Methylendioxy sind.

**5.** Verfahren nach Anspruch 4, in dem R Niederalkyl mit 1 bis 6 Kohlenstoffatomen ist.

**6.** Verfahren nach Anspruch 5, in dem (±)-2,3-Methylendioxy-12-methansulfonyl-5,6,8aα,9,10,11,12,12aα,13,13aα-decahydro-8H-isochino[2,1-g][1,6]naphthyridin;
(8aR,12aS,13aS)-2,3-Methylendioxy-12-methansulfonyl-5,6,8a,9,10,11,12,12a,13,13a-decahydro-8H-isochino [2,1-g][1,6]naphthyridin;
(±)-2,3-Methylendioxy-12-(2-methylpropansulfonyl)-5,6,8aα,9,10,11,12,12aα,13,13aα-decahydro-8H-isochino[2,1-g][1,6]naphthyridin;
(8aR,12aS,13aS)-2,3-Methylendioxy-12-(2-methylpropansulfonyl)-5,6,8a,9,10,11,12,12a,13,13a-decahydro-8H-isochino[2,1-g][1,6]naphthyridin;
(±)-3-Methoxy-12-methansulfonyl-5,6,8aα,9,10,11,12,12aα,13,13aα-decahydro-8H-isochino[2,1-g][1,6]-naphthyridin;
(8aR,12aS,13aS)-3-Methoxy-12-methansulfonyl-5,6,8a,9,10,11,12,12a,13,13a-decahydro-8H-isochino[2,1-g][1,6]naphthyridin;
(±)-3-Methoxy-12-(2-methylpropansulfonyl)-5,6,8aα,9,10,11,12,12aα,13,13aα-decahydro-8H-isochino[2,1-g][1,6]naphthyridin;
(8aR,12aS,13aS)-3-Methoxy-12-(2-methylpropansulfonyl)-5,6,8a,9,10,11,12,12a,13,13a-decahydro-8H-isochino[2,1-g][1,6]naphthyridin;
(±)-2,3-Dimethoxy-12-methansulfonyl-5,6,8aα,9,10,11,12,12aα,13,13aα-decahydro-8H-isochino[2,1-g]-[1,6]naphthyridin;
(8aR,12aS,13aS)-2,3-Dimethoxy-12-methansulfonyl-5,6,8a,9,10,11,12,12a,13,13a-decahydro-8H-isochino[2,1-g][1,6]naphthyridin;
(±)-12-Methansulfonyl-5,6,8aα,9,10,11,12,12aα,13,13aα-decahydro-8H-isochino[2,1-g][1,6]naphthyridin;
(8aR,12aS,13aS)-12-Methansulfonyl-5,6,8a,9,10,11,12,12a,13,13a-decahydro-8H-isochino[2,1-g][1,6]-naphthyridin;
(±)-12-(2-Methylpropansulfonyl)-5,6,8aα,9,10,11,12,12aα,13,13aα-decahydro-8H-isochino[2,1-g][1,6]-naphthyridin; oder
(8aR,12aS,13aS)-12-(2-Methylpropansulfonyl)-5,6,8a,9,10,11,12,12a,13,13a-decahydro-8H-isochino[2,1-g][1,6]naphthyridin;

oder ein pharmazeutisch annehmbares Salz davon, insbesondere das Hydrochloridsalz, hergestellt wird.

**7.** Verfahren nach Anspruch 4, in dem R $-NR^1R^2$ ist.

**8.** Verfahren nach Anspruch 7, in dem $R^1$ und $R^2$ unabhängig Wasserstoff oder Niederalkyl mit 1 bis 4 Kohlenstoffatomen sind.

**9.** Verfahren nach Anspruch 8, in dem (±)-12-(N,N-Dimethylaminosulfonyl)-5,6,8aα,9,10,11,12,12aα,13,13aα-decahydro-8H-isochino[2,1-g][1,6]naphthyridin; (8aR,12aS,13aS)-3-Methoxy-12-N,N-dimethylaminosulfonyl-5,6,8a,9,10,11,12,12a,13,13a-decahydro-8H-isochino[2,1-g][1,6]-naphthyridin; oder ein pharmazeutisch annehmbares Salz derselben hergestellt wird.

**10.** Verfahren nach Anspruch 4, in dem R $-(CH_2)_mOR^1$ ist.

**11.** Verfahren nach Anspruch 10, in dem m 2 ist und $R^1$ Methyl ist.

**12.** Verfahren nach Anspruch 11, in dem (±)-12-(2-Methoxyethansulfonyl)-5,6,8aα,9,10,11,12,12aα,13,13aα-decahydro-8H-isochino[2,1-g][1,6]naphthyridin;
(8aR,12aS,13aS)-12-(2-Methoxyethansulfonyl)-5,6,8a,9,10,11,12,12a,13,13a-decahydro-8H-isochino[2,1-g][1,6]naphthyridin;
(±)-3-Methoxy-12-(2-methoxyethansulfonyl)-5,6,8aα,9,10,11,12,12aα,13,13aα-decahydro-8H-isochino[2,1-g][1,6]naphthyridin; oder
(8aR,12aS,13aS)-3-Methoxy-12-(2-methoxyethansulfonyl)-5,6,8a,9,10,11,12,12a,13,13a-decahydro-8H-isochino[2,1-g][1,6]naphthyridin; oder ein pharmazeutisch annehmbares Salz davon hergestellt wird.

**13.** Verfahren nach Anspruch 2, in dem eine Verbindung der Formel (2) oder ein pharmazeutisch annehmbares Salz derselben hergestellt wird.

**14.** Verfahren nach Anspruch 13, in dem X und Y unabhängig Wasserstoff oder Niederalkoxy mit 1 bis 4 Kohlenstoffatomen sind oder X und Y zusammengenommen Methylendioxy sind.

**15.** Verfahren nach Anspruch 14, in dem R Niederalkyl mit 1 bis 4 Kohlenstoffatomen oder $-NR^1R^2$ ist.

**16.** Verfahren zur Herstellung einer pharmazeutischen Zusammensetzung, in dem eine therapeutisch wirksame Menge einer nach dem Verfahren von irgendeinem der vorangehenden Ansprüche hergestellten Verbindung oder eines pharmazeutisch annehmbaren Salzes derselben mit einem pharmazeutisch annehmbaren, nicht-toxischen Träger kombiniert wird.

**17.** Verfahren nach Anspruch 16, in dem die pharmazeutische Zusammensetzung zur Verabreichung an einen Säuger mit einem Krankheitszustand, der durch Behandlung mit einem $\alpha_2$-Blocker gemildert wird, geeignet ist.

**18.** Verfahren nach Anspruch 16, in dem die Verbindung (8aR,12aS,13aS)-2,3-Methylendioxy-12-methansulfonyl-5,6,8a,9,10,11,12,12a,13,13a-decahydro-8H-isochino[2,1-g][1,6]naphthyridin; oder (8aR,12aS,13aS)-3-Methoxy-12-methansulfonyl-5,6,8a,9,10,11,12,12a,13,13a-decahydro-8H-isochino[2,1-g][1,6]naphthyridin; oder ein pharmazeutisch annehmbares Salz davon, insbesondere das Hydrochloridsalz, ist.

**19.** Verfahren nach Anspruch 17 oder 18, in dem der Krankheitszustand Depression, übermäßige Blutplättchenaggregation, Diabetes, erhöhten intraoccularen Druck, männliche Impotenz, Reizdarmsyndrom, Hochdruck, Unruhe, zyklische Stimmungsstörungen bei Frauen und/oder Fettleibigkeit umfaßt.

**20.** Verwendung einer Verbindung, wie nach dem Verfahren von irgendeinem der Ansprüche 1 bis 15 hergestellt, bei der Herstellung eines Medikaments zur Behandlung eines Krankheitszustandes, der durch Behandlung mit einem $\alpha_2$-Blocker gemildert wird.

**21.** Verfahren zur Herstellung eines einzelnen Enantiomers der Formel:

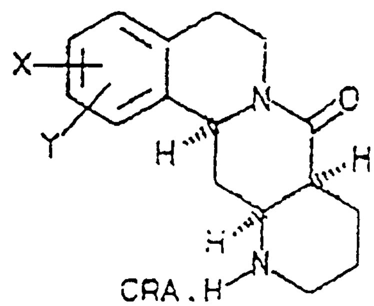

in der:
X und Y unabhängig Wasserstoff; Hydroxy; Niederalkyl mit 1 bis 6 Kohlenstoffatomen; Niederalkoxy mit 1 bis 6 Kohlenstoffatomen; oder Halogen sind; oder X und Y, wenn sie benachbart sind und zusammengenommen werden, Methylendioxy oder Ethylen-1,2-dioxy sind; und CRA eine optisch aktive Säure ist;
umfassend das Auftrennen der diastereomeren Salze, die durch das Umsetzen einer racemischen Mischung einer Verbindung der Formel (V), in der X und Y wie oben definiert sind,

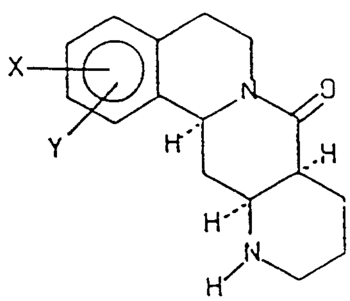

(V)

mit einer optisch aktiven Säure bei einer Temperatur zwischen 0 °C und der Rückflußtemperatur des zur fraktionierten Kristallisation verwendeten Lösungsmittels gebildet werden.

## Revendications

## Revendications pour les Etats contractants suivants : AT, BE, CH, DE, FR, GB, IT, LI, LU, NL, SE

**1.** Composé de formule (I) :

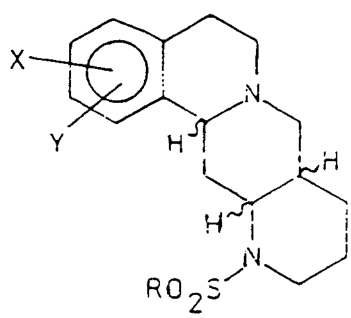

(I)

dans laquelle
X et Y représentent indépendamment l'hydrogène, des groupes hydroxy, alkyle inférieurs ayant

un à six atomes de carbone, alkoxy inférieurs ayant un à six atomes de carbone ou halogéno, ou bien X et Y, pris conjointement, représentent un groupe méthylènedioxy ou éthylène-1,2-dioxy ; et

R représente un groupe alkyle inférieur ayant un à six atomes de carbone ; phényle portant facultativement un ou deux substituants choisis entre des groupes halogéno ou amino ou des groupes alkyle inférieurs ou alkoxy inférieurs ayant un à quatre atomes de carbone ; $-(CH_2)_mOR^1$ ; ou $-NR^1R^2$ dans lequel m représente un nombre entier de 1 à 6 et $R^1$ et $R^2$ représentent indépendamment l'hydrogène ou des groupes alkyle inférieurs ayant 1 à 6 atomes de carbone ; ou bien le groupe $-NR^1R^2$ forme un hétérocycle de formule :

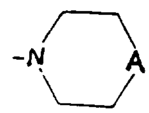

dans laquelle A représente un groupe $-CH_2-$, $-NR^1-$ ou l'oxygène, $R^1$ répondant à la définition précitée et les lignes ondulées indiquant que l'atome d'hydrogène fixé à la molécule est en position $\alpha$ ou $\beta$ ; ou un de ses sels pharmaceutiquement acceptables.

2. Composé suivant la revendication 1, répondant à la formule (1) ou (2) :

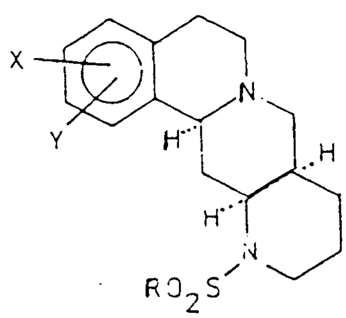

(1)

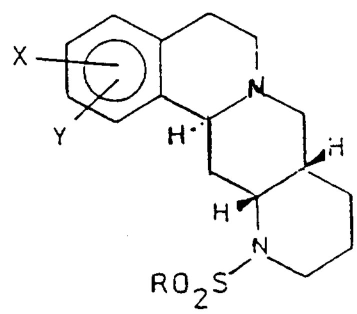

(2)

dans laquelle

X, Y et R répondent aux définitions suivant la revendication 1 ; ou un de ses sels pharmaceutiquement acceptables.

3. Composé suivant la revendication 2, qui est un composé de formule (1) ou un de ses sels pharmaceutiquement acceptables.

4. Composé suivant la revendication 3, dans lequel X et Y représentent indépendamment l'hydrogène ou des groupes alkoxy inférieurs ayant un à quatre atomes de carbone, ou bien X et Y, pris conjointement, représentent un groupe méthylènedioxy ; ou un de ses sels pharmaceutiquement acceptables.

5. Composé suivant la revendication 4, dans lequel R représente un groupe alkyle inférieur ayant un à six atomes de carbone ; ou un de ses sels pharmaceutiquement acceptables.

6. Composé suivant la revendication 5, à savoir la (±)-2,3-méthylènedioxy-12-méthanesulfonyl-5,6,8a$\alpha$,9,10,11,12,-12a$\alpha$,13,13a$\alpha$-décahydro-8H-isoquino-[2,1-g][1,6]-naphtyridine ;
la (8aR,12aS,13aS)-2,3-méthylènedioxy-12-méthanesulfonyl-5,6,8a,9,10,11,12,12a,13,13a-décahydro-8H-isoquino-[2,1-g][1,6]naphtyridine ;
la (±)-2,3-méthylènedioxy-12-(2-méthylpropanesulfonyl)-5,6,8a$\alpha$,9,10,11,12,12a$\alpha$,13,13a$\alpha$-décahydro-8H-isoquino[2,1-g][1,6]naphtyridine ;
la (8aR,12aS,13aS)-2,3-méthylènedioxy-12-(2-méthylpropanesulfonyl)-5,6,8a,9,10,11,12,12a,13,13a-décahydro-8H-isoquino[2,1-g][1,6]naphtyridine ;

la (±)3-méthoxy-12-méthanesulfonyl-5,6,8aα,9,10,11,12,12aα,13,13aα-décahydro-8H-isoquino[2,1-g][1,6]-naphtyridine ;
la (8aR,12aS,13aS)-3-méthoxy-12-méthanesulfonyl-5,6,8a,9,10,11,12,12a,13,13a-décahydro-8H-isoquino-[2,1-g][1,6]-naphtyridine ;
la (±)-3-méthoxy-12-(2-méthylpropanesulfonyl)-5,6,8aα,9,10,11,12,12aα,13,13aα-décahydro-8H-isoquino-[2,1-g][1,6]-naphtyridine ;
la (8aR,12aS,13aS)-3-méthoxy-12-(2-méthylpropanesulfonyl)-5,6,8a,9,10,11,12,12a,13,13a-décahydro-8H-isoquino-[2,1-g][1,6]naphtyridine ;
la (±)-2,3-diméthoxy-12-méthanesulfonyl-5,6,8aα,9,10,11,12,12aα,13,13aα-décahydro-8H-isoquino[2,1-g]-[1,6]-naphtyridine ;
la (8aR,12aS,13aS)-2,3-diméthoxy-12-méthanesulfonyl-5,6,8a,9,10,11,12,12a,13,13a,décahydro-8H-isoquino-[2,1-g][1,6]-naphtyridine ;
la (±)-12-méthanesulfonyl-5,6,8aα,9,10,11,12,12aα,13,13aα-décahydro-8H-isoquino[2,1-g][1,6]-naphtyridine ;
la (8aR,12aS,13aS)-12-méthanesulfonyl-5,6,8a,9,10,11,12,12a, 13,13a-décahydro-8H-isoquino[2,1-g]-[1,6]-naphtyridine ;
la (±)-12-(2-méthylpropanesulfonyl)-5,6,8aα,9,10,11,12,12aα, 13,13aα-décahydro-8H-isoquino[2,1-g][1,6]-naphtyridine ; ou
la (8aR,12aS,13aS)-12-(2-méthylpropanesulfonyl)-5,6,8a,9,10, 11,12,12a,13,13a-décahydro-8H-isoquino-[2,1-g][1,6]-naphtyridine;
ou un de ses sels pharmaceutiquement acceptables, notamment le chlorhydrate.

7. Composé suivant la revendication 4, dans lequel R représente un groupe $-NR^1R^2$, ou un de ses sels pharmaceutiquement acceptables.

8. Composé suivant la revendication 7, dans lequel $R^1$ et $R^2$ représentent indépendamment l'hydrogène ou des groupes alkyle inférieurs ayant un à quatre atomes de carbone, ou un de ses sels pharmaceutiquement acceptables.

9. Composé suivant la revendication 8, à savoir la (±)-12-(N,N-diméthylaminosulfonyl)-5,6,8aα,9,10,11,12,12aα,13,13aα-décahydro-8H-isoquino[2,1-g][1,6]-naphtyridine ; la (8aR,12aS,13aS)-3-méthoxy-12-N,N-diméthylamino-sulfonyl-5,6,8a,9,10,11,12,12a,13,13a-décahydro-8H-isoquino[2,1-g]-[1,6]naphtyridine ; ou un de ses sels pharmaceutiquement acceptables.

10. Composé suivant la revendication 4, dans lequel R représente un groupe $-(CH_2)_mOR^1$ ; ou un de ses sels pharmaceutiquement acceptables.

11. Composé suivant la revendication 10, dans lequel m est égal à 2 et $R^1$ représente un groupe méthyle ; ou un de ses sels pharmaceutiquement acceptables.

12. Composé suivant la revendication 11, à savoir la (±)-12-(2-méthoxyéthanesulfonyl)-5,6,8aα,9,10,11,12,12aα,13,13aα-décahydro-8H-isoquino[2,1-g][1,6]-naphtyridine ;
la (8aR,12aS,13aS)-12-(2-méthoxyéthanesulfonyl)-5,6,8a,9,10,11,12,12a,13,13a-décahydro-8H-isoquino-[2,1-g][1,6]-naphtyridine ;
la (±)-3-méthoxy-12-(2-méthoxyéthanesulfonyl)-5,6,8aα,9,10, 11,12,12aα,13,13aα-décahydro-8H-isoquino-[2,1-g][1,6]naphtyridine ; ou
la (8aR,12aS,13aS)-3-méthoxy-12-(2-méthoxyéthanesulfonyl)-5,6,8a,9,10,11,12,12a,13,13a-décahydro-8H-isoquino[2,1-g][1,6]naphtyridine ; ou un de ses sels pharmaceutiquement acceptables.

13. Composé suivant la revendication 2, qui est un composé de formule (2) ou un de ses sels pharmaceutiquement acceptables.

14. Composé suivant la revendication 13, dans lequel X et Y représentent indépendamment l'hydrogène ou des groupes alkoxy inférieurs ayant un à quatre atomes de carbone ou bien X et Y, pris conjointement, représentent un groupe méthylènedioxy, ou un de ses sels pharmaceutiquement acceptables.

15. Composé suivant la revendication 14, dans lequel R représente un groupe alkyle inférieur ayant un à quatre atomes de carbone ou $-NR^1R^2$, ou un de ses sels pharmaceutiquement acceptables.

**16.** Composition pharmaceutique comprenant un support non toxique pharmaceutiquement acceptable et une quantité à effet thérapeutique d'un composé suivant l'une des revendications précédentes.

**17.** Composition pharmaceutique suivant la revendication 16, convenant pour l'administration à un mammifère présentant un état pathologique qui est soulagé par traitement avec un agent $\alpha_2$-bloquant.

**18.** Composition pharmaceutique suivant la revendication 16, dans laquelle le composé suivant l'une des revendications précédentes est
la (8aR,12aS,13aS)-2,3-méthylènedioxy-12-méthanesulfonyl-5,6,8a,9,10,11,12,12a,13,13a-décahydro-8H-isoquino-[2,1-g][1,6]naphtyridine ; ou
la (8aR,12aS,13aS)-3-méthoxy-12-méthanesulfonyl-5,6,8a,9,10,11,12,12a,13,13a-décahydro-8H-isoquino-[2,1-g][1,6]naphtyridine ; ou un de ses sels pharmaceutiquement acceptables, notamment le chlorhydrate.

**19.** Composition pharmaceutique suivant la revendication 17 ou 18, dans laquelle l'état pathologique consiste en une dépression, une agrégation plaquettaire excessive, un diabète, une pression intraoculaire élevée, l'impuissance masculine, le syndrome d'irritabilité intestinale, l'hypertension, l'anxiété, des perturbations cycliques d'humeur chez la femme et/ou l'obésité.

**20.** Utilisation d'un composé suivant l'une quelconque des revendications 1 à 15 dans la production d'un médicament destiné au traitement d'un état pathologique qui est soulagé par traitement avec un agent $\alpha_2$-bloquant.

**21.** Composé de formule

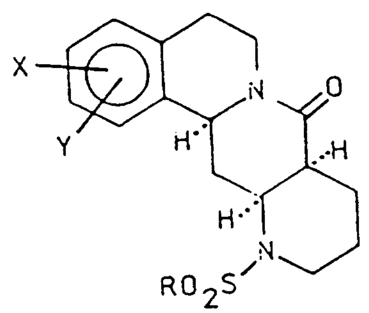

dans laquelle
X, Y et R répondent aux définitions suivant la revendication 1.

**22.** Composé de formule

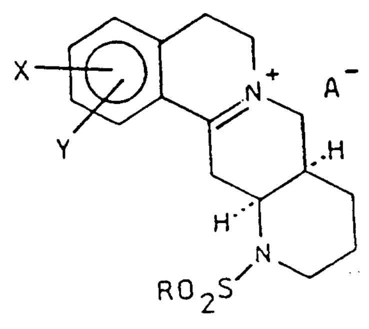

dans laquelle
X, Y et R répondent aux définitions suivant la revendication 1 et A représente un anion organique ou inorganique.

**23.** Composé de formule

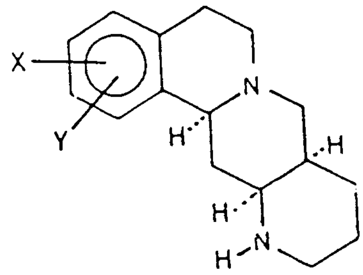

dans laquelle
X et Y répondent aux définitions suivant la revendication 1.

**24.** Composé suivant la revendication 23, dans lequel au moins l'un des substituants X et Y ne représente pas l'hydrogène.

**25.** Composé suivant la revendication 24, dans lequel X représente un groupe alkoxy inférieur ayant un à six atomes de carbone et Y représente l'hydrogène, ou bien X et Y, pris conjointement, représentent un groupe méthylènedioxy ou éthylène-1,2-dioxy.

**26.** Procédé de préparation d'un composé suivant la revendication 1, ledit procédé comprenant
(a) la sulfonylation d'un composé de formule

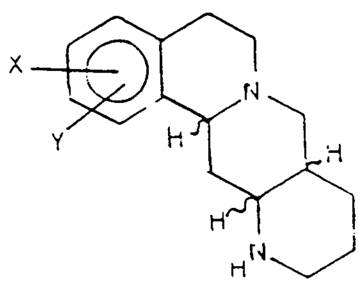

dans laquelle
X, Y et les lignes ondulées répondent aux définitions précitées dans la revendication 1 ; avec un acide sulfonique de formule $RO_2SOH$ ou une de ses formes activées, R répondant à la définition précitée ; ou
(b) la réduction d'un composé de formule

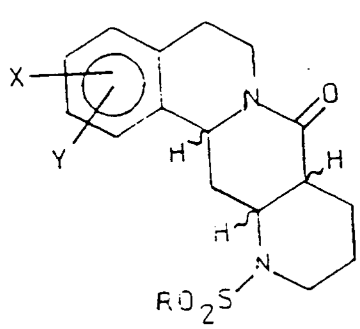

dans laquelle
X, Y, R et les lignes ondulées répondent aux définitions précitées dans la revendication 1 ; ou

(c) l'alkylation d'un composé de formule (I) dans laquelle :

au moins l'un des substituants X et Y représente un groupe hydroxy et R et les lignes ondulées répondent aux définitions précitées dans la revendication 1, ce qui donne un composé de formule (I)

dans laquelle :

au moins l'un des substituants X et Y représente un groupe alkoxy inférieur ayant un à six atomes de carbone, ou bien X et Y, pris conjointement, représentent un groupe méthylènedioxy ou éthylène-1,2-dioxy ; ou

(d) l'oxydation d'un composé de formule

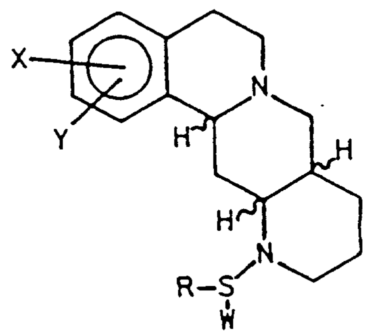

dans laquelle X, Y, R et les lignes ondulées répondent aux définitions précitées dans la revendication 1 et W représente une paire d'électrons ou un atome d'oxygène ; ou

(e) la réduction d'un composé de formule

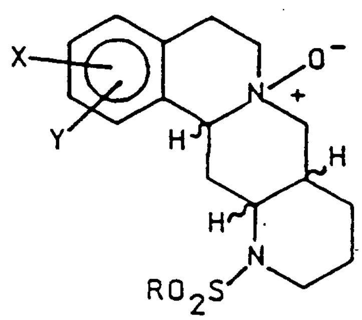

dans laquelle X, Y, R et les lignes ondulées répondent aux définitions précitées dans la revendication 1 ; ou

(f) l'épimérisation d'un composé de formule

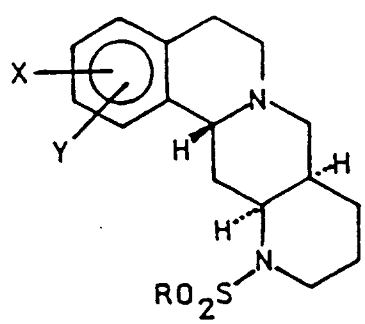

dans laquelle X, Y et R répondent aux définitions précitées dans la revendication 1, en un composé de formule

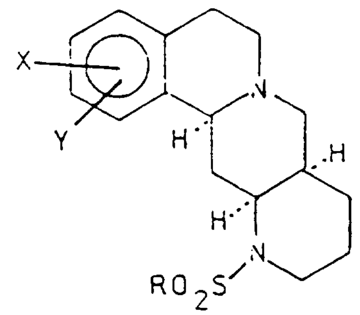

dans laquelle X, Y et R répondent aux définitions précitées dans la revendication 1 ; ou
(g) la réduction d'un composé de formule

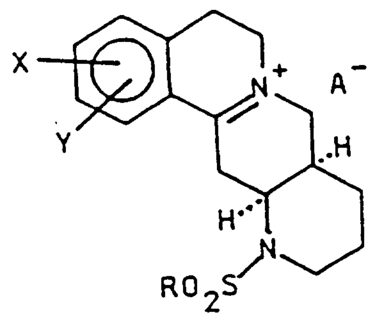

dans laquelle X, Y et R répondent aux définitions précitées dans la revendication 1 et A représente un anion organique ou inorganique, ce qui donne un composé de formule

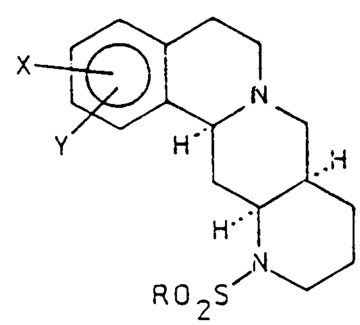

dans laquelle X, Y et R répondent aux définitions précitées dans la revendication 1 ; ou
(h) la réduction d'un composé de formule (I) dans laquelle :

X, Y et les lignes ondulées répondent aux définitions précitées dans la revendication 1 et R représente un groupe phényle substitué avec un ou deux groupes nitro, ce qui donne un composé de formule (I) dans laquelle :

X, Y et les lignes ondulées répondent aux définitions précitées dans la revendication 1 et R représente un groupe phényle substitué avec un ou deux groupes amino ; ou
(i) la désalkylation d'un composé de formule (I) dans laquelle :

X, Y et les lignes ondulées répondent aux définitions précitées dans la revendication 1 et R représente un groupe $-(CH_2)_mOR^1$ dans lequel m est un nombre entier de 1 à 6 et $R^1$ représente un groupe alkyle inférieur,

ce qui donne un composé de formule (I) dans laquelle :

X, Y et les lignes ondulées répondent aux définitions précitées dans la revendication 1 et R représente un groupe $-(CH_2)_mOH$ dans lequel m répond à la définition précitée dans la revendication 1 ; ou

(j) l'alkylation d'un composé de formule (I) dans laquelle :

X, Y et les lignes ondulées répondent aux définitions précitées dans la revendication 1 et R représente un groupe $-(CH_2)_mOH$ dans lequel m est un nombre entier de 1 à 6, ce qui donne un composé de formule (I) dans laquelle :

X, Y et les lignes ondulées répondent aux définitions précitées dans la revendication 1 et R représente un groupe $-(CH_2)_mOR^1$ dans lequel m répond à la définition précitée dans la revendication 1 et $R^1$ représente un groupe alkyle inférieur ; ou

(k) la transformation d'un composé de formule (I) en un de ses sels ; ou

(l) la transformation d'un sel d'un composé de formule (I) en le composé libre de formule (I) ; ou

(m) la transformation d'un sel d'un composé de formule (I), de préférence d'un sel soluble, en un autre sel du composé de formule (I), de préférence moins soluble que ledit sel soluble ; ou

(n) la résolution d'un mélange racémique ou non racémique des composés de formules (1) et (3)

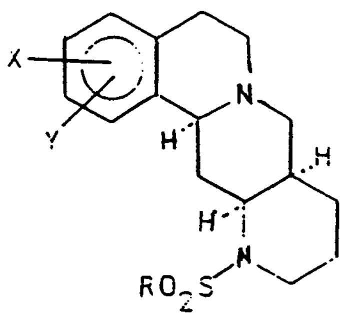

(1)

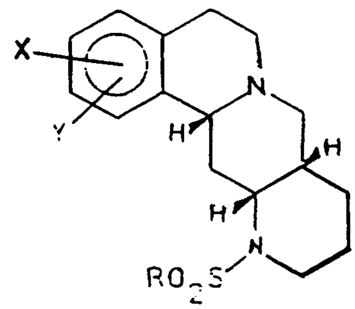

(3)

dans lesquelles

X, Y et R répondent aux définitions précitées dans la revendication 1,

ce qui donne un composé enrichi optiquement de formule (1).

**27.** Procédé suivant la revendication 26, dans lequel la (±)-2,3-méthylènedioxy-12-méthanesulfonyl-5,6,8aα,9,10,11,12,12aα,13,13aα-décahydro-8H-isoquino-[2,1-g][1,6]naphtyridine ;
la (8aR,12aS,13aS)-2,3-méthylènedioxy-12-méthanesulfonyl-5,6,8a,9,10,11,12,12a,13,13a-décahydro-8H-isoquino-[2,1-g][1,6]naphtyridine ;
la (±)-3-méthoxy-12-méthanesulfonyl-5,6,8aα,9,10,11,12,12aα,13,13aα-décahydro-8H-isoquino[2,1-g]-[1,6]-naphtyridine ;
la (8aR,12aS,13aS)-3-méthoxy-12-méthanesulfonyl-5,6,8a,9,10,11,12,12a,13,13a-décahydro-8H-isoquino-[2,1-g][1,6]-naphtyridine ; ou un de ses sels pharmaceutiquement acceptables, notamment le chlorhydrate, est préparé.

**28.** Procédé de préparation d'une composition pharmaceutique dans laquelle un composé suivant l'une quelconque des revendications 1 à 15 ou un composé qui a été préparé par le procédé suivant la revendication 26 ou 27 est mélangé à un support non toxique pharmaceutiquement acceptable.

**29.** Enantiomère distinct, de formule :

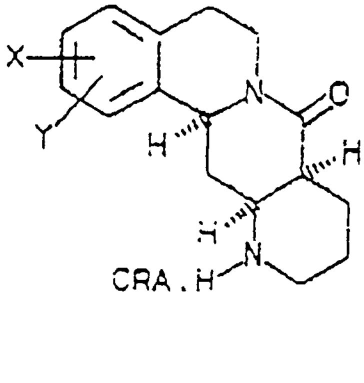

dans laquelle

X et Y représentent indépendamment l'hydrogène ; des groupes hydroxy ; alkyle inférieurs ayant un à six atomes de carbone, alkoxy inférieurs ayant un à six atomes de carbone ; ou halogéno ; ou bien X et Y, lorsqu'ils sont adjacents et pris conjointement, représentent un groupe méthylènedioxy ou éthylène-1,2-dioxy ; et le terme CRA désigne un acide optiquement actif.

**Revendications pour les Etats contractants suivants : ES, GR**

**1.** Procédé de préparation d'un composé de formule (I)

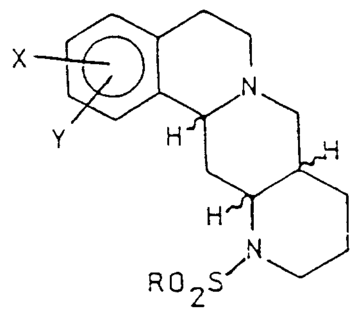

(I)

dans laquelle :

X et Y représentent indépendamment l'hydrogène, des groupes hydroxy, alkyle inférieurs ayant un à six atomes de carbone, alkoxy inférieurs ayant un à six atomes de carbone ou halogéno, ou bien X et Y, pris conjointement, représentent un groupe méthylènedioxy ou éthylène-1,2-dioxy ; et

R représente un groupe alkyle inférieur ayant un à six atomes de carbone ; phényle portant facultativement un ou deux substituants choisis entre des groupes halogéno ou amino ou des groupes alkyle inférieurs ou alkoxy inférieurs ayant un à quatre atomes de carbone ; $-(CH_2)_mOR^1$ ; ou $-NR^1R^2$ dans lequel m est un nombre entier de 1 à 6 et $R^1$ et $R^2$ représentent indépendamment l'hydrogène ou des groupes alkyle inférieurs ayant 1 à 6 atomes de carbone ; ou bien le groupe $-NR^1R^2$ forme un hétérocycle de formule :

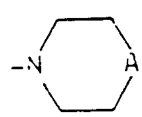

dans laquelle A représente un groupe $-CH_2-$, $-NR^1-$ ou l'oxygène, $R^1$ répondant à la définition précitée et les lignes ondulées indiquant que l'atome d'hydrogène fixé à la molécule est en position $\alpha$ ou $\beta$ ; ou d'un de ses sels pharmaceutiquement acceptables ; ledit procédé comprenant

(a) la sulfonylation d'un composé de formule

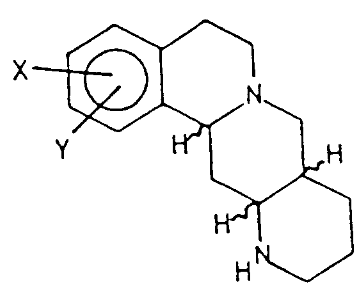

dans laquelle

X, Y et les lignes ondulées répondent aux définitions précitées ; avec un acide sulfonique de formule $RO_2SOH$ ou une de ses formes activées, R répondant à la définition précitée ; ou

(b) la réduction d'un composé de formule

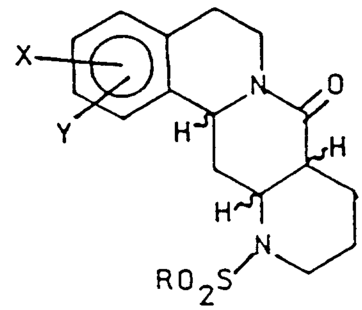

dans laquelle

X, Y, R et les lignes ondulées répondent aux définitions précitées ; ou

(c) l'alkylation d'un composé de formule (I) dans laquelle :

au moins l'un des substituants X et Y représente un groupe hydroxy et R et les lignes ondulées répondent aux définitions précitées, ce qui donne un composé de formule (I)

dans laquelle :

au moins l'un des substituants X et Y représente un groupe alkoxy inférieur ayant un à six atomes de carbone, ou bien X et Y, pris conjointement, représentent un groupe méthylènedioxy ou éthylène-1,2-dioxy ; ou

(d) l'oxydation d'un composé de formule

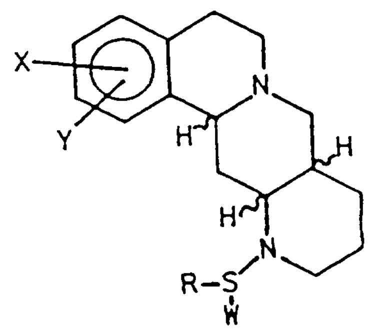

dans laquelle X, Y, R et les lignes ondulées répondent aux définitions précitées et W représente une paire d'électrons ou un atome d'oxygène ; ou

(e) la réduction d'un composé de formule

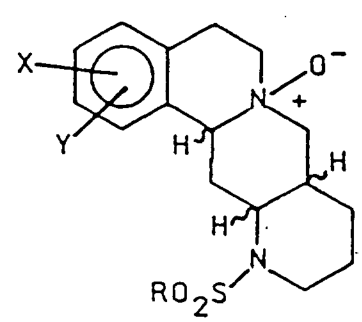

dans laquelle X, Y, R et les lignes ondulées répondent aux définitions précitées ; ou

(f) l'épimérisation d'un composé de formule

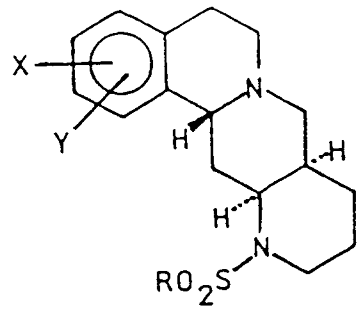

dans laquelle X, Y et R répondent aux définitions précitées, en un composé de formule

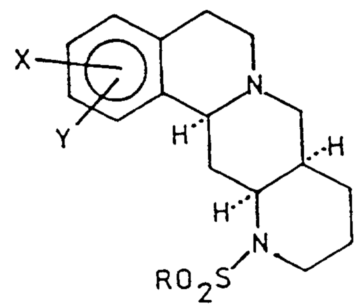

dans laquelle X, Y et R répondent aux définitions précitées ; ou
(g) la réduction d'un composé de formule

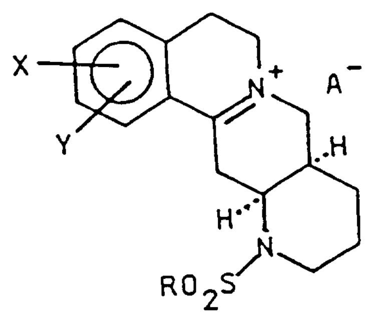

dans laquelle X, Y et R répondent aux définitions précitées et A représente un anion organique ou inorganique, ce qui donne un composé de formule

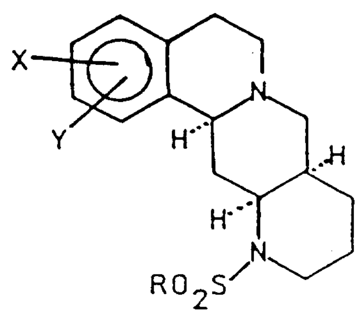

dans laquelle X, Y et R répondent aux définitions précitées ; ou

(h) la réduction d'un composé de formule (I) dans laquelle :

X, Y et les lignes ondulées répondent aux définitions précitées et R représente un groupe phényle substitué avec un ou deux groupes nitro, ce qui donne un composé de formule (I) dans laquelle :

X, Y et les lignes ondulées répondent aux définitions précitées et R représente un groupe phényle substitué avec un ou deux groupes amino ; ou

(i) la désalkylation d'un composé de formule (I) dans laquelle :

X, Y et les lignes ondulées répondent aux définitions précitées et R représente un groupe $-(CH_2)_mOR^1$ dans lequel m est un nombre entier de 1 à 6 et $R^1$ représente un groupe alkyle inférieur,

ce qui donne un composé de formule (I) dans laquelle :

X, Y et les lignes ondulées répondent aux définitions précitées et R représente un groupe $-(CH_2)_mOH$ dans lequel m répond à la définition précitée ; ou

(j) l'alkylation d'un composé de formule (I) dans laquelle :

X, Y et les lignes ondulées répondent aux définitions précitées et R représente un groupe $-(CH_2)_mOH$ dans lequel m est un nombre entier de 1 à 6,

ce qui donne un composé de formule (I) dans laquelle :

X, Y et les lignes ondulées répondent aux définitions précitées et R représente un groupe $-(CH_2)_mOR^1$ dans lequel m répond à la définition précitée et $R^1$ représente un groupe alkyle inférieur ; ou

(k) la transformation d'un composé de formule (I) en un de ses sels ; ou

(l) la transformation d'un sel d'un composé de formule (I) en le composé libre de formule (I) ; ou

(m) la transformation d'un sel d'un composé de formule (I), de préférence un sel soluble, en un autre sel du composé de formule (I), de préférence moins soluble que ledit sel soluble ; ou

(n) la résolution d'un mélange racémique ou non racémique des composés de formules (1) et (3)

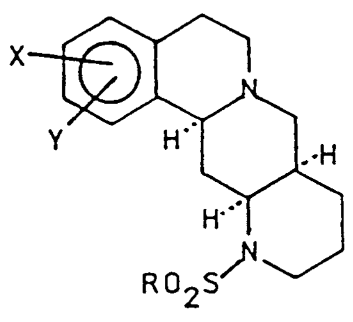

(1)

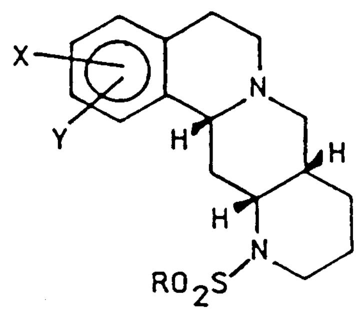

(3)

dans lesquelles

X, Y et R répondent aux définitions précitées,

ce qui donne un composé optiquement enrichi de formule (1).

**2.** Procédé suivant la revendication 1, dans lequel un composé de formule (1) ou (2) :

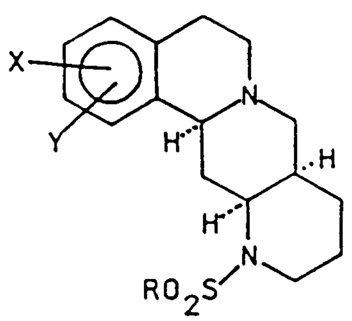

(1)

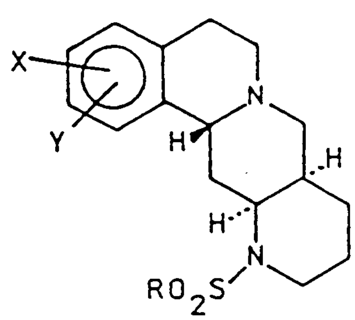

(2)

dans laquelle

X, Y et R répondent aux définitions précitées ; ou un de ses sels pharmaceutiquement acceptables, est préparé.

**3.** Procédé suivant la revendication 2, dans lequel un composé de formule (1), ou un de ses sels pharmaceutiquement acceptables, est préparé.

**4.** Procédé suivant la revendication 3, dans lequel X et Y représentent indépendamment l'hydrogène ou des groupes alkoxy inférieurs ayant un à quatre atomes de carbone, ou bien X et Y, pris conjointement, représentent un groupe méthylènedioxy.

**5.** Procédé suivant la revendication 4, dans lequel R représente un groupe alkyle inférieur ayant un à six atomes de carbone.

**6.** Procédé suivant la revendication 5, dans lequel la (±)-2,3-méthylènedioxy-12-méthanesulfonyl-5,6,8aα,9,10,11,12,12aα,13,13aα-décahydro-8H-isoquino-[2,1-g][1,6]naphtyridine ;
la (8aR,12aS,13aS)-2,3-méthylènedioxy-12-méthanesulfonyl-5,6,8a,9,10,11,12,12a,13,13a-décahydro-8H-isoquino-[2,1-g][1,6]naphtyridine ;
la (±)-2,3-méthylènedioxy-12-(2-méthylpropanesulfonyl)-5,6,8aα,9,10,11,12,12aα,13,13aα-décahydro-8H-isoquino[2,1-g][1,6)naphtyridine ;
la (8aR,12aS,13aS)-2,3-méthylènedioxy-12-(2-méthylpropanesulfonyl)-5,6,8a,9,10,11,12,12a,13,13a-décahydro-8H-isoquino[2,1-g][1,6]naphtyridine ;
la (±)-3-méthoxy-12-méthanesulfonyl-5,6,8aα,9,10,11,12,12aα,13,13aα-décahydro-8H-isoquino[2,1-g]-[1,6]-naphtyridine ;
la (8aR,12aS,13aS)-3-méthoxy-12-méthanesulfonyl-5,6,8a,9,10,11,12,12a,13,13a-décahydro-8H-isoquino-[2,1-g][1,6]-naphtyridine ;
la (±)-3-méthoxy-12-(2-méthylpropanesulfonyl)-5,6,8aα,9,10,11,12,12aα,13,13aα-décahydro-8H-isoquino-[2,1-g][1,6]naphtyridine ;
la (8aR,12aS,13aS)-3-méthoxy-12-(2-méthylpropanesulfonyl)-5,6,8a,9,10,11,12,12a,13,13a-décahydro-8H-isoquino-[2,1g][1,6]naphtyridine ;
la (±)-2,3-diméthoxy-12-méthanesulfonyl-5,6,8aα,9,10,11,12,12aα,13,13aα-décahydro-8H-isoquino[2,1-g]-[1,6]-naphty ridine ;
la(8aR,12aS,13aS)-2,3-diméthoxy-12-méthanesulfonyl-5,6,8a,9,10,11,12,12a,13,13a,décahydro-8H-isoquino-[2,1-g][1,6]naphtyridine ;
la (±)-12-méthanesulfonyl-5,6,8aα,9,10,11,12,12aα,13,13aα-décahydro-8H-isoquino[2,1-g][1,6]-naphtyridine ;
la (8aR,12aS,13aS)-12-méthanesulfonyl-5,6,8a,9,10,11,12,12a, 13,13a-décahydro-8H-isoquino[2,1-g]-[1,6]-naphtyridine ;
la (±)-12-(2-méthylpropanesulfonyl)-5,6,8aα,9,10,11,12,12aα,13,13aα-décahydro-8H-isoquino[2,1-g][1,6]-naphtyridine ; ou
la (8aR,12aS,13aS)-12-(2-méthylpropanesulfonyl)-5,6,8a,9,10,11,12,12a,13,13a-décahydro-8H-isoquino-[2,1-g][1,6]-naphtyridine ;

ou un de ses sels pharmaceutiquement acceptables, notamment le chlorhydrate, est préparé.

**7.** Procédé suivant la revendication 4, dans lequel R représente un groupe $-NR^1R^2$.

**8.** Procédé suivant la revendication 7, dans lequel $R^1$ et $R^2$ représentent indépendamment l'hydrogène ou des groupes alkyle inférieurs ayant un à quatre atomes de carbone.

**9.** Procédé suivant la revendication 8, dans lequel la (±)-12-(N,N-diméthylaminosulfonyl)-5,6,8aα,9,10,11-12,12aα,13,13aα-décahydro-8H-isoquino[2,1-g][1,6]-naphtyridine ; ou
la (8aR,12aS,13aS)-3-méthoxy-12-N,N-diméthylaminosulfonyl-5,6,8a,9,10,11,12,12a,13,13a-décahydro-8H-isoquino[2,1-g][1,6]naphtyridine ; ou un de ses sels pharmaceutiquement acceptables, est préparé.

**10.** Procédé suivant la revendication 4, dans lequel R représente un groupe $-(CH_2)_mOR^1$.

**11.** Procédé suivant la revendication 10, dans lequel m est égal à 2 et $R^1$ représente un groupe méthyle.

**12.** Procédé suivant la revendication 11, dans lequel la (±)-12-(2-méthoxyéthanesulfonyl)-5,6,8aα,9,10,11,12,12aα,13,13aα-décahydro-8H-isoquino[2,1-g][1,6]-naphtyridine ;
la (8aR,12aS,13aS)-12-(2-méthoxyéthanesulfonyl)-5,6,8a,9,10,11,12,12a,13,13a-décahydro-8H-isoquino-[2,1-g][1,6]-naphtyridine ;
la (±)-3-méthoxy-12-(2-méthoxyéthanesulfonyl)-5,6,8aα,9,10, 11,12,12aα,13,13aα-décahydro-8H-isoquino-[2,1-g][1,6]naphtyridine ; ou
la (8aR,12aS,13aS)-3-méthoxy-12-(2-méthoxyéthanesulfonyl)-5,6,8a,9,10,11,12,12a,13,13a-décahydro-8H-isoquino-[2,1-g][1,6]naphtyridine ; ou un de ses sels pharmaceutiquement acceptables, est préparé.

**13.** Procédé suivant la revendication 2, dans lequel un composé de formule (2), ou un de ses sels pharmaceutiquement acceptables, est préparé.

**14.** Procédé suivant la revendication 13, dans lequel X et Y représentent indépendamment l'hydrogène ou des groupes alkoxy inférieurs ayant un à quatre atomes de carbone, ou bien X et Y, pris conjointement, représentent un groupe méthylènedioxy.

**15.** Procédé suivant la revendication 14, dans lequel R représente un groupe alkyle inférieur ayant un à quatre atomes de carbone ou $-NR^1R^2$.

**16.** Procédé de préparation d'une composition pharmaceutique, dans lequel une quantité thérapeutiquement efficace d'un composé préparé par le procédé suivant l'une quelconque des revendications précédentes ou d'un de ses sels pharmaceutiquement acceptables est mélangée à un support non toxique pharmaceutiquement acceptable.

**17.** Procédé suivant la revendication 16, dans lequel la composition pharmaceutique convient pour l'administration à un mammifère présentant un état pathologique qui est soulagé par traitement avec un agent $\alpha_2$-bloquant.

**18.** Procédé suivant la revendication 16, dans lequel le composé est la (8aR,12aS,13aS)-2,3-méthylènedioxy-12-méthanesulfonyl-5,6,8a,9,10,11,12,12a,13,13a-décahydro-8H-isoquino-[2,1-g][1,6]-naphtyridine ; ou
la (8aR,12aS,13aS)-3-méthoxy-12-méthanesulfonyl-5,6,8a,9,10,11,12,12a,13,13a-décahydro-8H-isoquino-[2,1-g][1,6]naphtyridine ; ou un de ses sels pharmaceutiquement acceptables, notamment le chlorhydrate.

**19.** Procédé suivant la revendication 17 ou 18, dans lequel l'état pathologique consiste en une dépression, une agrégation plaquettaire excessive, un diabète, une pression intraoculaire élevée, l'impuissance masculine, le syndrome d'irritabilité intestinale, l'hypertension, l'anxiété, des perturbations cycliques de l'humeur chez la femme ou l'obésité.

**20.** Utilisation d'un composé préparé par le procédé suivant l'une quelconque des revendications 1 à 15, dans la production d'un médicament pour le traitement d'un état pathologique qui est soulagé par traitement avec un agent $\alpha_2$-bloquant.

**21.** Procédé de production d'un énantiomère distinct, de formule

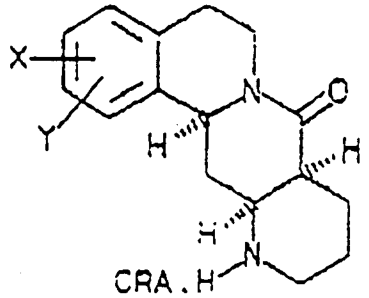

dans laquelle

X et Y représentent indépendamment l'hydrogène ; des groupes hydroxy ; alkyle inférieurs ayant un à six atomes de carbone ; alkoxy inférieurs ayant un à six atomes de carbone ; ou halogéno ; ou bien X et Y, lorsqu'ils sont adjacents et pris conjointement, représentent un groupe méthylènedioxy ou éthylène-1,2-dioxy ; et le terme CPA désigne un acide optiquement actif ;

procédé comprenant la séparation des sels diastéréoisomères formés par réaction d'un mélange racémique d'un composé de formule (V), dans laquelle X et Y répondent aux définitions précitées

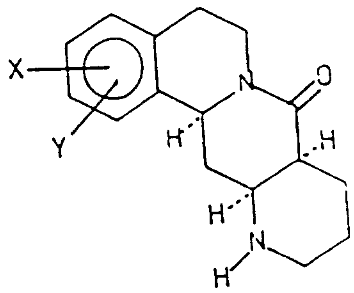

(V)

avec un acide optiquement actif, à une température comprise dans l'intervalle de 0 °C à la température de reflux du solvant utilisé pour la cristallisation fractionnée.